# FASCICULE DE BREVET EUROPEEN

(11) **EP 0 977 730 B1**
(45) Date de publication et mention de la délivrance du brevet: **12.03.2003**
(21) Numéro de dépôt: 98922850.7
(22) Date de dépôt: 21.04.1998
(51) Int. Cl.: C07C 259/06, C07C 323/41, C07D 209/26, A61K 31/16, A61K 31/40

(54) **DERIVES D'AMINO-ACIDES INHIBITEURS DES METALLOPROTEASES DE LA MATRICE EXTRACELLULAIRE ET DE LA LIBERATION DU TNF ALPHA**
AMINOSÄUREDERIVATE ALS INHIBITOREN FÜR EXTRAZELLULÄREN MATRIX-METALLOPROTEASEN UND DER FREISETZUNG VON TNF ALPHA
AMINO ACID DERIVATIVES INHIBITING EXTRACELLULAR MATRIX METALLOPROTEINASE AND TNF ALPHA RELEASE

(30) Priorité: 22.04.1997 FR 9704971
(43) Date de publication de la demande: 09.02.2000
(73) Titulaire: CHIESI FARMACEUTICI S.p.A., I-43100 Parma (IT)
(72) Inventeur: JEANPETIT, Christian, F-78380 Bougival (FR); PRIGENT, Didier, F-91440 Bures sur Yvette (FR); SETTEMBRE, Pierre-André, F-78800 Houilles (FR); TRANCART, Marie-Michèle, F-78960 Voisins le Bretonneux (FR)
(74) Mandataire: Minoja, Fabrizio, Dr.
(86) Numéro de dépôt international: FR9800801
(87) Numéro de publication internationale: WO98047863

(56) Documents cités:
- EP-A- 0 613 883
- WO-A-97/03966
- A. K. GHOSE ET AL: "Determination of pharmacophoric geometry for collagenase inhibitors using a novel computational method and its verification using molecular dynamics, NMR, and X-ray crystallography" JOURNAL OF THE AMERICAN CHEMICAL SOCIETY, vol. 117, no. 16, 1995, pages 4671-4682, XP002051616 DC US cité dans la demande
- R. P. ROBINSON ET AL: "Inhibitors of MMP-1: an examination of P1' Ca gem-disubstitution in the succinamide hydroxymate series" BIOORGANIC & MEDICINAL CHEMISTRY LETTERS, vol. 6, no. 14, 1996, pages 1719-1724, XP002051617 cité dans la demande
- B. J. BROUGHTON ET AL: "Studies concering the antibiotic Actinomycin. Part VIII. Structure-activity relationships in the actinonin series" JOURNAL OF THE CHEMICAL SOCIETY, PERKIN TRANSACTIONS 1., no. 9, 1975, pages 857-860, XP002051618 LETCHWORTH GB
- S. BAILEY ET AL: "Hydroxamate-based inhibitors of low affinity IgE receptor(CD23)processing" BIOORGANIC & MEDICINAL CHEMISTRY LETTERS, vol. 8, no. 1, janvier 1998, pages 23-28, XP002076276

## Description

La présente invention concerne des nouveaux dérivés d'amino-acides possédant une activité inhibitrice de métalloprotéases de la matrice extracellulaire, et plus particulièrement la gélatinase, un procédé pour la production de ces dérivés et des compositions pharmaceutiques les contenant. Ces dérivés possèdent également une activité d'inhibition de la libération du TNF α (Tumor Necrosis Factor ou facteur de nécrose tumorale) ainsi que de la production de TGF α (Tumor Growth Factor ou facteur de croissance tumorale).

La dégradation de la matrice extraceilulaire est principalement due à l'action enzymatique des métalloprotéases (MMP).

L'activité enzymatique de ces métalloprotéases est physiologiquement régulée par des inhibiteurs naturels tels que les TIMP (Tissue Inhibitor of Metalloproteinase ou inhibiteur tissulaire des métalloprotéases) ou l'alpha-2-macroglobuline. Un déséquilibre dans la production des enzymes et de leurs inhibiteurs conduit à une activité protéasique élevée observée dans des pathologies impliquant un processus de dégradation de la matrice extracellulaire.

Les composés ayant la propriété d'inhiber l'action des métalloprotéases impliquées dans la dégradation de la matrice extracellulaire telles que les collagénases, gélatinases et stromélysines peuvent donc être utiles dans le traitement de pathologies dans lesquelles les métalloprotéases sont impliquées telles que l'arthrite rhumatoïde, l'ostéoarthrite, l'ostéoporose, l'ulcération de la cornée, les périodontites, les gingivites ou l'invasion tumorale et la prolifération métastatique, l'athérosclérose, le SIDA, les maladies inflammatoires chroniques de l'intestin, ces exemples n'étant pas limitatifs.

Le TNF α est une cytokine proinflammatoire qui est produite initialement sous la forme d'un précurseur inactif de 28 kDa. Le clivage de ce précurseur conduit à la libération d'une forme active de 17 kDa impliquée dans de nombreuses pathologies inflammatoires, immunologiques, infectieuses ou malignes. Des composés inhibant la libération de TNFα peuvent donc être utiles dans le traitement de nombreuses pathologies dans lesquelles le TNF α est impliqué telles que l'arthrite rhumatoïde, la maladie de Crohn, la sclérose en plaques, le choc septique, le cancer ou la cachexie associée à une immunodéficience, ces exemples n'étant pas limitatifs.

Le TGF α est un facteur de croissance faisant partie de la famille de l'EGF (Epidermal Growth Factor ou facteur de croissance épidermique). Il est produit par les tissus embryonnaires, les kératinocytes, les macrophages, les éosinophiles, les épithéliums (glande mammaire et cornée), le pancréas, la muqueuse gastrique, l'hypophyse et le cerveau.

Le TGF α interagit avec le récepteur de l'EGF ; il s'ensuit une cascade de réactions aboutissant à la mitose. Le TGF α est également mitogène pour les cellules tumorales.

Le TGF α induit la transformation et la croissance de cellules in vitro.

Une surproduction de TGF α est observée dans les tumeurs ainsi que dans les lignées cellulaires dérivées de tumeur mammaire. Le TGFα est également impliqué dans l'angiogénèse. Il stimule également l'hypercalcémie et inhibe la sécrétion acide gastrique. Enfin, il est impliqué dans les réactions inflammatoires.

Des composés inhibant la production de TGF α peuvent donc être utiles dans le traitement de pathologies où le TGF α est impliqué telles que le cancer, le psoriasis, l'eczéma, la formation de chéloïdes, la rétinopathie diabétique, l'athérosclérose, les maladies inflammatoires, ces exemples n'étant pas limitatifs.

De nombreux inhibiteurs des MMP et/ou de la libération de TNF sont déjà connus, les plus actifs étant les dérivés d'acide hydroxamique de formule générale I : 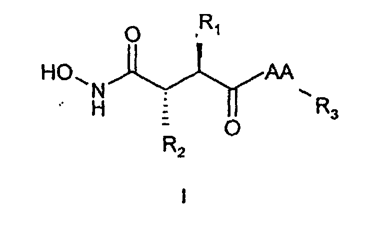 ou R₁ représente une chaîne alkyle généralement isobutyle et AA un acide aminé ou un enchaînement d'acides aminés. De tels composés sont décrits par exemple dans les demandes de brevet EP 0214639, WO 93/20047, WO 94102447, WO 94/21625, WO 94/10990, WO 95/06031. Des inhibiteurs de MMP inhibant la production de TGF_{α} sont décrits dans la demande internationale WO 96/25156.

D'autres composés ont été décrits comme inhibiteurs de métalloprotéases de la matrice dans lesquels la fonction hydroxamique a été remplacée par une fonction thiol (formule générale II) ou phosphinique (formule générale III).

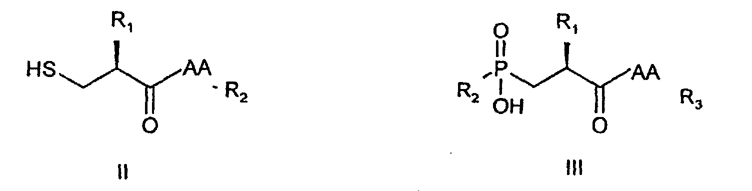

Enfin des dérivés de N-carboxyméthyl peptides ont été également revendiqués (formule générale IV)

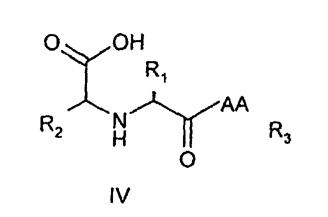

Dans ces différentes familles, le résidu R₁ interagit avec le sous-site S'₁ des différentes enzymes. La stéréochimie du carbone portant ce résidu est essentielle pour l'activité et doit être de configuration précise, R, dans le cas des dérivés hydroxamiques (J. Enzyme Inhibition, (1987), 2, 1-22) et phosphiniques. Aucun des brevets existants décrivant des inhibiteurs de MMP ne mentionne de disubstitution sur le carbone portant R₁.

La substitution de ce carbone est donc d'une extrême importance pour l'activité et il a été montré en particulier que la substitution de l'hydrogène de ce carbone par un reste méthyle induit une perte d'activité d'un facteur 300 entre le composé V et le composé VI (J. Am. Chem.Soc. (1995), 117, 4671-4682).

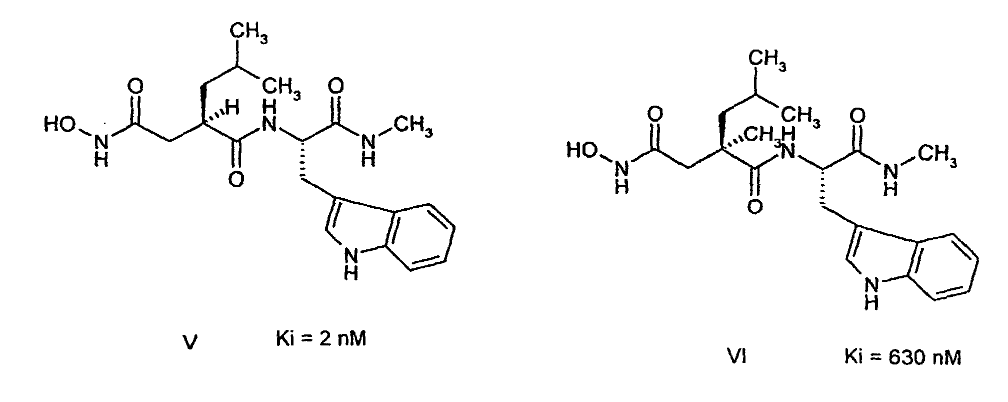

R.P. ROBINSON et al. (Bioorg.Med. Chem. Letters (1996), 6, 1719-1724) ont également étudiés la substitution de ce carbone. La gem disubstitution conduit systématiquement à une perte importante d'activité (composé VII versus composés VIII et IX)

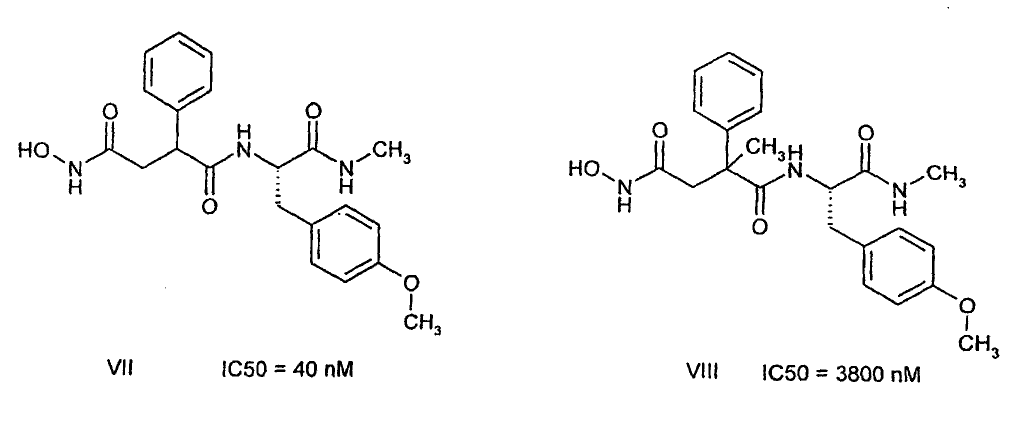

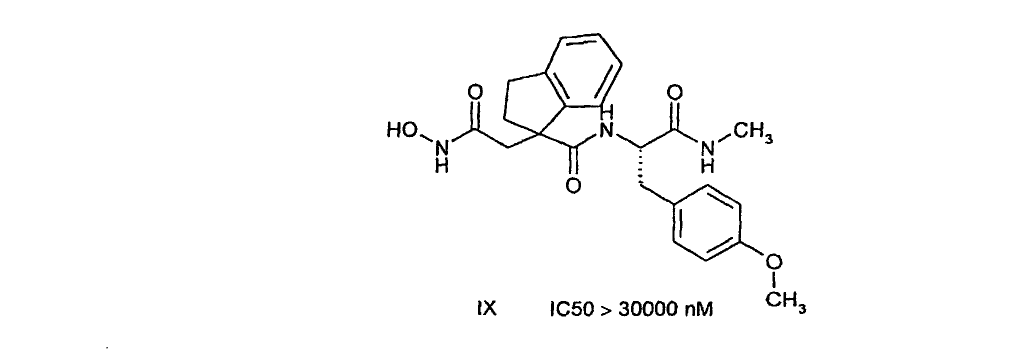

La présente invention découle de la découverte faite par les Inventeurs, que, de façon tout à fait inattendue compte tenu de l'état de la technique exposé ci-dessus, les composés de formule générale X suivante : 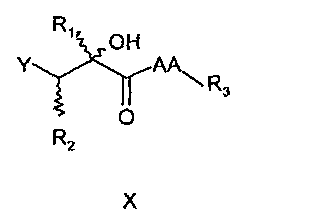 caractérisés par une gem disubstitution de ce carbone avec un résidu R₁ et une fonction alcool sont des inhibiteurs puissants des métalloprotéases de la matrice extracellulaire, et plus particulièrement de la gélatinase (concentration inhibitrice à 50 % : Cl50 < 200 nM). Ces composés inhibent également la libération de TNF α de macrophages de souris stimulés par du LPS (lipopolysaccharides) (CI50 : 10 à 0,01 µM) ainsi que la production de TGF α.

La présente invention a pour but de fournir de nouveaux composés inhibiteurs des métalloprotéases, et/ou de la libération de TNF α et/ou de la production de TGF α, les différentes activités d'inhibition de ces composés étant comparables voire supérieures à celles des composés de l'état de la technique décrit ci-dessus.

La présente invention a également pour but de fournir de nouveaux médicaments contenant à titre de principe actif les nouveaux composés susmentionnés, et présentant l'avantage de posséder une meilleure biodisponibilité que les composés de l'état de la technique décrit ci-dessus.

La présente invention a également pour but l'utilisation des nouveaux composés susmentionnés pour la préparation de nouveaux médicaments décrits ci-dessus, susceptibles d'être utilisés dans le cadre du traitement de pathologies dans lesquelles les métalloprotéases de la matrice extracellulaire et/ou le TNFα sont impliqués ainsi que les pathologies dans lesquelles une surproduction de TGF α est impliquée.

La présente invention a pour objet les composés de formule générale (X) suivante : 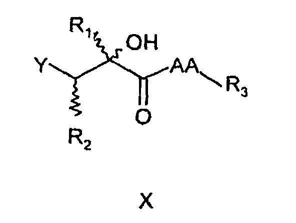 dans laquelle :
- Y représente :
   - CONHOH, ou
   - -SH, ou
   - un groupe de formule 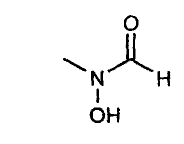 ou
   - un groupe de formule 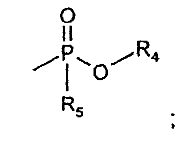 dans lequel :
      - R₄ représente -H, ou un groupe alkyle en C₁ à C₆, où un groupe phénylalkyle dans lequel le groupe alkyle est en C₁ à C₆,
      - R₅ représente un groupe de formule 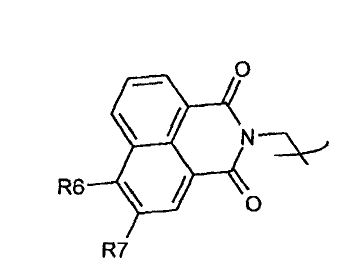 dans lequel :
         - • R₆ représente -H, ou un groupe alkoxy en C₁ à C₆, ou un groupe benzyloxy,
         - • R₇ représente -H, ou un atome d'halogène tel que -Cl ou -Br,
- R₁ représente :
   - une chaîne alkyle linéaire ou ramifiée en C₃ à C₁₆, ou cyclisée en C₃ à C₆, ladite chaîne comportant le cas échéant un hétéroatome tel que O, S ou N,
   - un groupe phénoxyalkyle, ou phénylalkyle, substitués ou non, ou un groupe hétéroarylalkyle, le groupe alkyle étant en C₂ à C₅,
- R₂ représente :
   - un atome d'hydrogène, ou,
   - un groupe alkyle en C₁ à C₅, ou alkylidène en C₂ à C₅, ou
   - un hydroxyle, un alkoxy en C₁ à C₆ ou un benzyloxy, à condition que Y représente -CONHOH lorsque R₂ représente un hydroxyle, ou
   - un groupe hydroxyméthyle, ou alkoxyméthyle en C₁ à C₆, ou
   - un groupe arylalkyle dans lequel la partie alkyle est en C₁ à C₆, un groupe aryloxyméthyle, un groupe arylthiométhyle, un groupe hétéroarylthiométhyle, dans lesquels aryle désigne un reste phényle éventuellement substitué, notamment par -OH, -OCH₃, un groupe alkyle en C₁ à C₃ linéaire ou ramifié, un halogène tel que -Cl ou -Br, un groupe aminé tel que -NH₂, -NHCOCH₃, -NHCOOR₁₀, R₁₀ représentant un groupe alkyle en C₁ à C₃ linéaire ou ramifié, ou
   - un groupe phtalimido alkyle dans lequel la partie alkyle est en C₁ à C₆, ou
   - un groupe alkoxycarbonylméthyle (alkoxy désignant méthoxy, éthoxy), un benzyloxycarbonylméthyle, un acétylméthyle, à condition dans ces trois cas que Y représente -SH,
- AA représente un acide aminé, ou un enchaînement d'acides aminés, ces acides aminés étant naturels ou non, et avantageusement de configuration absolue S, notamment un acide aminé de formule 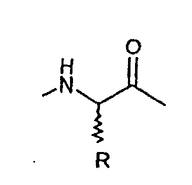 dans laquelle R représente :
   - une chaîne alkyle linéaire ou ramifiée en C₁ à C₄,
   - un groupe -CH₂-Y' dans lequel Y' représente un cycle de 4 à 6 atomes de carbone dans le cycle, comportant le cas échéant un ou plusieurs hétéroatomes tels que O, S ou N, ledit cycle étant aromatique ou non, le cas échéant substitué notamment par un ou plusieurs groupes -OCH₃, -NO₂, -NH₂, ou par un ou plusieurs atomes d'halogène notamment choisis parmi -Cl, -Br, -F et -I,
   - un groupe de formule 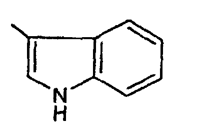
- R₃ représente un groupe de formule -NH-(R₈)ₙ-R₉ dans laquelle :
   - n représente 0 ou 1,
   - R₈ représente une chaîne alkyle linéaire ou ramifiée, de 1 à 8 atomes de carbone, comportant le cas échéant un ou plusieurs hétéoratomes tels que O ou S,
   - R₉ représente un atome d'hydrogène ou un groupe méthyle, nitrile, morpholino, phényle, méthoxy, hydroxyle, thiométhyle, ou un groupe de formule -CH(NH₂) = N-OH, ou un groupe -N(CH₃)₂.

L'invention a plus particulièrement pour objet les composés caractérisés par la formule générale (Xa) suivante : 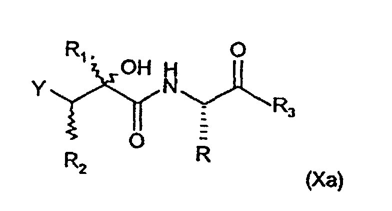 dans laquelle :
- Y représente -CONHOH,
- R₁ représente :
   - CH(CH₃)₂,
   - CH₂ - CH(CH₃)₂,
   - 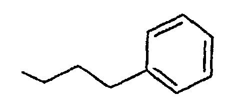
- R₂ représente :
   - un groupe alkyle de 1 à 5 atomes de carbone, notamment un groupe méthyle, ou propyle,
   - un hydroxyle, ou
   - un groupe alkoxy de 1 à 5 atomes de carbone, notamment un groupe méthoxy,
- R représente :
   - -C(CH₃)₃,
   - -CH₂ - CH(CH₃)₂,
   - un groupe 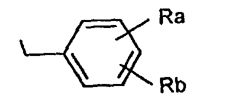 aromatique ou non, dans lequel Rₐ et R_{b}, indépendamment l'un de l'autre, représentent -H, -Cl, -Br, -I, -F, -OCH₃, -NO₂, -NH₂,
   - un groupe de formule 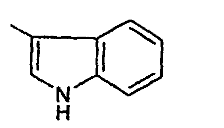
- R₃ représente un groupe -NH - (CH₂)ₙ₁ - R₉ dans lequel :
   - n₁ représente 0,1 1 ou 2,
   - R₉ représente -CH₃, -C≡N, -COOCH₃, -SCH₃, -O-(CH₂)₂-OH, -O-(CH₂)₂-OCH₃, -CH(NH₂)=N-OH,

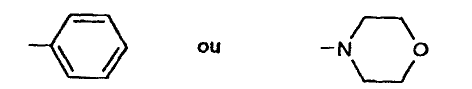

Des composés particulièrement préférés dans le cadre de la présente invention sont ceux possédant une stéréochimie telle que les substituants R₁ et R₂ sont positionnés en anti par rapport au reste succinique selon la formule (XI.1) suivante:

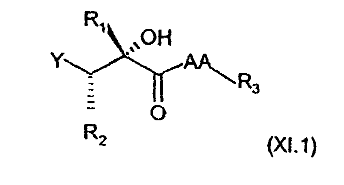

L'invention a plus particulièrement pour objet, à titre de composés préférés, ceux répondant aux formules X ou Xa susmentionnées, dans lesquelles R représente un groupe de formule 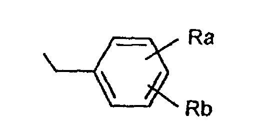 dans laquelle Rₐ et R_{b} représentent un atome d'halogène, notamment un atome de chlore.

L'invention a également plus particulièrement pour objet, à titre de composés préférés, ceux répondant aux formules X ou Xa susmentionnées, dans lesquelles R₃ représente un groupe de formule

-NH -(CH₂)₂ -SCH₃

L'invention a également pour objet tout mélange comprenant, d'une part des composés de formule (XI. 1) suivante : 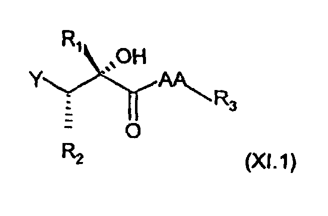 dans laquelle Y, R₁ et R₂, AA et R₃ sont tels que définis ci-dessus, et, d'autre part des composés de formule (XI.2) suivante : 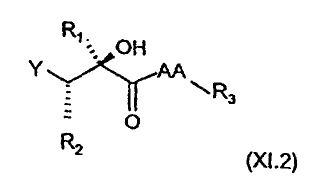 dans laquelle Y, R₁, R₂, AA et R₃ ont la signification indiquée ci-dessus, la proportion des composés (XI. 1) et (XI.2) dans le mélange étant avantageusement d'environ 50 % à environ 99 % pour le composé de formule (XI.1) et d'environ 50 % à environ 1 % pour le composé de formule (XI.2).

Des composés particulièrement préférés dans le cadre de la présente invention sont ceux répondant aux formules suivantes :

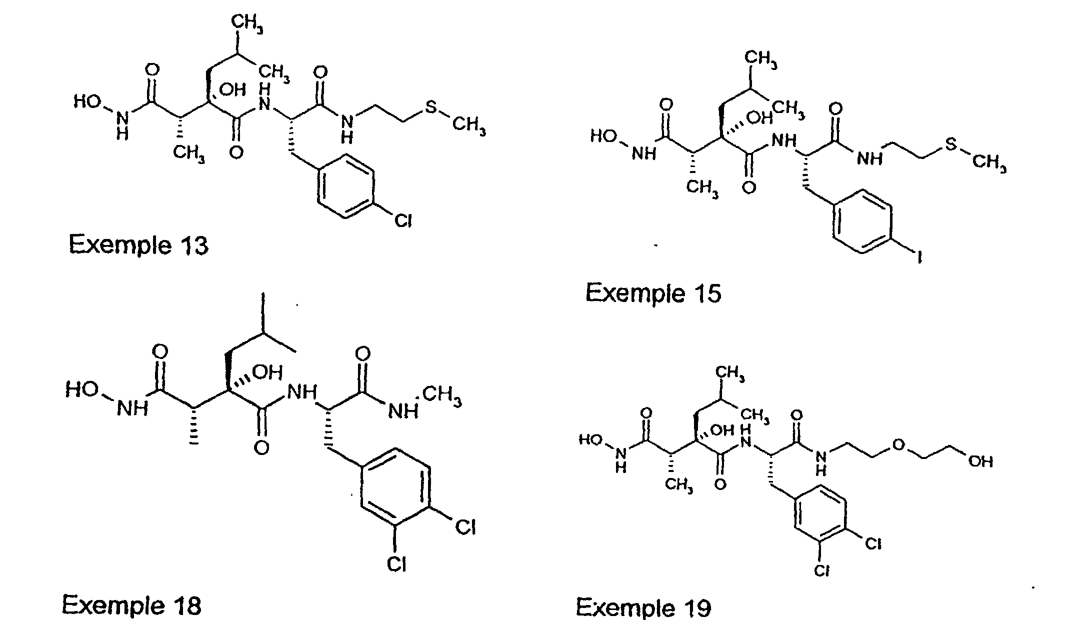

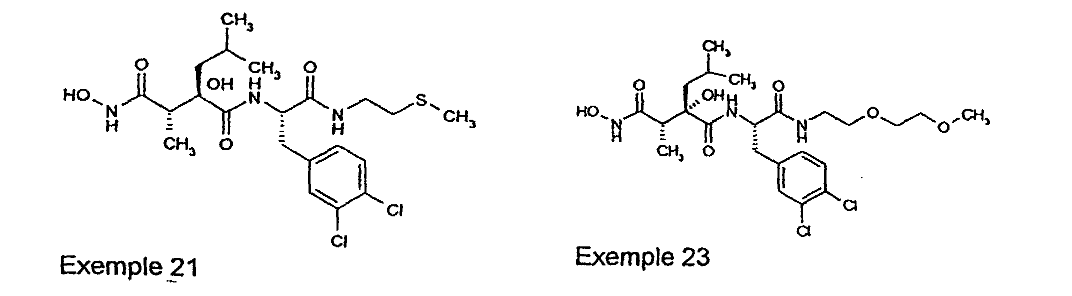

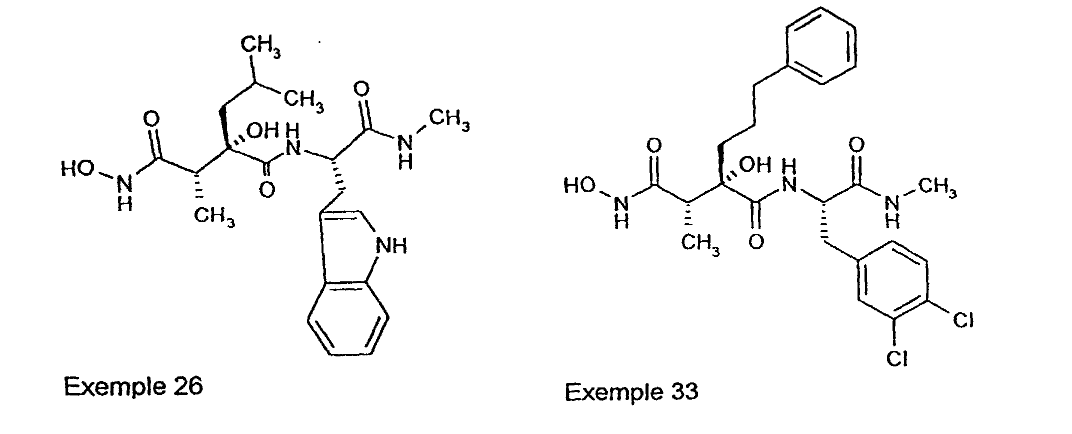

L'invention a également pour objet toute composition pharmaceutique comprenant, à titre de principe actif, un (ou plusieurs) composé(s) et/ou un (ou plusieurs) mélange(s), tels que décrits ci-dessus, en association avec un véhicule pharmaceutiquement acceptable.

Avantageusement les compositions pharmaceutiques selon l'invention, se présentent sous une forme administrable par voie orale, ou parentérale, ou rectale.

De préférence, les compositions pharmaceutiques selon l'invention, sont caractérisées en ce que la posologie en principe actif est d'environ 0,1 à environ 500 mg/kg/jour, de préférence de 1 à 300 mg/kg/jour par voies orale et rectale, et d'environ 0,1 µg/kg/jour à 1 mg/kg/jour par voie parentérale.

Des compositions pharmaceutiques préférées selon l'invention, se présentent sous une forme administrable par voie orale, en dose unitaire de 1 mg à 250 mg de principe actif par prise, et de préférence de 10 mg à 250 mg de principe actif par prise, à raison de 1 à 4 prises par jour.

Des compositions pharmaceutiques également préférées selon l'invention, se présentent sous une forme administrable par voie parentérale, en dose unitaire de 1µg à 50 mg de principe actif par injection, à raison de 1 à 2 injections par jour.

L'invention a également pour objet l'utilisation d'un (ou plusieurs) composé(s) et/ou d'un (ou plusieurs) mélange(s), tels que décrits ci-dessus, pour la préparation d'un médicament destiné au traitement de pathologies humaines ou animales dans lesquelles sont impliqués les métalloprotéases, et/ou le TNFα, et/ou le TGFα.

L'invention a plus particulièrement pour objet l'utilisation d'un (ou plusieurs) composé(s) et/ou d'un (ou plusieurs) mélange(s), tels que décrits ci-dessus, pour la préparation d'un médicament ayant la propriété d'inhiber l'action des métalloprotéases impliquées dans la dégradation de la matrice extracellulaire, telles que les collagénases, gélatinases et stromélysines, ce médicament étant destiné au traitement de pathologies humaines ou animales liées à cette action des métalloprotéases, notamment au traitement:
- de l'arthrite rhumatoïde,
- de l'ostéoarthrite,
- de l'ostéoporose,
- de l'ulcération de la cornée,
- des périodontites,
- des gingivites,
- des invasions tumorales,
- de la prolifération métastatique,
- de l'athérosclérose,
- du SIDA,
- des maladies inflammatoires chroniques de l'intestin,
- des maladies neurodègénératives telles que la maladie d'Alzheimer, la sclérose en plaques.

L'invention a plus particulièrement pour objet l'utilisation d'un (ou plusieurs) composé(s) et/ou d'un (ou plusieurs) mélange(s), tels que décrits ci-dessus, pour la préparation d'un médicament ayant la propriété d'inhiber la libération de TNFα à partir de son précurseur inactif, ce médicament étant destiné au traitement de pathologies humaines ou animales, dans lesquelles le TNFα est impliqué, notamment au traitement des pathologies inflammatoires, immunologiques, infectieuses ou malignes, telles que :
- l'arthrite rhumatoïde,
- la maladie de Crohn,
- la sclérose en plaques,
- le choc septique,
- le cancer,
- la cachexie associée à une immunodéficience.

L'invention a plus particulièrement pour objet l'utilisation d'un (ou plusieurs) composé(s) et/ou d'un (ou plusieurs) mélange(s), tels que décrits ci-dessus, pour la préparation d'un médicament ayant la propriété d'inhiber la production de TGFα, ce médicament étant destiné au traitement de pathologies humaines ou animales dans lesquelles le TGFα est impliqué, telles que :
- le cancer,
- le psoriasis,
- l'eczéma,
- la formation de chéloïdes,
- la rétinopathie diabétique,
- l'athérosclérose,
- les maladies inflammatoires.

D'une manière générale, les différentes pathologies susceptibles d'être traitées dans le cadre de la présente invention peuvent être classées de la façon suivante :
I. Syndrome de réponse inflammatoire systémique, dont :
   - les sépticémies, notamment à germe gram positif, à germe gram négatif, fongiques, ou la méningococcémie,
   - les traumatismes et hémorragies,
   - les brûlures,
   - les expositions aux radiations ionisantes,
   - la pancréatite aigüe,
   - le syndrome de détresse respiratoire chez l'adulte.
II. Les blessures de reperfusion, telles que l'ischémie de reperfusion.
III. Les maladies cardiovasculaires, telles que :
   - l'infarctus du myocarde,
   - l'insuffisance cardiaque congestive.
IV. Les maladies infectieuses, dont :
   - le SIDA,
   - la méningite,
   - l'hépatite,
   - l'arthrite;
   - la périarthrite,
   - la pneumonie,
   - l'épiglottite,
   - l'infection à E. Coli 0157:H7,
   - le syndrome urémique hémolytique,
   - le purpura thrombocytopénique thrombolytique,
   - le paludisme,
   - la dengue hémorrragique,
   - la leishmaniose,
   - la lèpre,
   - le choc septique,
   - la myosite à streptoccoque,
   - la gangrène gazeuse,
   - la tuberculose,
   - l'orchite,
   - la maladie du légionnaire,
   - la maladie de lyme,
   - la grippe,
   - la mononucléose infectieuse dans le lymphome de Burkitt,
   - le cancer du rhinopharinx,
   - l'encéphalite virale.
V. En obstétrie, gynécologie, dont :
   - l'accouchement prématuré,
   - la fausse couche,
   - la stérilité.
VI. Les maladies inflammatoires autoimmunes, dont :
   - l'arthrite rhumatoïde, et les arthropathies séronégatives,
   - l'ostéoarthrite,
   - la maladie de Crohn, colite ulcéreuse,
   - le lupus érythémateux,
   - l'iridocyclite, l'uvéite et l'inflammation du nerf optique,
   - la fibrose pulmonaire idiopathique,
   - la vascularite systémique et la granulomatose de Wegener,
   - la sarcoïdose,
   - l'orchite.
VII. Les maladies allergiques et atopiques, dont :
   - l'asthme,
   - les rhinites allergiques,
   - l'eczéma,
   - la dermatite de contact allergique,
   - la conjonctivite allergique,
   - la pneumonite d'hypersensibilité.
VIII. Les maladies malignes, dont :
   - la leucémie lymphoblastique aigüe,
   - la leucémie monocytaire aiguë,
   - la leucémie myéloïde chronique,
   - la leucémie lymphocytaire chronique,
   - la maladie d'Hodgkin,
   - la spiénomégalie myéloïde,
   - le sarcome de Kaposi,
   - le carcinome colorectal,
   - l'histiocytose maligne,
   - le syndrome paranéoplastique et l'hypercatiémie des maladies malignes.
IX. Les transplantations, dont :
   - le rejet de greffe,
   - la réaction du greffon contre l'hôte.
X. La cachéxie.
XI. Les maladies congénitales, dont :
   - la mucoviscidose,
   - la lymphohistiocytose familiale,
   - l'anémie drépanocytaire.
XII. Les maladies dermatologiques, dont :
   - le psoriasis,
   - l'allopécie.
XIII. Les maladies neurologiques, dont :
   - la sclérose en plaques,
   - les maux de têtes.
XIV. Les maladies rénales, dont
   - le syndrome néphrétique,
   - l'hémodialyse,
   - l'urémie.
XV. Les traitements toxiques, dont :
   - la thérapie à OKT3,
   - la thérapie anti-CD3,
   - la thérapie à la cytokine,
   - la chimiothérapie,
   - la radiothérapie,
   - l'intoxication chronique aux salicylates.
XVI. Les maladies métaboliques, idiopathiques, dont :
   - la maladie de Wilson,
   - l'hémochromatose,
   - la déficience en α1-antitrypsine;
   - les diabètes,
   - la thyroïdite d'Hashimoto,
   - l'ostéoporose.

L'invention a également pour objet les procédés de préparation des composés ou des mélanges décrits ci-dessus, et faisant l'objet de la description qui suit.

Les abréviations utilisées dans la description des procédés de préparation de l'invention et de la description détaillée de la partie expérimentale ci-après, sont les suivantes ;
- AcOEt: Acétate d'éthyle
- AD-mix α: Mélange α de dihydroxylation asymétrique
- AD-mix β: Mélange (3 de dihydroxylation asymétrique
- Ar: Aromatique
- Buli: Butyl lithium
- CH₂Cl₂: Dichlorométhane
- DCC: Dicyclohexyle carbodiimide
- DIPEA: Diisopropyl éthylamine
- DMF: Diméthylformamide
- Et₂O: Ether diéthylique
- HMPT: Hexaméthylphosphoretriamide
- HOBT: Hydroxybenzotriazole
- LDA: Diisopropyle amidure de lithium
- LHMSA: Hexaméthyldisyle amidure de lithium
- MeOH: Méthanol
- Na₂SO₄: Sulfate de sodium
- NEt₃: Triéthylamine
- PyBop: Tris pyrrolidino benzotriazolyl oxyphosphonium, hexaflurophosphate
- tBuOH: Tert-butanol
- THF: Tétrahydrofuranne
- THP: Tétrahydropyrane
- TMSCI: Chlorure de triméthylsilyle
- Tos: Paratoluènesulfonate
- WSC: Carbodiimide soluble dans l'eau

Les composés selon l'invention dans laquelle Y est un groupe CONHOH (encore désignés ci-après composés de formule XV) peuvent être obtenus selon le schéma I suivant : 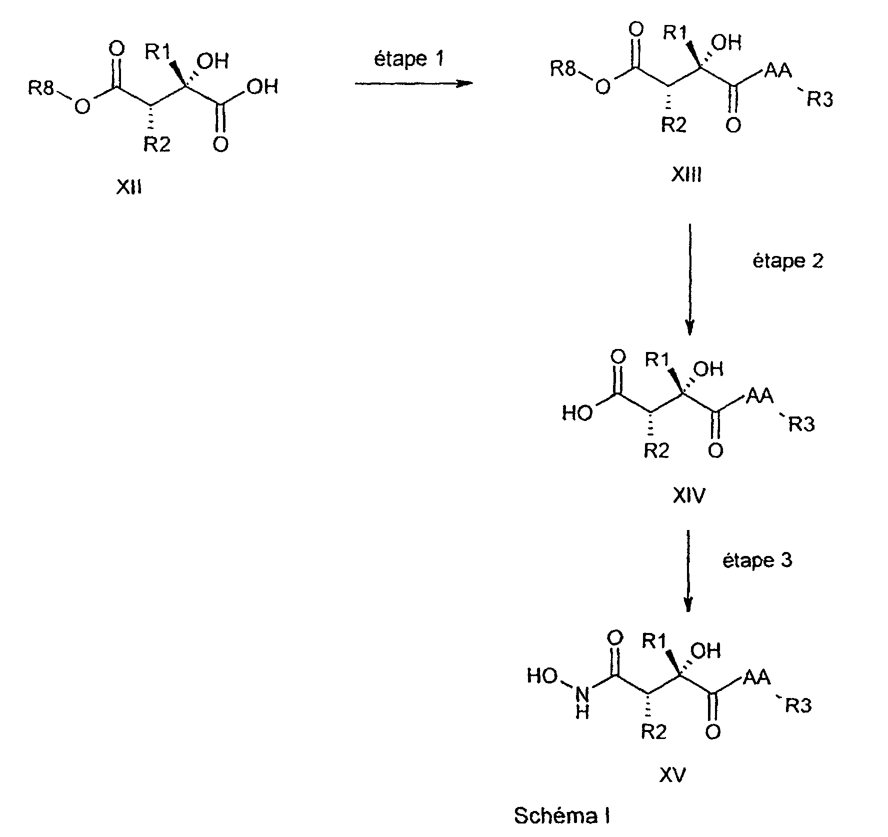 dans lequel :
- l'étape I consiste à condenser l'acide α-hydroxysuccinique XII (où R₈ est un groupe protecteur compatible avec les différents éléments de la molécule tel que t-butyle ou benzyle) avec un reste AA-R₃ où AA et R₃ sont tels que définis précédemment, par une méthode de couplage utilisée en synthèse peptidique et de préférence le PyBop à température ambiante durant 1 à 24 h (dans le cas où R₂ = OH, les alcools peuvent être protégés au préalable avec par exemple un dérivé silylé),
- l'étape 2 consiste à hydrolyser l'ester XIII, obtenu à I' étape précédente en acide carboxylique XIV avec de l'acide trifluoroacétique, notamment à température ambiante dans un solvant tel que CH₂Cl₂ durant 1 à 10 h lorsque R₈ est t-butyle, ou à hydrogénolyser l'ester XIII en acide XIV avec par exemple H₂ Pd/C lorsque R₈ est benzyle (notamment sous pression atmosphérique dans un solvant polaire tel que l'éthanol durant 30 minutes à 10 h),
- dans l'étape 3, l'acide hydroxamique XV est formé par réaction de l'hydroxylamine, l'hydroxylamine O protégée ou l'hydroxylamine N,O diprotégée, préférentiellement avec l'hydroxylamine O-THP ou l'hydroxylamine O-benzyle (lorsque R₂ est différent d'alkylidène, aryloxyméthyle et hétéroarylthiométhyle) en présence d'un réactif de couplage tel que DCC/HOBT ou WSC/HOBT à température ambiante dans un solvant tel que THF, CH₂Cl₂, ou DMF pendant 1 à 24 h (lorsque R₂ = OH, les alcools sont protégés au préalable avec par exemple TMSCI) ; les hydroxylamines O ou N-O (di)protégées sont ensuite déprotégées suivant la nature du groupe protecteur par exemple en milieu acide pour l'hydroxylamine O-THP (notamment à température ambiante dans un mélange THF-H₂O pendant 1 à 24 h) ou H₂ Pd/C pour l'hydroxylamine O-benzyle (notamment sous pression atmosphérique dans un solvant polaire tel que l'éthanol pendant 30 minutes à 10 h).

Les composés XIV et XV peuvent également être obtenus lorsque R₂ est différent de d'hétéroarylthiométhyle et R₁ différent d'hétéroarylalkyle par la suite des réactions du schéma 2 suivant : 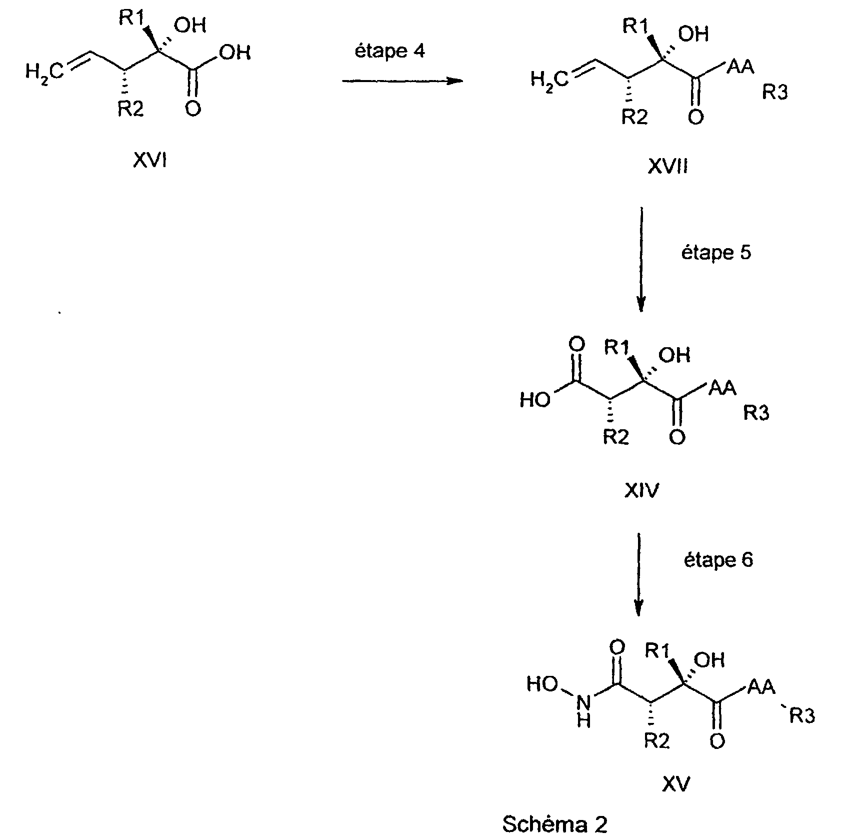 dans lequel :
- les étapes 4 et 6 sont réalisées comme dans les étapes 1 et 3 du schéma 1 respectivement, et à partir des composés XVI et XIV, ce qui conduit aux composés de formule XVII et XV respectivement,
- l'étape 5 consiste à oxyder la double liaison éthylénique du composé de formule XVII en acide notamment par ozonolyse (par exemple à -60°C dans CH₂Cl₂ jusqu'à obtention d'une coloration bleue persistante) puis oxydation (notamment à température ambiante avec NaClO₂ et NaH₂PO₄ dans tBuOH-H₂O durant 15 h) ou directement par KMnO₄/NalO₄ (notamment à température ambiante dans un mélange tBuOH-H₂O durant 1 à 10 h), ce qui conduit au composé de formule XIV

Les composés XIV et XV peuvent être également préparés selon la suite de réactions (sauf R₂ = OH, alkoxy ou benzyloxy) du schéma 3 suivant : 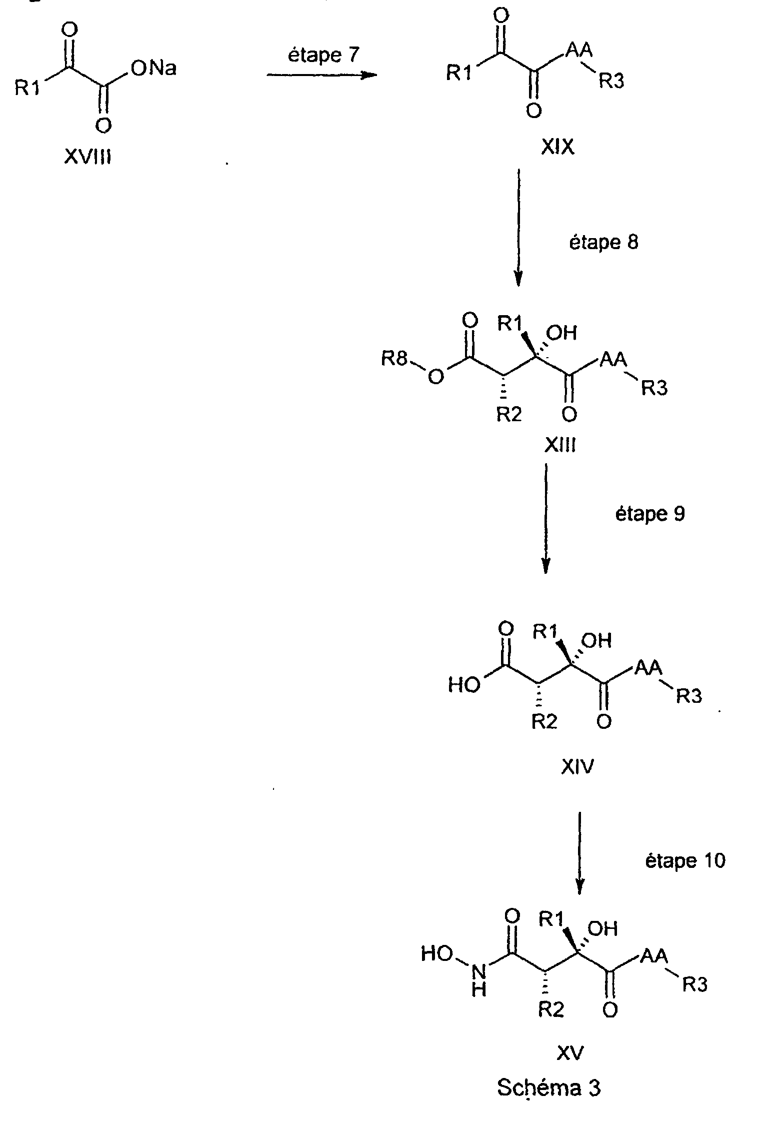 dans lequel :
- les étapes 9 et 10 sont identiques aux étapes 2 et 3 du schéma 1 et sont réalisées à partir des composés XIII et XIV respectivement, ce qui conduit aux composés XIV et XV respectivement,
- l'étape 7 consiste à faire réagir le sel de sodium d'un cétoacide XVIII avec AA-R₃ au moyen d'un agent de couplage, par exemple le chlorure d'oxalyle avec du DMF à température ambiante durant 1 à 10 h,
- l'étape 8 consiste en une réaction de Réformatsky (Rathke, Org. Reac. 22, 423-460, 1975) entre le composé XIX et un bromo-ester de formule : 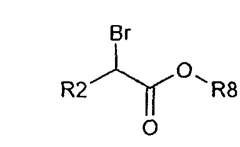 (dans lequel R₂ et R₈ sont identiques à précédemment) en présence de zinc (notamment dans un mélange benzène-éther diéthylique, à 80°C pendant 1h30) ; cette réaction conduit à un mélange de stéréoisomères qui peuvent être séparés par exemple par une méthode chromatographique pour fournir les composés XIII.

Les acides succiniques XII peuvent être préparés par réaction de Réformatsky réalisée comme précédemment selon le schéma 4 suivant entre d'une part un composé de formule R₂-CHBr-CO-OR₈ dans laquelle R₂ est tel que défini ci-dessus (sauf R₂ = OH, alkoxy, benzyloxy) et R₈ est tel que défini ci-après, et, d'autre part, un composé de formule XX dans laquelle R₁ est tel que défini ci-dessus, et R₉ est tel que défini ci-après : 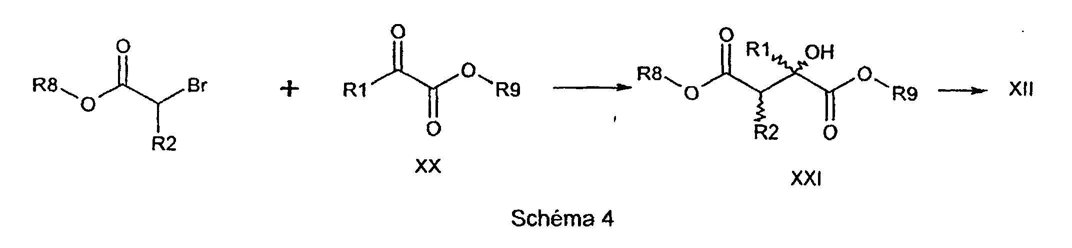 dans lequel R₈ et R₉ sont des groupes protecteurs d'acides carboxyliques qui peuvent être clivés de façon sélective : R₉ peut être par exemple un reste benzyle sensible à l'hydrogénolyse catalytique et

R₈ un groupe éthyle saponifiable ou t-butyle sensible à l'hydrolyse acide.

Dans ces conditions, la réaction fournit un mélange des quatre diastéréoisomères de formule XXI qui peuvent être séparés par exemple par une méthode chromatographique pour fournir les composés XII.

Les composés XII peuvent également être obtenus par les oxazolidinones d'Evans (J. Am. Chem.

Soc. 104, 1737-1739, 1982; J. Am. Chem. Soc. 112, 8215-8216, 1990) selon la suite de réactions (sauf R₂ = OH, alkoxy ou benzyloxy) du schéma 5 suivant : 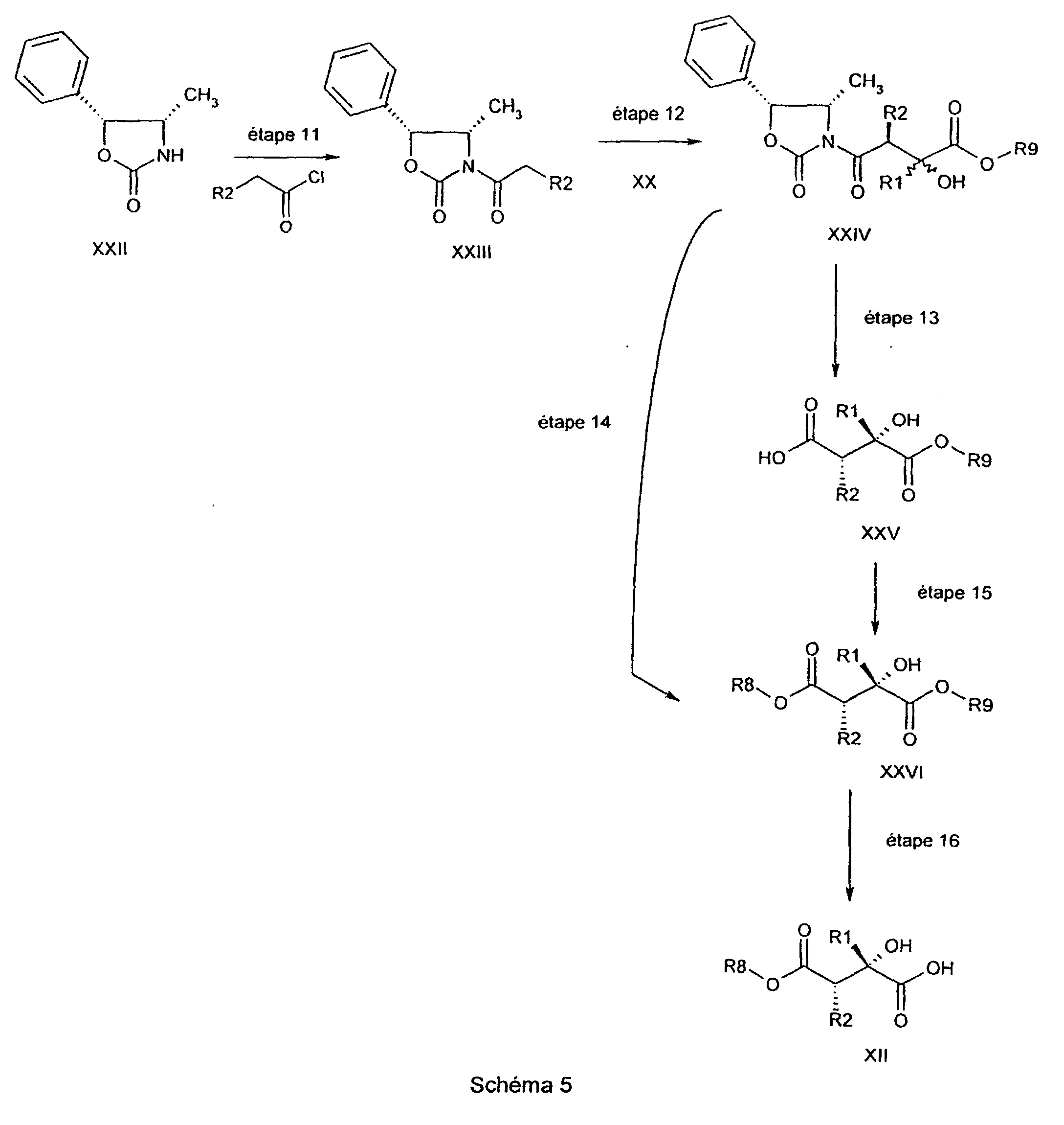 dans lequel :
- l'étape 11 consiste à acyler l'oxazolidinone XXII préalablement traitée au Buli (notamment à -70°C dans le THF) ou au NaH (notamment à 0°C dans le THF) par un chlorure d'acide 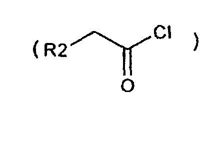 (notamment à température ambiante pendant 15 h), dans lequel R₂ et tel que défini ci-dessus,
- l'étape 12 consiste à condenser l'énolate du dérivé XXIII (préparé par l'action d'une base, par exemple LDA, LHMSA à -60°C dans le THF pendant 30 minutes ou un acide de Lewis, par exemple TiCl₄ à 0°C dans CH₂Cl₂ pendant 1 h) avec un cétoester XX, notamment à -60°C dans le THF ou CH₂Cl₂ pendant 2 h, R₉ étant un groupe protecteur d'acide carboxylique (chiral ou non) compatible avec l'étape suivante ; dans ces conditions, la réaction conduit à un mélange de stéréoisomères qui peuvent être séparés par exemple par une méthode chromatographique,
- l'étape 13 consiste à cliver la copule chirale du composé XXIV avec une base aqueuse par exemple KOH aqueux (2N) compatible avec R₉ soit par LiOOH (préparé à partir de LiOH + H₂O₂) de manière à obtenir l'acide carboxylique XXV, notamment dans un mélange THF-H₂O à température ambiante pendant 1h30,
- l'étape 14 consiste à cliver la copule du composé XXIV avec une base organique par exemple MeOMgBr, LiOBr, notamment dans le THF à 0°C pendant 1h30, compatible avec R₉ de façon à obtenir l'ester directement,
- l'étape 15 consiste à protéger l'acide carboxylique du composé XXV avec un groupe protecteur R₈; R₈ et R₉ doivent pouvoir être clivés sélectivement, par exemple R₈ = t-butyle (isobutène dans CH₂Cl₂ en présence d'une quantité catalytique d'acide tel que l'acide sulfurique à température ambiante dans un récipient fermé pendant 1 à 24 h) et R₉ = benzyle (K₂CO₃ dans l'acétonitrite en présence d'un halogénure de benzyle à 80°C pendant 1 à 10 h),
- l'étape 16 consiste à hydrolyser le groupe protecteur R₉ du composé XXVI soit en milieu basique par exemple NaOH aqueux ou en milieu acide par exemple acide trifluoroacétique ou à hydrogénoliser par exemple H₂ Pd/C, dépendant de la structure R₉ de façon à obtenir les acides succiniques de la même manière que précédemment.

Une méthode d'obtention des composés de structure XVI consiste à réaliser une réaction d'aldolisation à partir d'un céto-ester XXVII et d'un alcène XXVIII (notamment en présence d'un acide de Lewis tel que SnCl₄ à -80°C dans un solvant tel que CH₂Cl₂ durant 5 minutes à 2 h), ou à partir d'un céto-acide (sous forme de sel de sodium ou triéthylamine) XXX et un d'alcène XXXI (notamment à température ambiante entre 1 et 10 h dans un mélange THF-H₂O) selon le schéma 6 suivant : 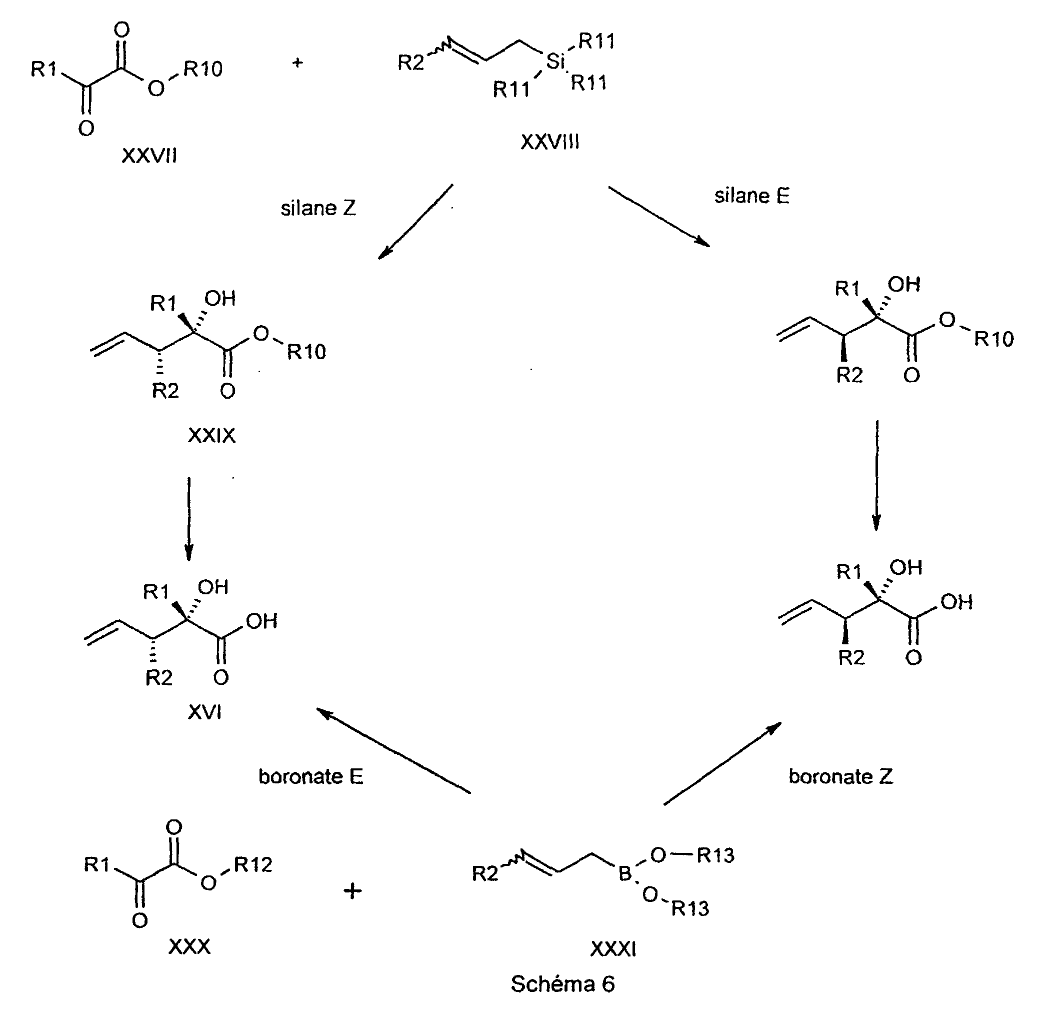 dans lequel :
- R₁ et R₂ ont la même signification que dans le schéma 2,
- R₁₀ est un alkyle éventuellement ramifié en C₁-C₁₂, un benzyle ou un composé optiquement pur tel que l'acide mandélique estérifié par un alkyle en C₁-C₃ linéaire ou ramifié, ou un benzyle,
- R₁₁ est un alkyle en C₁-C₃ linéraire ou ramifié, ou un chlore,
- R₁₂ est le sodium ou la triéthylamine,
- R₁₃ est l'hydrogène, un alkyle en C₁-C₃ linéaire ou ramifié ; R₁₃ peut également représenter un enchaînement formant un cycle avec l'atome de bore tel que par exemple le di-isopropyltartrate,
- les réactions sont diastéréosélectives et conduisent aux dérivés de stéréochimie XVI si la double liaison est de géométrie Z pour les composés XXVIII et de géométrie E pour les composés XXXI.

Une méthode plus particulièrement préférée consiste à prendre comme substituant R₁₀ un composé optiquement pur tel que l'acide mandélique éthyle ester, ce qui permet l'obtention des composés XVI optiquement purs.

Les composés XXIX et XVI peuvent également permettre l'obtention de l'acide XII selon le schéma réactionnel 7 suivant : 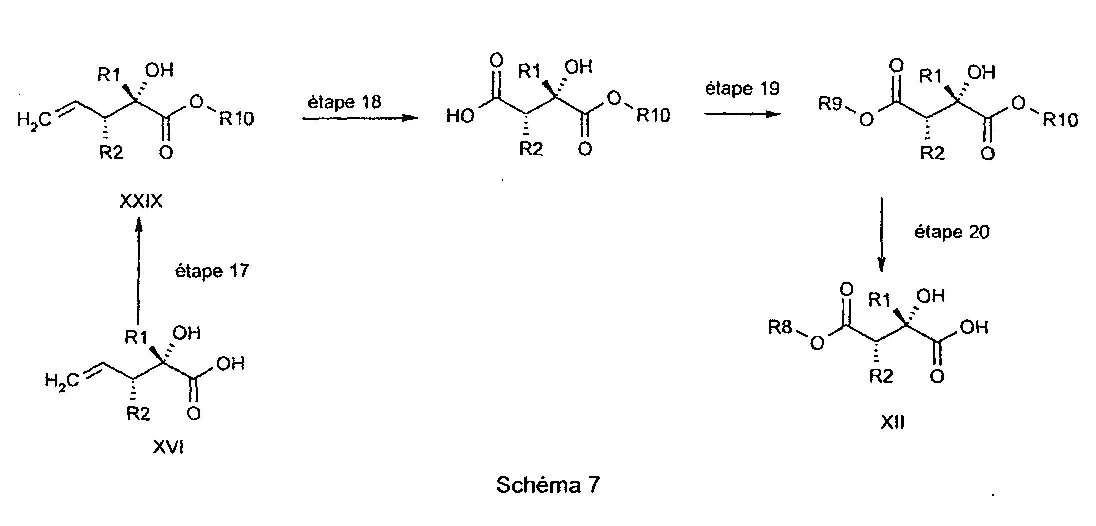 dans lequel :
- l'étape 17 consiste à estérifier le composé XVI de façon à obtenir un des composés XXIX, par exemple un ester de benzyle ou mandélique avec PyBop,
- l'étape 18 consiste à oxyder la double liaison du composé XXIX de façon identique à l'étape 5 du schéma 2,
- l'étape 19 consiste à protéger l'acide carboxylique avec un groupe R₉ compatible avec la déprotection du groupe R₁₀, par exemple R₉ est t-butyle lorsque R₁₀ est le mandélate d'éthyle,
- l'étape 20 consiste à déprotéger l'acide portant R₁₀ de façon identique à l'étape 16 du schéma 5, ce qui conduit à l'obtention du composé de formule XII.

Les céto-acides ou esters XX, XXVII, XXX lorsqu'ils ne sont pas commerciaux peuvent être préparés par le shéma réactionel 8 suivant : 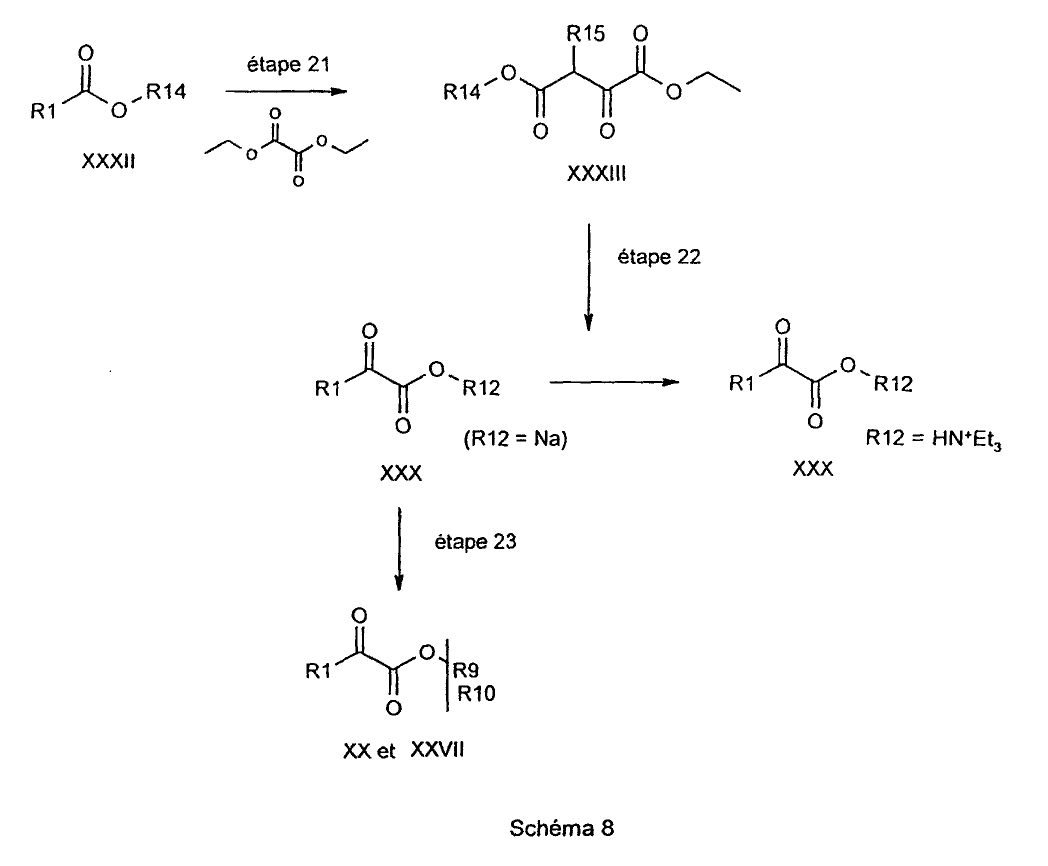 dans lequel :
- R₁₄ est un alkyle en C1-C3 linéaire ou ramifié,
- R₁₅ est R₁ moins un carbone,
- R₉, R₁₀ et R₁₂ sont comme précédemment,
- l'étape 21 consiste à faire réagir sur l'ester correspondant en présence d'une base, par exemple tBuOK, l'oxalate de diéthyle (addition de l'oxalate de diéthyle sur tBuOK, dans l'éther diéthylique, à t < 10°C puis addition de l'ester à température ambiante et agitation à cette température 15 h),
- l'étape 22 consiste à hydrolyser à chaud en milieu acide, par exemple acide sulfurique 5N, les esters puis neutraliser par une base, par exemple la soude pour obtenir le produit XXX (R₁₂ = Na⁺),
- le composé XXX (R₂ = HN⁺Et₃) est aisément obtenu par traitement du sel de sodium par du chlorhydrate de triéthylamine,
- l'étape 23 consiste à estérifier le composé XXX (R₁₂ = Na+) par les méthodes classiques d'estériftcation, par exemple, chlorure d'oxalyle, DMF.

Les dérivés silylés XXVIII de géométrie Z peuvent être obtenus selon le schéma 9 suivant 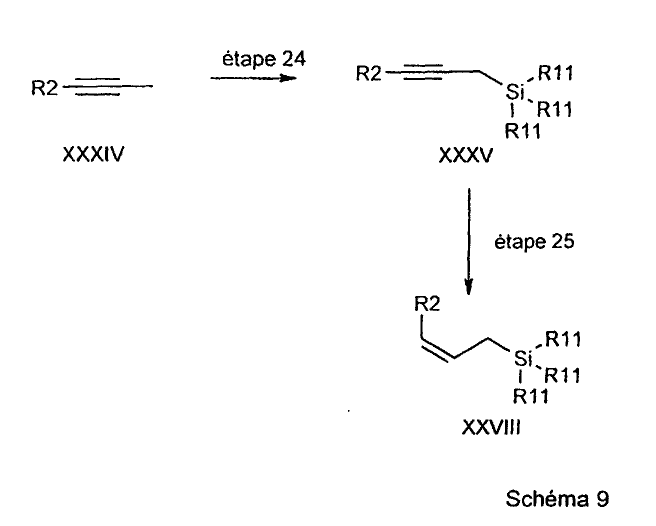 dans lequel :
- R2 est tel que décrit dans le schéma 2,
- R11 est tel que décrit plus haut,
- l'étape 24 consiste à réaliser une alkylation d'un alcyne au moyen d'une base par exemple t-Buli (notamment dans l'éther diéthylique à -70°C), avec ClSi(R₁₁)₃ (notamment à -70°C dans l'éther diéthylique puis à température ambiante entre 5 et 45 minutes),
- l'étape 25 consiste à réduire la triple liaison en double liaison par un réducteur tel que hydrogène en présence d'un catalyseur tel que Nickel acétate/NaBH4 dans l'éthanol à pression atmosphérique pendant 2 h.

Une autre méthode pour obtenir le dérivé XXVIII (R2 = CH₃) est décrite ci-après dans le schéma 10 : 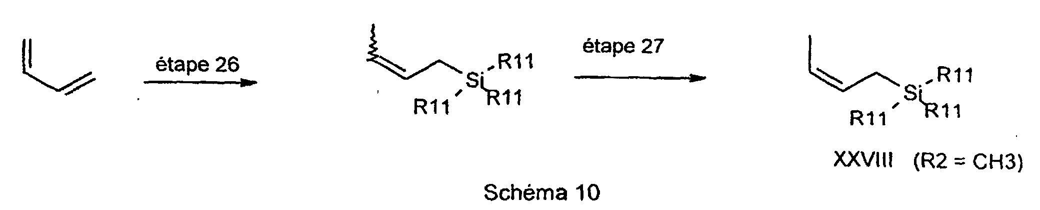 dans lequel :
- R11 est tel que décrit précédemment,
- l'étape 26 consiste à faire réagir un alkylsilane avec le butadiène en présence de triéthylaluminium et de Ni acétylacétonate dans un récipient fermé, notamment à 60°C durant 5 à 20 h,
- le composé obtenu est un mélange E + Z,
- l'étape 27 consiste à isomériser la double liaison par chauffage puis à distiller le produit.

Les acides hydroxamiques pour lesquels R2 = OH ou alkoxy ou benzyloxy peuvent être obtenus selon le schéma 11 suivant : 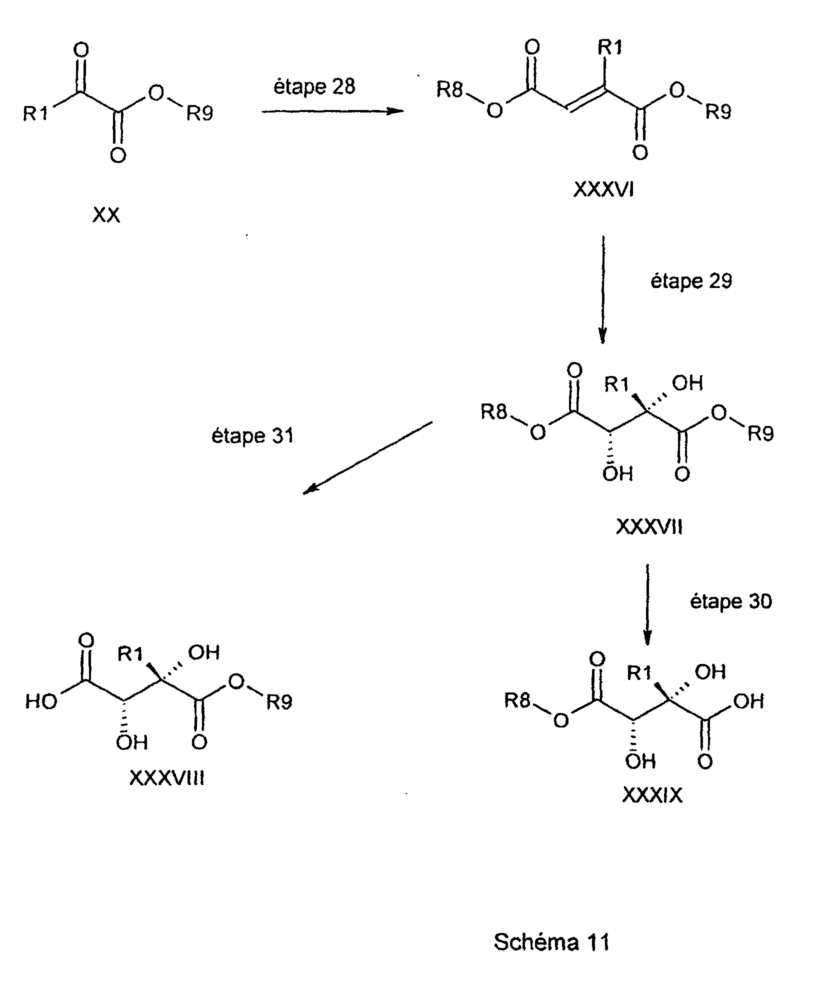 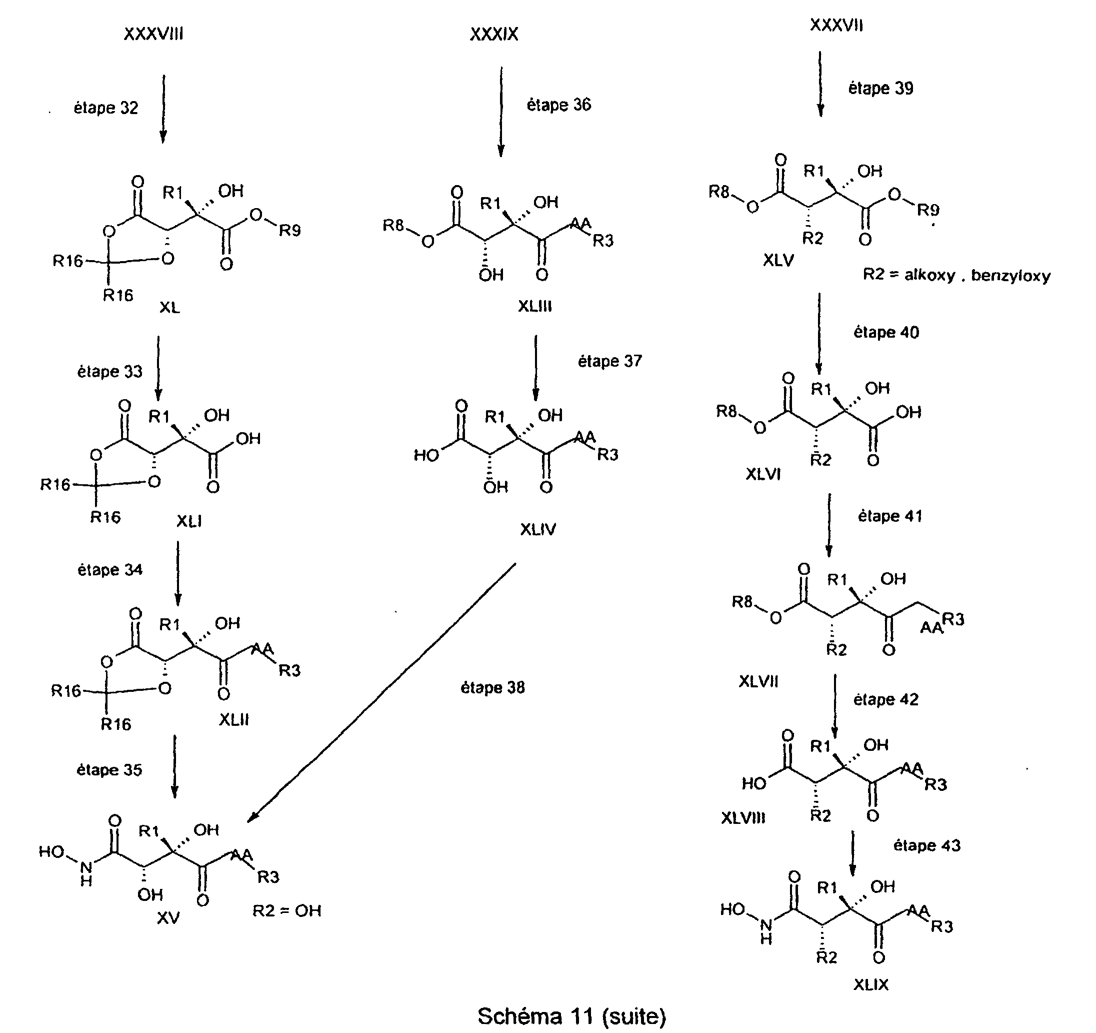 dans lequel :
- R₁, R₈, R₉ sont tels que décrits dans le schéma 4 sauf lorsque R₂ = benzyloxy. R₈ et R₉ ne peuvent être clivés par hydrogénolyse,
- R₁₆ sont des alkyles en C₁-C₅ linéaires ou ramifiés ou représente un enchaînement et forme un cycle avec les deux atomes d'oxygène,
- l'étape 28 consiste à réaliser une réaction de Wittig entre les composés XX et un sel de phosphonium de structure : 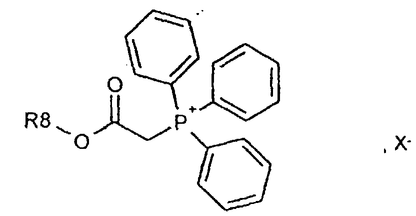 (X étant un halogène) dans le DMF à température ambiante durant 1 à 10 h ou un phosphonate de structure : 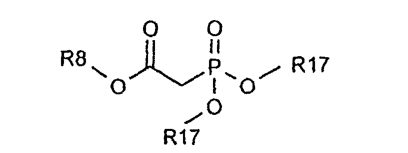 R₁₇ étant un alkyle en C₁-C₃, linéaire ou ramifié,
   cette réaction permettant de conduire à un mélange d'alcènes E et Z qui doivent être séparés, par exemple, par une méthode chromatographique de manière à obtenir un composé XXXVI de géométrie E,
- l'étape 29 consiste à réaliser une hydroxylation asymétrique de Sharpless (Chem. Rev. 2483-2547. 1994) en présence d'AD-mix β et de méthanesulfonamide, notamment dans un mélange tBuOH-H₂O à température ambiante durant 1 à 10 h ; cette réaction conduit à un produit optiquement pur ; l'utilisation d'AD-mix α ou β sur l'alcène Z conduit aux deux autres diastéréoisomères.
- l'étape 30 est identique à l'étape 16 du schéma 5,
- l'étape 31 consiste à hydrolyser l'ester en acide carboxylique en milieu basique ou acide dépendant de la structure de R₈,
- l'étape 32 consiste à protéger l'α-hydroxyacide sous forme de dioxolane en le faisant réagir avec un acétal, par exemple 2,2-diméthoxypropane dans le DMF à 50°C, 15 h,
- l'étape 33 consiste à déprotéger par hydrogénolyse l'ester, le groupe R₉ dans ce cas est exclusivement de type benzylique pour être compatible avec le dioxolane qui ne supporte pas le milieu acide ou basique aqueux,
- l'étape 34 consiste à coupler l'amino-acide AA-R₃ par une méthode compatible avec le dioxolane, par exemple PyBop comme précédemment,
- l'étape 35 consiste à substituer le dioxolane par l'hydroxylamine, notamment dans un mélange MeOH-H₂O à -20°C pendant 1 à 15 minutes,
- les étapes 36, 37 et 38 sont identiques aux étapes 1,2 et 3 du schéma 1,
- l'étape 39 consiste à alkyler l'alcool secondaire en le faisant réagir avec une base, par exemple NaH, puis un électrophile, par exemple, un halogénure d'alkyle ou de benzyle, notamment dans le THF à température ambiante durant 1 à 10 h,
- l'étape 40 est identique à l'étape 16 du schéma 5,
- les étapes 41, 42 et 43 sont identiques aux étapes 1, 2 et 3 du schéma 1.

Les composés selon l'invention dans laquelle Y = SH, 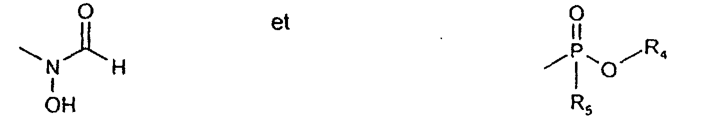 peuvent être obtenus selon le schéma 12 suivanrt : 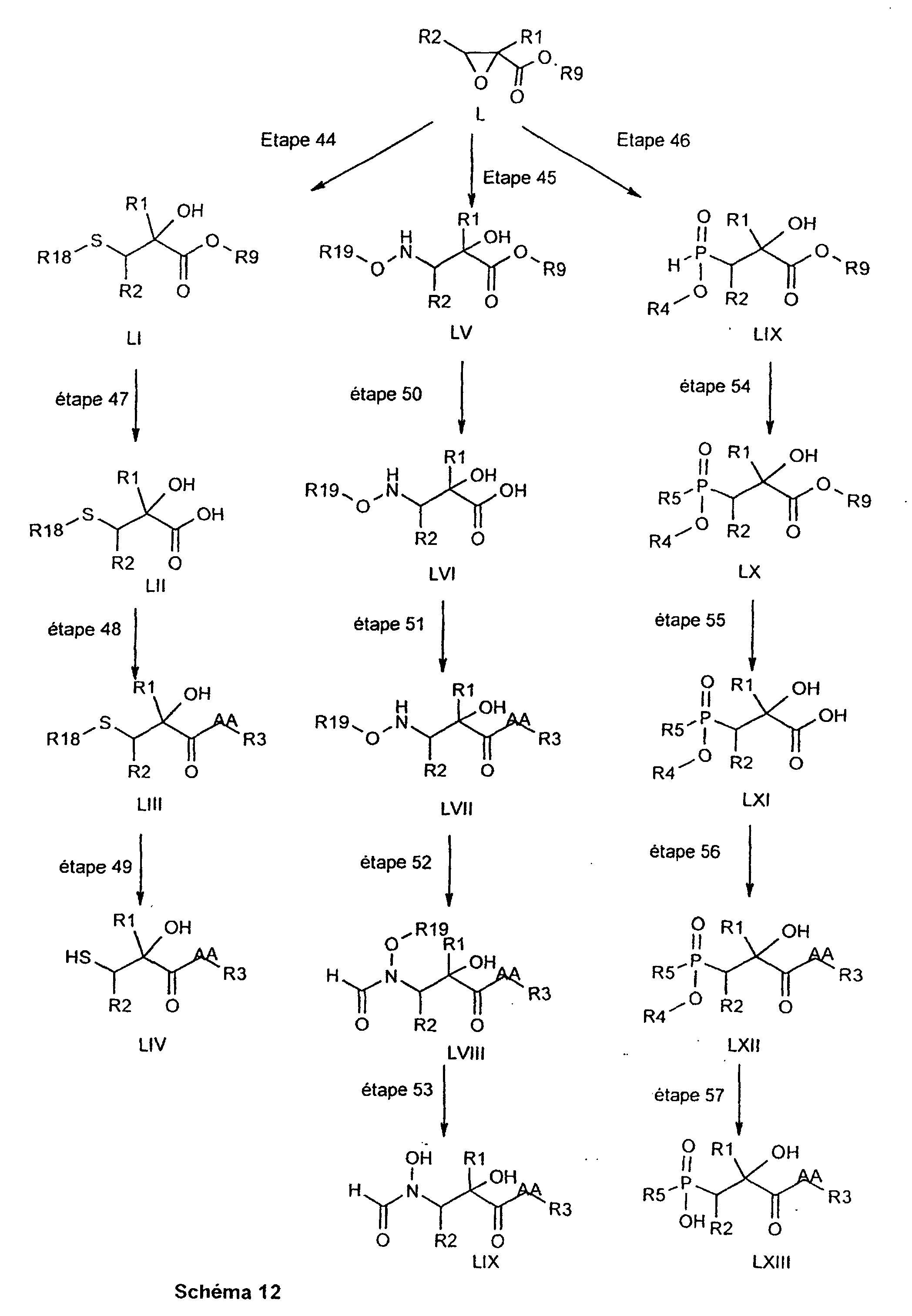 dans lequel :
- l'étape 44 consiste à ouvrir un époxyde de formule L dans laquelle R₁ et R₂ sont tels que définis ci-dessus, et R₉ est un groupe protecteur d'acide carboxylique, notamment un reste benzyle sensible à f'hydrogénolyse catalytique, cette ouverture de l'epoxyde L étant effectuée par un nucléophile par exemple un thiol protégé par un groupe R₁₈ compatible avec R₉, par exemple un benzyle dans le méthanol durant 1 h à 60°C,
- l'étape 47 consiste à déprotéger l'ester LI, par exemple avec de l'acide trifluoroacétique comme précédemment,
- l'étape 48 est identique à l'étape 1 du schéma 1, et réalisé à partir du compose LII obtenu à l'étape précédente,
- l'étape 49 consiste à déprotéger le soufre par exemple avec du sodium dans l'ammoniac liquide, notamment à -60°C durant 5 à 15 minutes,
- l'étape 45 consiste à ouvrir l'époxyde L par de l'hydroxylamine protégée telle que définie précédemment, par exemple, R₁₉ = benzyle ou THP comme précédemment,
- l'étape 50 consiste à déprotéger l'ester LV par une méthode compatible avec R₁₉,
- l'étape 51 est identique à l'étape 48, et réalisée à partir du composé LVI obtenu à l'étape précédente,
- l'étape 52 consiste à faire réagir l'hydroxylamine LVII avec de l'acide formique et de l'anhydride acétique, notamment à une température d'au moins 100°C durant 1 à 15 h,
- l'étape 53 consiste à cliver R₁₉ sur le composé LVIII avec par exemple H₂ Pd/C ou HCI 1N dépendant de la structure de R₁₉ comme précédemment,
- l'étape 46 consiste à ouvrir un époxyde L par de l'acide hypophosphoreux de formule H₃PO₂ puis esterification avec un agent de couplage tel que DCC et un groupe R₄OH dans lequel R₄ est tel que défini ci-dessus, en présence par exemple de triméthylorthoformate et de tétraméthylguanidine à température ambiante pendant 5 h,
- l'étape 54 consiste à traiter le composé LIX avec un composé de formule : 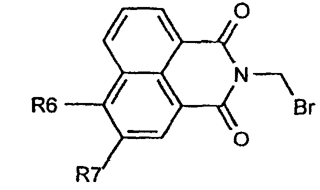 (préparé selon les méthodes décrites dans la littérature) dans laquelle R₆ et R₇ sont tels que définis ci-dessus, notammant dans CH₂Cl₂ en présence de bis triméthylsilyle acétamide à température ambiante pendant 5 h,
   ce qui conduit à l'obtention du composé de formule LX dans laquelle R₅ représente : 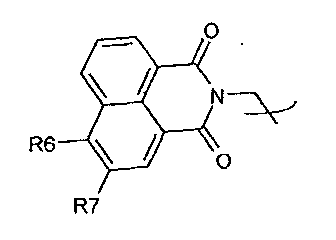
- l'étape 55 consiste à cliver l'ester R₉ par une méthode compatible avec R₄ comme précédemment.
- l'étape 56 est identique à l'étape 48, et réalisée à partir du composé LXI obtenu à l'étape précédente
- l'étape 57 consiste à cliver le groupe R₄ du composé LXII obtenu à l'étape précédente, par exemple, à l'aide de Nal dans l'acétone au reflux durant 15 h.

Les composés du schéma 12 sont des mélanges de diastéréoisomères ou optiquement purs, dépendant du composé L de départ. Les mélanges de diastéréoisomères pourront être séparés par exemple par une méthode chromatographique.

Préparation des composés L de façon racémique selon le schéma 13 suivant : 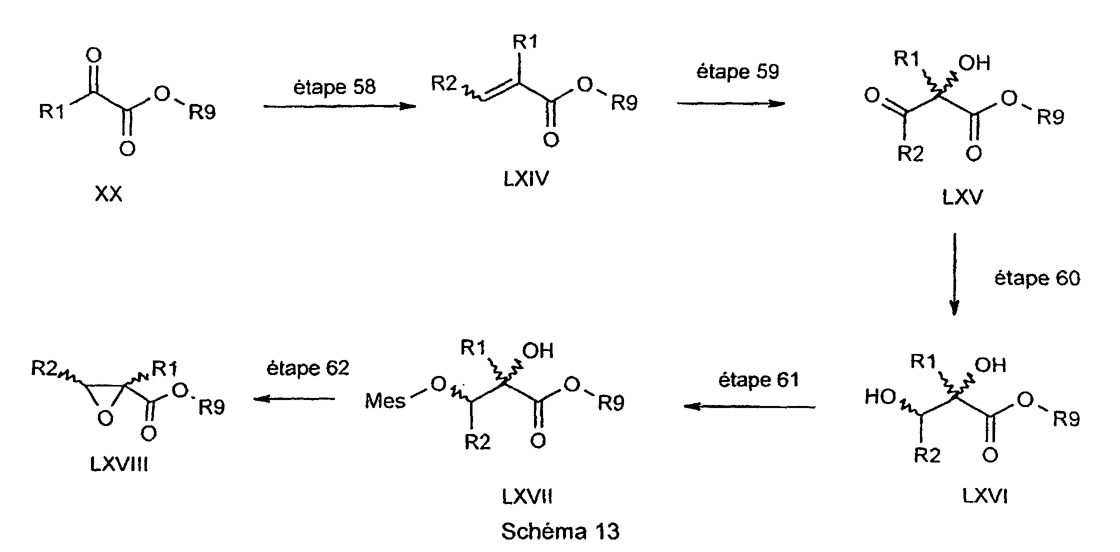 dans lequel :
- R₁, R₂, R₉ sont tels que décrits précédemment,
- l'étape 58 consiste à réaliser une réaction de Wittig entre un sel de phosphonium 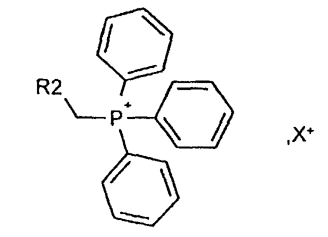 (R₂ et X⁻ sont identiques à précédemment) et le composé XX en présence d'une base, par exemple, BuLi dans le THF à une température comprise entre 0°C et 60°C durant 1 h ; l'oléfine LXIV obtenue est un mélange E et Z qui peuvent être séparés, par exemple, par une méthode chromatographique,
- l'étape 59 consiste à oxyder la double liaison avec par exemple KMnO₄-acide acétique dans l'acétone à une température de -10°C durant 1h30,
- l'étape 60 consiste à réduire le carbonyle avec un réducteur par exemple NaBH₄ dans l'éthanol à 0°C durant 15 minutes,
- l'étape 61 consiste à transformer l'alcool secondaire en groupe partant en le faisant réagir avec par exemple le chlorure d'acide méthane-sulfonique en présence d'une base, par exemple NEt₃ dans l'éther diéthylique à 0°C durant 1 h,
- l'étape 62 consiste à traiter le composé LXVIII avec une base, par exemple, NaH pour former l'époxyde dans le DMF à température ambiante durant 1 à 3 h.

L'oléfine LXIV de géométrie E peut également être obtenue par la suite de réactions du schéma 14 suivant : 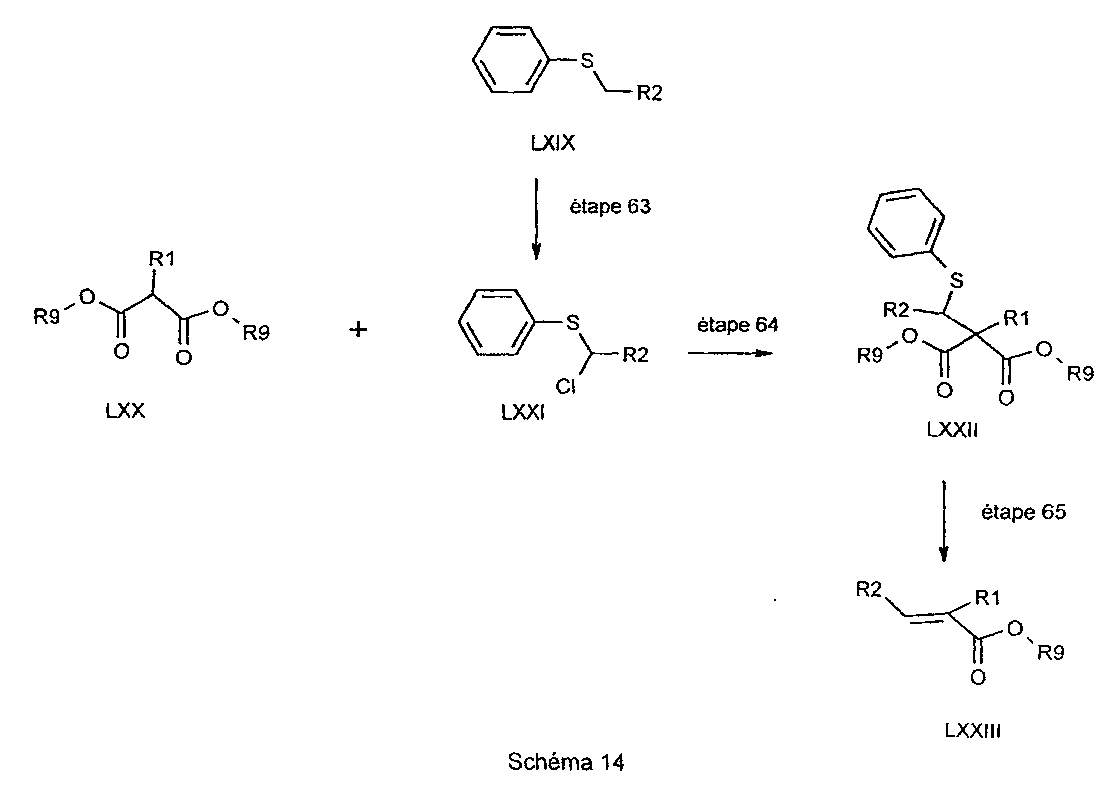 dans lequel :
- l'étape 63 consiste à réaliser une chloration du composé LXIX avec par exemple le chlorure de sulfuryle dans le dichlorométhane à 35°C pendant 30 minutes,
- l'étape 64 consiste à réaliser une alkylation en présence d'une base, par exemple NaH dans un mélange THF-HMPT à température ambiante pendant 15 h,
- l'étape 65 consiste à faire une déalcoxycarbonylation suivie d'une élimination avec par exemple LiCl en chauffant dans un solvant tel que DMF, DMSO ou HMPT.

Le produit obtenu dans ces conditions est de géométrie E.

Préparation de l'époxyde optiquement pur selon le schéma 15 suivant : 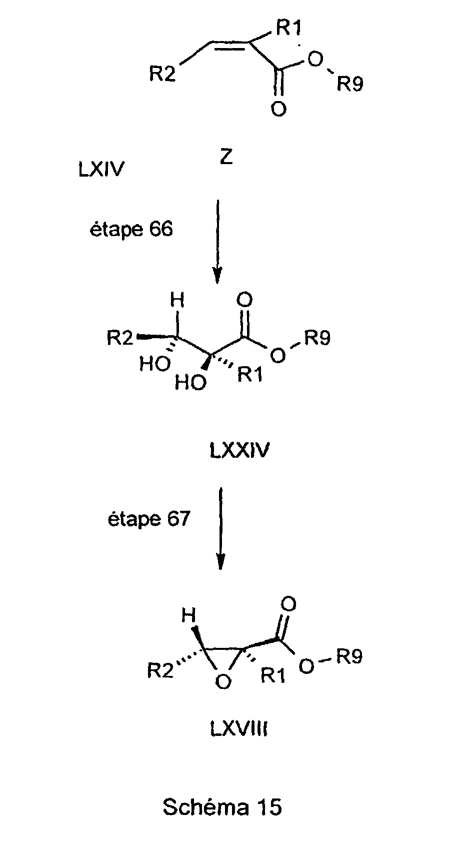 dans lequel:
- l'étape 64 consiste à réaliser une dihydroxylation asymétrique comme précédemment à l'aide d'AD-mix β pour conduire au composé LXXIV ; l'énantiomère peut être obtenu par AD-mix α et les deux autres diastéréoisomères à partir de l'oléfine E et AD-mix β ou α,
- l'étape 67 est identique aux étapes 61 et 62 du schéma 13.

### Partie expérimentale

### Intermédiaire 1 : Acide 2(S(*) hydroxy-2 (S*)-(3-méthylpropyl) 3 (R*) méthyl pent-4-ènoïque

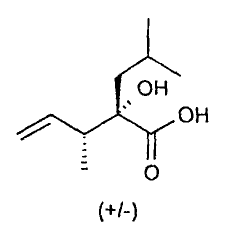

### Méthode A :

### a) Acide (E) -but-2-enyl boronique bis (methyl-2-propyl ester)

A 23,8 g (212 mmoles) de tBuOK dans 175 ml de THF sec refroidi à -78°C, ajouter 21 ml (230 mmoles) de trans-2-butène. Ajouter ensuite en 1h30, 98 ml (212 mmoles) de nBuli (2.45 M dans l'hexane). En fin d'addition, agiter 1/2 h à -50°C. Refroidir à -78°C et ajouter 49 ml (212 mmoles) de triisopropyl borate, agiter 1/2 h.

Ajouter 200 ml d'HCl N saturé par NaCl. Extraire avec 4 fois 200 ml d'éther éthylique.

Rassembler les phases éthérées, sécher sur sulfate de sodium.

Ajouter 35,8 ml (467 mmoles) d'alcool isopropylique, 66 g de sulfate'de sodium anhydre et agiter 1 nuit à température ambiante.

Décanter le minéral, évaporer l'éther à 30°C à l'évaporateur rotatif (sous vide). Récupérer 17,95 g d'huile soit 45 %. A conserver sous azote. Eb : 30 °C /0,3 mmHg.
RMN (CDCl₃) : δ 5,5 (m, 2H, CH=CH) ; 4,4 (m, 2H, OCH-(Me)₂) ;
1,7 (m, 5H, CH₃-CH= et =CH-CH₂-B) ; 1,3 (m, 12H, O-CH-(CH₃)₂).

### b) Acide 2(S*) hydroxy-2(S*) 3-méthylpropyl 3(R*) méthyl pent-4-enoïque

Disperser 3,77 g (24,8 mmoles) de 4-méthyl-2-oxo-pentanoate de sodium dans 30 ml de CH₂Cl₂. Ajouter 30 ml d'HCI N saturé en NaCl. Extraire 3x15 ml de CH₂Cl₂. Rassembler et sécher sur Na₂SO₄ les phases organiques. Filtrer et introduire cette solution dans un ballon tricol 250 ml. Refroidir à -25°C, ajouter 3,48 ml (24,8 mmoles) de triéthylamine puis ajouter 4,57 g (24,8 mmoles) du produit a) et agiter une nuit à température ambiante. Verser sur HCI 6N, extraire par CH₂Cl₂, sécher sur Na₂SO₄. Filtrer. Evaporer.

Purifier par flash chromatographie sur 200 g de silice (éluant : CH₂Cl₂:MeOH ; 95:5).

Récupérer 2,73 g de solide blanc (rendement 60 %).
P. F.: 86°C
IR (CDCl₃) : ν CO : 1706 cm⁻¹ ; ν C=C : 1639 cm⁻¹
RMN (CDCl₃) : δ 5,8 (m, 1H, CH₂=CH) ; 5,15 (m, 2H, CH₂=CH-) ; 2,5 (m, 1H, =CH-CH(CH₃)-) ; 1,75 (m, 3H, CH₂-CH-(CH₃)₂ ); 1 (3d, 9H, CH₃).

### Méthode B

A 4,8 g (42,7 mmoles) de tBuOK dans 35 ml de THF à -78°C ajouter 4,2 ml (45,2 mmoles) de trans-2-butène. Sans dépasser -65°C ajouter 21,35 ml (42,7 mmoles) de nBuli 2 M dans l'hexane en 1h. En fin d'addition agiter 1/2 h à -50°C puis refroidir à nouveau à -78°C et additionner 9,85 ml
(42,7 mmoles) de triisopropyl borate et agiter 30 mn à -78°C.

Solubiliser 6,49 g (42,7 mmoles) de 4-méthyl-2-oxovalérate de sodium dans 15 ml d'eau. Ajouter cette solution au milieu réactionnel et agiter une nuit à température ambiante.

Acidifier par HCl 6N et extraire avec 3 fois 50 ml d'acétate d'éthyle.

Purifier par flash chromatographie sur 600 g de silice (éluant : CH₂Cl₂:MeOH:AcOH , 97:3:0,3). Récupérer 6,05 g de produit b) soit 77 %. RMN identique à b) de la méthode A.

### Intermédiaire 2 : (Z) but-2-enyl triéthylsilane

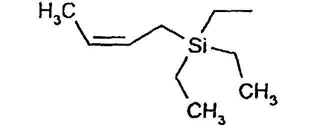

Dans un mini autoclave refroidi à - 20°C, introduire :
9,8 ml (113 mmoles) de butadiène
18 ml (113 mmoles) de triéthylsilane
60 mg (0,22 mmoles) de nickel acétylacétonate
0,32 ml (2,3 mmoles) de triéthylaluminium.

Agiter 24 h à 60°C. Distiller à 50-53°C sous 7 mmHg. Récupérer 12,7 g (67 %) de produit Z.
RMN (CDCl₃) : δ 5,4 (2H, m, -CH=CH); 1,6 (3H, m, CH₃-CH=); 1,55 (2H, m, HC=CH-CH₂₎ ;
1 (9H, m, 3CH₃) ; 0,5 (6H, m, 3CH₃Si).

### Intermédiaire 3 : 1(Z) hex-2-enyl triéthylsilane

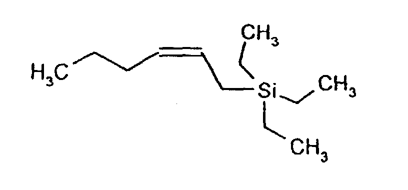

### a) 1(hex-3-ynyl) triéthylsilane

Sous atmosphère d'azote, introduire 8,8 ml (13 mmoles) de tBuli 1,45 M dans le pentane. Ajouter Et₂O (12 ml), 1,84 ml (12 mmoles) de TMEDA, 1,37 ml (12 mmoles) de 2-hexyne et laisser revenir à 0°C.

Agiter 1 h à 0°C puis refroidir de nouveau à -78°C et introduire 2,45 ml (15 mmoles) de chlorotriéthylsilane. Laisser revenir à +20°C en 45 mn environ.

Ajouter 20 ml d'eau. Extraire à l'éther diéthylique. Sécher. Evaporer. Distiller à 75 % sous 0,4 mmHg au four à boules. Récupérer 2,76 g (soit 100 %) de produit.
RMN (CDCl₃) : δ 2,15 (2H, m,-CH₂-C≡) ; 1,5 (4H, m, CH₂-CH₂-C≡C-CH₂-Si) ;
1 (12H, m, Si(CH₂-CH₃)₃ et CH₃-CH₂-CH₂) ; 0,65 (6H, m, Si(CH₂-CH₃)₃).

### b) 1-(Z)hex-2-enyl triéthylsilane

A 9,5 ml d'éthanol absolu contenant 0,5 ml de soude 2N ajouter 400 mg de NaBH₄. Agiter 10 mn. Filtrer dans 15 ml d'éthanol absolu contenant 370 mg (1,5 mmoles) d'acétate de Nickel, ajouter 1,5 ml (1,5 mmoles) de la solution filtrée. Mettre sous atmosphère d'hydrogène. Ajouter 2,35 g (12 mmoles) de produit **a)** et agiter 2 h à température ambiante. Filtrer sur célite. Concentrer. Distiller à 125°C/22 mmHg. Récupérer 1,64 g soit 68 %.
RMN (CDCl₃) : δ 5,4 et 5,25 (2 m, 2H, CH=CH); 2 (2H, m, CH₂-CH=) ; 1,55 (2H, d, =CH-CH₂-Si) ; 1,4 (m, 2H, CH₃-CH₂-CH₂-CH=) ; 1 (12H, m, CH₃-CH₂-CH₂ et Si(CH₂-CH₃)₃);
0,55 (6H, m, Si(CH₂-CH₃)₃).

### Intermédiaire 4 : Acide 2(S)[1(S) (1,1 diméthyl) éthoxycarbonyl) éthyl]2(S) hydroxy 4-méthyl pentanoïque

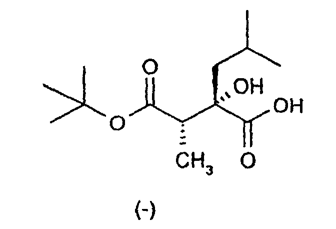

### a) 4-Methyl-2-oxopentanoate de sodium

Dans un réacteur de 20 1, introduire 771,8 g (6 moles) de tBuOK et 6 I d'éther éthylique. Purger à l'azote. Refroidir à +8°C. Ajouter en 1 h 846 ml (6 moles) d'oxalate d'éthyle. Revenir à +20°C et ajouter en 20 mn 900 ml (6 moles) d'isovalérate d'éthyle et agiter à température ambiante une nuit. Glacer à 0°C et ajouter 6 I d'HCl N. Extraire avec de l'éther éthylique. Sécher (Na₂SO₄) et évaporer les phases organiques.

Reprendre l'huile obtenue avec 3 I de dioxane et 3 I d'H₂SO₄ 5 N et chauffer 4 jours à 100°C. Glacer et neutraliser avec 2,2 I de NaOH 10N (pH 7). Laver 2 fois par AcOEt. Evaporer à sec la phase aqueuse. Sécher le solide obtenu à la pompe à palettes. Reprendre avec 7 I de méthanol. Brasser, filtrer, concentrer. Recristalliser les 1 020 g obtenus dans 6,4 I d'éthanol absolu. Récupérer 454 g (50 %) de produit pur.
RMN (CD₃OD) : δ 2,6 (2H, d, CH-CH₂COCOONa) ; 2,15 (1H, m, (CH₃)₂CH-CH₂) ;
0,95 (6H, d, (CH₃)₂ CH).

### b) (S) mandelate d'éthyle

Dans 20 ml de CH₂Cl₂ sec, ajouter 1,52 g (10 mmoles) d'acide S mandélique, ε de DMAP, 1,70 ml (21 mmoles) de pyridine et ε de DMF. Ajouter 2,7 ml (21 mmoles) de TMSCI et agiter 2 h à température ambiante. Ajouter 0,91 ml (10,5 mmoles) de chlorure d'oxalyle et agiter 2 h à température ambiante. Ajouter 20 ml d'éthanol et agiter 1/2 h à température ambiante. Laver avec 2x20 ml d'HCl N puis NaHCO₃. Sécher (Na₂SO₄), évaporer à sec. Récupérer 1,73 g (96 %).
[α]_{D} = 127,7° à t = 21°C (c = 3, CHCl₃).
RMN (CDCl₃) : δ 7,4 (5H, m, CH(Ar)) ; 5,2 (1H, s, Ar-CH-OH) ;
4,2 (2H, m, OCH₂) ; 1,25 (3H, t, OCH₂CH₃).

### c) Acide 4-méthyl-2-oxo pentanoïque 1 (S) éthoxycarbonyl phényl méthyl ester

Disperser 50 g (0,329 mole) de méthyl-4-oxo-2 pentanoate de sodium dans 900 ml de CH₂Cl₂ contenant ε de DMF. Ajouter au goutte à goutte 28,7 ml (0,329 mole) de chlorure d'oxalyle. En fin d'addition, agiter 30 mn à température ambiante. Refroidir à +10°C et ajouter 56,4 g (0,313 M) du composé **b)** solubilisé dans 300 ml de CH₂Cl₂. Ajouter ensuite 57,3 ml (0,411 mole) de triéthylamine diluée dans 200 ml de CH₂Cl₂. Agiter une nuit à température ambiante. Laver par HCI N puis NaHCO₃. Sécher, évaporer. Purifier par flash chromatographie sur 800 g de silice (éluant : heptane:AcOEt; 95:5). Récupérer 71 g d'huile (78 %).
RMN (CDCl₃) : δ 7,5 (5H, m, H(Ar) ; 6 (1H, s,-O-CH-CO₂Et) ; 4,2 (2H, m, OCH₂-CH₃ ;
2,8 (2H, d, -CH₂-COCO) ; 2,3 (1H, m, CH-(CH₃)₂₎ ; 1,25 (3H, t, OCH₂-CH₃₎ ;
1 (6H, d (CH₃)₂CH).

### d) Acide -2(S) hydroxy-2(S)[(2-méthyl)propyl] 3(R)méthyl pent-4-ènoïque 1 (S) éthoxy carbonyl phénylméthyl ester

Solubiliser 10 g (34,2 mmoles) du composé c) dans 200 ml de CH₂Cl₂ sec. Refroidir à -78°C et ajouter 3,94 ml (34,2 mmoles) de SnCl₄. Agiter 30 mn à -78°C et ajouter 5,83 g (34,2 mmoles) de l'intermédiaire 2 dilué dans 50 ml de CH₂Cl₂. Agiter 1h30 à -78°C et ajouter HCI N. Extraire au CH₂Cl₂, sécher (Na₂SO₄), évaporer. Eliminer le triéthylsilanol à l'évaporateur rotatif (70°C sous 1 mmHg). Récupérer 11,6 g d'huile (97 %).
RMN (CDCl₃) : δ 7,45 (5H, m, H(Ar)) ; 5,95 (1H, s, OCHAr) ; 5,85 (1H, m, CH₂=CH) ;
5,1 (2H, m, CH₂=) ; 4,25 (2H, m, OCH₂-CH₃) ; 3,1 (1H, s large, OH); 2,6 (1H, m, =CH-CH(CH₃₎-) ; 1,65 (3H, m, CH₂-CH-(CH₃)₂ ; 1,3 (3H, t, OCH₂-CH₃) ; 1,25 (3H, d, =CH-CH(CH₃)-) ;
0,95 (3H, d) et 0,7 (3H, d, -CH(CH₃)₂₎

### e) Acide 2 (S)[-1(-1(S) hydroxycarbonyléthyl]-2 (S) hydroxy 4-méthyl pentanoïque (S) (éthoxycarbonyl) (phényl)méthyl ester

54,7 g (157 mmoles) du produit d), sont dispersés dans 550 ml de CH₂Cl₂ sec. Refroidir à -60°C et ozonolyser jusqu'à coloration bleue persistante. Purger à l'azote et ajouter 20,4 g (314 mmoles) de Zn et 18,3 ml (314 mmoles) d'acide acétique. Agiter 1 h à température ambiante. Filtrer et évaporer. Reprendre le résidu obtenu dans 550 ml de tBuOH. Ajouter 50 ml (471 mmoles) de 2-méthyl-2-butène. Additionner ensuite une solution aqueuse contenant 48,9 g (314 mmoles) de NaH₂PO₄, 2H₂O, 35,9 g (361 mmoles) de NaClO₂, H₂O 215 ml. Agiter une nuit à température ambiante. Ajouter une solution saturée en NaHCO₃. Laver au pentane et extraire à l'éther éthylique. Sécher, évaporer. Récupérer 46,1 g d'huile blanche (soit 80 %).
[α]_{D} : + 60,2 ° à t = 20°C (c = 1, CHCl₃).
RMN (CDCl₃) : δ 7,45 (5H, s, HAr) ; 6 (1H, s, O-CH(CO)-Ar) ; 4,25 (2H, m OCH₂CH₃₎ ;
3,1 (1H, q, HO₂C-CH(CH₃₎); 1,8 (3H, m, CH₂-CH(CH₃)₂); 1,4 (3H, d, CH(CH₃)-COOH);
1,3 (3H, t, OCH₂-CH₃) ; 0,95 et 0,75 (6H, 2d, CH-(CH₃)₂)

### f) Acide 2-(S) [1(S) (1,1 diméthyl éthoxycarbonyl)éthyl]-2(S) hydroxy-4-méthyl pentanoïque (S) éthoxy carbonyl phénylméthyl ester

34,5 g (94,1 mmoles) du composé e) sont solubilisés dans 330 ml de CH₂Cl₂ sec. Refroidir à -20°C l'autoclave, ajouter 300 ml d'isobutène, 0,4 ml d'acide sulfurique concentré. Fermer l'autoclave et agiter une nuit à température ambiante.

Verser sur une solution de NaHCO₃ saturée. Sécher la phase organique, filtrer, évaporer à sec. Récupérer 31,9 g de produit pur (80 %).
[α]_{D} = + 70,2° à t = 20°C (c = 1, MeOH)
RMN (CDCl₃) : δ 7,45 (5H, m, HAr) ; 6 (1H, s, O-CH Ar) ; 4,2 (2H, m, OCH₂CH₃) ;
3,75 (1H, s large, OH) ; 2,9 (1H, q, tBuOCOCH) ; 1,7 (3H, m, CH₂CH(CH₃)₂) ;
1,5 (9H,s, (CH₃)₃-C) ; 1,4 (3H, d, CH-CH₃) ; 1,25 (3H, t, OCH₂CH₃) ; 0,95 et
0,7 (6H, 2d, CH-(CH₃)₂).

### g) Acide 2-(S) [1 (S)((1,1 diméthyl) éthoxycarbonyl) éthyl] 2 (S) hydroxy 4-méthyl pentanoïque.

Solubiliser 31,7 g (75 mmoles) du produit f) dans 320 ml d'éthanol absolu. Sous azote, ajouter 3,2 g de Pd/C 10 %. Agiter 2 h à 20°C sous atmosphère d'hydrogène. Filtrer le catalyseur, le rincer à l'éthanol. Evaporer à sec. Reprendre à l'éther éthylique, extraire avec 75 ml de soude N.

Laver la phase aqueuse puis acidifier avec 75 ml d'HCI N. Extraire à l'éther éthylique, sécher, évaporer; Récupérer 18,7 g de solide blanc (95 %).
P.F. : 67°C
[α]_{D} = - 9,9° à t = 20°C (c = 1, CHCl₃)
RMN (CDCl₃) : δ 8,9 et 4,6 (2H très large, OH et COOH) ; 2,75 (1H, q, tBuOCOCHCH₃) ;
1,85 (2H, m, CH₂-CH) ; 1,55 (1H, m, CH₂-CH) ; 1,5 (9H, s, (CH₃)₃C) ; 1,2 (3H, d, COCHCH₃) ;1 et 0,9 (6H, 2d, CH(CH₃)₂).

### Intermédiaire 5 : Acide 2-(S), 3(S)dihydroxy-3(S)-hydroxycarbonyle-5 méthyl hexanoïque, 1,1-diméthyléthyl ester

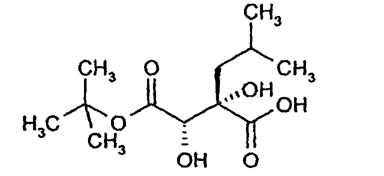

### a) Acide 4-méthyl-2-oxo pentanoïque phénylméthyl ester

Disperser 78,7 g (0,51 mole) du composé a) de l'intermédiaire 4 dans 500 ml de CH₂Cl₂ sec. Ajouter 39,2 ml (0,51 mole) de DMF. Refroidir à -20°C et ajouter 45 ml (0,51 mole) de chlorure d'oxalyle. Agiter 2 h à température ambiante.

A 0°C, ajouter le mélange de 50 ml de CH₂Cl₂, 44 ml (0,425 mole) d'alcool benzylique, 143,4 ml de triéthylamine. Agiter 18 h à température ambiante. Laver par HCI N puis NaHCO₃ (solution saturée). Sécher sur Na₂SO₄. Filtrer, évaporer à sec. Distiller à 114°C sous 3 mmHg. Récupérer 70,85 g (63 %).
RMN (CDCl₃) : δ 7,45 (5H, m, H (Ar)) ; 5,3 (2H, s, OCH₂Ar) ; 2,7 (2H, d, CH₂CO) ; 2,15 (1H, m, CH-(CH₃)₂) ; 0,95 (6H, d, CH(CH₃)₂).

### b) Acide 5-méthyl-3(phénylméthoxycarbonyl)(E) hex-2-enoïque, diméthyl 1-1 éthyl ester

Dans un ballon, introduire 46 g (0,10 mole) de bromure de tert-butoxycarbonyl méthyl triphényl phosphonium. Ajouter 12,8 g (0,105 mole) de tBuOK et agiter 30 mn à température ambiante. Ajouter 20 g (0,091 mole) du composé a) dilué avec 60 ml de DMF. Agiter une nuit à 20°C. Evaporer à sec. Brasser dans de l'éther isopropylique, filtrer, évaporer.

Purifier par flash chromatographie sur 600 g de silice (éluant heptane:AcOEt ; 95:5)

Récupérer 19,9 g (69 %) de produit E.
RMN (CDCl₃) : δ 7,4 (5H, s, HAr) ; 6,75 (1H, s, COCH=) ; 5,2 (2H, s, OCH₂Ar) ;
2.75 (2H, d, CH₂-C=) ; 1,9 (1H, m, CH-(CH₃)₂) ; 1,55 (9H, s, C(CH₃)) ; 0,95 (6H, d, CH(CH₃)₂).

### c) Acide 2(S), 3(S) dihydroxy-5 méthyl-3(S) phénylméthoxycarbonyl hexanoïque, diméthyl 1,1 éthyl ester

Introduire dans un ballon 11,5 g d'AD mix β, 0,78 g (8,1 mmoles) de méthyl sulfonamide, 83 ml d'un mélange 1/1 de tBuOH et H₂O. Agiter 2 mn à + 20°C puis refroidir à 0°C.

Ajouter 2,6 g (8,1 mmoles) du composé b). Agiter 4 h à 0°C puis 2 h à température ambiante. Ajouter 3 g supplémentaire d'AD-mix β et agiter 1 nuit à 20°C. A 0°C ajouter 16,4 g de sulfite de sodium, agiter 1 h à température ambiante.

Extraire par CH₂Cl₂. Laver à l'eau puis avec KOH 2N. Sécher, filtrer, évaporer à sec.

Récupérer 3,2 g d'huile (100 %).
RMN (CDCl₃) : δ 7,4 (5H, s, HAr) ; 5,25 (2H, s, OCH₂Ar) ; 4,2 (1H, d, OH) ; 3,55 (1H, s, OH) ;
3,4 (1H, d, -CH-OH) ; 1,75 (3H, m, CH₂-CH-(CH₃)₂) ; 1,55 (9H, s, OC(CH₃)₃) ;
1 et 0,85 (6H, 2d, CH-(CH₃)₂).

### d) Acide 2(S)[(S) 4'diméthyléthoxycarbonyl-S-hydroxyméthyl] 2S hydroxy 4 méthyl pentanoïque

Solubiliser 2 g du composé c) (5,66 mmoles) dans 20 ml de méthanol sous azote. Ajouter 200 mg de Pd/c 10 %. Purger à l'hydrogène et agiter 3 h à 20°C. Filtrer le catalyseur sur célite, évaporer à sec. Récupérer 1,1 g (95 %) de solide jaune.
PF: 110°C.
RMN (CDCl₃) : δ 5,9 (1H, s très large, OH) ; 4,25 (1H, s, CHOH) ; 1,9 (2H, d, CH₂-CH) ; 1,8 (1H, m, CH-(CH₃)₂) ; 1,55 (9H, s, (CH₃)₃C); 1 et 0,9 (6H, 2d, (CH₃)₂CH).

### Intermédiaire 6 : Acide 3 (S) hydroxy-3 (S) hydroxycarbonyle-2(S) méthoxy 5-méthylhexanoïque, 1,1-diméthyléthyl ester

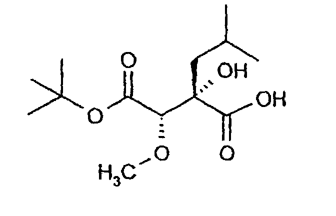

### a) Acide 3(S)-hydroxy-2(S) méthoxy-5 méthyl 3(S) phénylméthoxycarbonyl hexanoïque, diméthyl 1,1 éthyl ester

A une suspension de 85 mg (3,4 mmoles) de NaH dans 10 ml de THF sec, ajouter à 0°C 1 g (2,8 mmoles) du composé c) de l'intermédiaire 5; Agiter 30 mn à 20°C.

A 0°C ajouter 0,9 ml (14 mmoles) de CH₃l. Agiter une nuit à température ambiante.

Ajouter HCI N. Extraire par CH₂Cl₂. Sécher, évaporer. Purifier par flash chromatographie (éluant heptane:AcOEt ; 95:5).

Récupérer 380 mg (38 %) de produit pur.
RMN (CDCl₃) : δ 7,4 (5H, m, HAr) ; 5,25 (2H, dd, OCH₂Ar); 3,85 (1H, s, CHOCH₃) ;
3,4 (1H, s, OH) ; 3,25 (3H, s, OCH₃) ; 1,8 (2H, d, CH₂CH) ; 1,7 (1H, m, CH₂-CH-(CH₃)₂) ;
1,55 (9H, s, C(CH₃)₃) ; 1 et 0,85 (6H, 2d, CH(CH₃)₂).

### b) Acide 3(S) hydroxy hydroxycarbonyl-2(S) méthoxy-5méthyl hexanoïque, diméthyl-1,1 éthyl ester

A une suspension de 40 mg de Pd/c 10 % dans 5 ml de méthanol, ajouter 380 ml (1 mmole) du composé précédent. Agiter 2 h à 20°C sous atmosphère d'hydrogène. Filtrer sur célite. Rincer au méthanol . Evaporer à sec. Récupérer 270 mg (93 %).
RMN (CDCl₃) : δ 3,9 (1H, s, CH(OCH₃)) ; 3,45 (3H, s, OCH₃) ; 1,75 (2H, m, CH₂CH(CH₃)₂) ; 1,6(10 H, m, C(CH₃)₃ + CH₂CH) ; 1 ( 6H, dd (CH(CH₃)₂).

### Intermédiaire 7 : Acide 2(S)[-2(S)-but-3-enyl]-2(S) hydroxy 4-méthyl pentanoïque

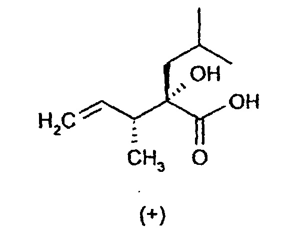

A 930 mg (2,67 mmoles) de composé d) de l'intermédiaire 4 dans 8 ml d'éthanol ajouter 8 ml de soude N (8 mmoles) et chauffer une nuit à reflux.

Refroidir à 20°C, laver à l'éther éthylique. Acidifier par HCI N et extraire au dichlorométhane. Sécher sur Na₂SO₄, filtrer, évaporer.

Purifier par flash chromatographie sur 37 g de silice les 810 mg d'huile obtenus (éluant :
CH₂Cl₂:MeOH:AcOH ; 95:5:0,5).
Récupérer 410 mg de produit pur (82 %).
[α]₃₆₅ = + 24,1° à t = 20°C (c = 1,25, MeOH).
RMN (DMSO) : δ 5,7 (1H, m, CH₂=CH-) ; 5,05 (1H, d, CH₂=) ; 5 (1H, s, CH₂=) ;
2,35 (1H, m, CH₂=CH-CH-CH₃) ; 1,65 (1H, m, CH-(CH₃)₂) ; 1,5 (2H, d, CH₂-CH(CH₃)₂) ; 0,85 et 0,8 (9H, 3d, CH₃).

### Intermédiaire 8 : Acide 2-oxo-5-phénylpentanoïque (S) éthoxycarbonyl (S) phénylméthyl ester

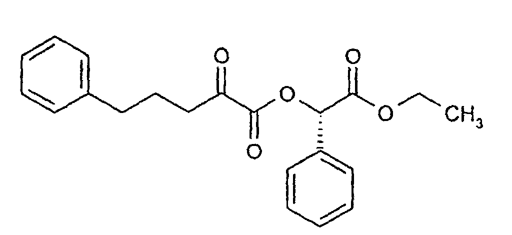

Synthétisé de la même manière que pour l'intermédiaire 4.

### a) Acide 2-oxo-5 phényl pentanoïque, sel de sodium

RMN (DMSO) : 7,2 (5H, m, H(Ar)) ; 2,5 (4H, m, COCH₂CH₂CH₂) ; 1,55 (2H, m, COCH₂CH₂). IR: ν cétone : 1706 cm⁻¹
ν COONa : 1625 cm⁻¹

### b) Acide 2-oxo-5 phényl pentanoïque (S) éthoxycarbonyl (S) phényl méthyl ester

RMN (CDCl₃) : δ 7,4 (10H, m, H(Ar) ; 6 (1H, s, OCHAr) ; 4,25 (2H, m, OCH₂);
2,95 (2H, m, ArCH₂); 2,8 (2H, t, COCH₂); 2,05 (2H, m, COCH₂CH₂); 1,25 (3H, t, CH₂CH₃)

### Intermédiaire 9 : Acide 2(S) hydroxy-3(R)méthyl 2(S)-(2-propyl) pent-4-ènoïque

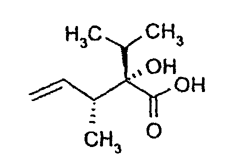

### a) Acide 2-oxo-3-méthyl butyrique (S) éthoxycarbonyl (S) phénylméthyl ester Synthétisé de la même manière que l'intermédiaire 4c.

RMN (CDCl₃) : δ 7,4 et 7,5 (5H, 2m, H(Ar)) ; 6,05 (1H, s, OCHAr) ; 4,2 (2H, m, OCH₂CH₃); 3,3 (1H, m, CH(CH₃)₂) ; 1,25 (9H, m, OCH₂CH₃ et CH(CH₃)₂)

### b) Acide 2(S)hydroxy-3(R) méthyl-2(S)-(2propyl) pent-4-énoïque (S) éthoxycarbonyl(S) phényl méthyl ester

Ce produit a été synthétisé de la même façon que le composé **4d**.
RMN (CDCl₃) : δ 7,45 (5H, m, H(Ar)) ; 6 (1H, s, OCHAr) ; 5,85 (1H, m, CH₂=CH-) ;
5,75 (2H, m, CH₂=CH) ; 4,25 (2H, m, OCH₂CH₃) ; 3,1 (1H, s large, OH) ;
2,6 (1H, m, CH₂=CH-CH-CH₃) ; 2,15 (1H, m, CH(CH₃)₂) ; 1,3 (6H, 2d, CH₂=CH-CH₃ et OCH₂CH₃); 0,9 (6H, 2d, CH(CH₃)₂)

### c) Intermédiaire 9 : Acide 2(S) hydroxy-3(R) méthyl 2(S)-(2-propyl) pent-4-énoïque

Ce composé a été préparé de la même manière que l'intermédiaire **7**.
RMN (CDCl₃) : δ 5,85 (1H, m, CH₂=CH-) ; 5,2 (2H, m, CH₂=CH-) ; 3 (1H, s très large, OH) ;
2,75 (1H, m, CH₂=CH-CH-CH₃) ; 2,15 (1H, sept., CH(CH₃)₂) ; 1,15 (3H, d, =CHCH₃) ; 1,05 (6H, 2d, CH(CH₃)₂).

### Intermédiaire 10 : 4-chlorophénylalanine N-(2-méthylthio-1-éthyl) amide

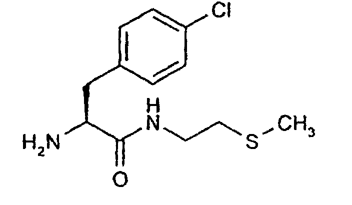

Solubiliser dans 5 ml de CH₂Cl₂ sec 500 mg (1,7 mmoles) de Boc 4-chlorophénylalanine. Ajouter 160 µl (1,7 mmoles) de 2-méthylthioéthylamine, 415 mg (2mmoles) de DCC et 270 mg (2 mmoles) de HOBT. Agiter 1 nuit à température ambiante. Filtrer la DCU. Laver par HCI 1N puis NaHCO₃. Sécher. Evaporer.

Reprendre par 6 ml de CH₂Cl₂ sec. Ajouter 1,5 ml de CF₃COOH et agiter 3 h à température ambiante. Evaporer à sec. Reprendre par AcOEt. Extraire le produit avec HCI N. Neutraliser la phase aqueuse par NaHCO3 et extraire par CH2CI2. Sécher. Evaporer. Récupérer 300 mg (soit 74 %) de produit pur.
PF : 70°C.
RMN (CDCl₃) : δ 7,6 (1H, s large, CONH) ; 7,3 et 7,5 (4H, 2d, H(Ar) ; 3,6 (1H, m, NH₂CH); 3,55 (2H, q, CONHCH₂) ; 3,25 (1H, 2d, CH₂Ar); 2,75 (1H, m, CH₂Ar) ; 2,65 (2H, t, CH₂SCH₃) ; 2,15 (3H, s, SCH₃) : 1,3 (2H, s, NH₂)

De la même façon, ont été synthétisés les intermédiaires 11 à 17

### Intermédiaire 11 : 4-chlorophénylalanine N-(2-(4-morpholino)-1-éthyl) amide

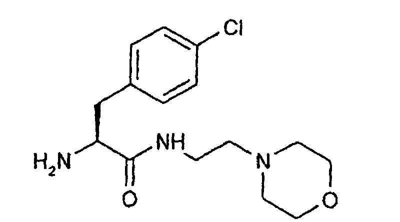

RMN (CDCl₃) : δ 7,35 (1H, s large, CONH) ; 7,3 et 7,2 (4H, 2d, H(Ar)) ; 3,7 (4H, m, -(CH₂)₂O) ;
3,6 (1H, m, H₂N-CH-CO) ; 3,35 (2H, q, CONHCH₂-) ; 3,15 (1H, dd, CH₂Ar) ; 2,75 (1H, dd, CH₂Ar) ; 2,4 (6H, m, -CH₂-N-(CH₂)₂-) ; 1,7 (2H, s, large, NH₂)

### Intermédiaire 12 : 4-iodophénylalanine N-(2-méthylthio-1-éthyl) amide

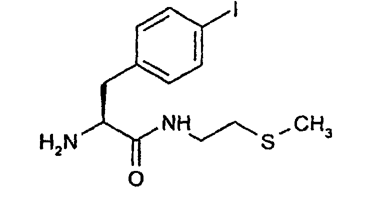

RMN (CDCl₃) : δ 7,65 (2H, d, H(Ar)) ; 7,5 (1H, s large, CONH) ; 6,95 (2H, d, H(Ar)) ;
3,6 (1H, m, H₂NCHCO) ; 3,45 (2H,m, CONHCH₂) ; 3,15 (1H, 2d, CH₂Ar) ; 2,7 (1H, 2d, CH₂Ar) ; 2,6 (2H, m, -CH₂SCH₃) ; 2,1 (3H, s, SCH₃) ; 1,7 (2H, s, large, H₂N)

### Intermédiaire 13 : 3-4-dichlorophénylalanine N-(2-méthylthio-1-éthyl)amide

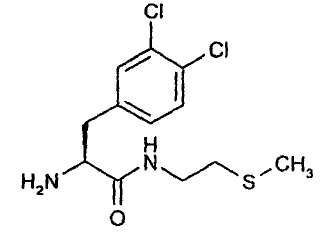

RMN (CDCl₃) : δ 7,55 (1H, s large, CONH) ; 7,3 (2H, m, H(Ar)) ; 7,05 (1H, m, H(Ar)) ;
3,6 (1H, m, H₂NCHCO) ; 3,5 (2H, m, CONHCH₂) ; 3,2 (1H, dd, CH₂Ar) ; 2,75 (1H, dd, CH₂Ar) ; 2,6 (2H, m, CH₂S) ; 2,15 (3H, s, SCH₃) ; 1,3 (2H, s très large, H₂N)

### Intermédiaire 14 : 4-chlorophénylalanine N-(2-cyano-1-éthyl) amide

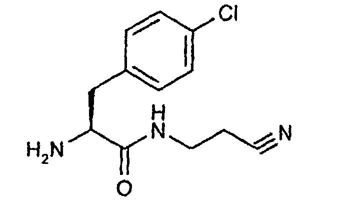

RMN (CDCl₃) : δ 7,8 (1H, s large, CONH) ; 7,3 (2H, d, H(Ar)) ; 7,15 (2H, d, H(Ar));
3,65 (1H, m, H₂NCHCO) ; 3,55 (2H, m, CONHCH₂-) ; 3,25 (1H, dd, CH₂Ar) ; 2,75 (1H, dd, CH₂Ar); 2,65 (2H, t, CH₂CN) ; 1,45 (2H, s très large, NH₂)

### Intermédiaire 15 : 3,4-dichlorophénylalanine N-[2-(2 hydroxyéthyl)oxy éthyl] amide

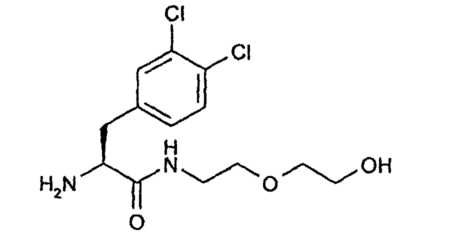

RMN (CDCl₃) : δ 7,5 (1H, s large, CONH) ; 7,3 (2H, m, H(Ar)) ; 7,1 (1H, m, H(Ar)) ;
3,75 (2H, m, CH₂OH); de 3,65 à 3,45 (9H, m, H₂N-CH-CONHCH₂-CH₂O-CH₂);
3,2 (1H, 2d, CH₂Ar) ; 2,7 (1H, 2d, CH₂Ar)

### Intermédiaire 16 : 3,4-dichlorophénylalanine N-[2-(2-méthoxyéthoxy) 1-éthyl]amide

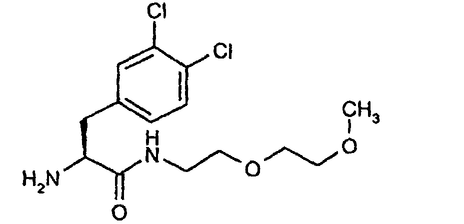

RMN (CDCl₃) : δ 7,5 (1H, s large, CONH) ; 7,35 (2H, m, H(Ar)) ; 7,1 (1H, m, H(Ar)) ;
de 3,45 à 3,7 (9H, m, H₂NCHCONH-CH₂-CH₂-O-CH₂CH₂-OCH₃) ; 3,4 (3H, s, OCH₃);
3,15 (1H, dd, CH₂Ar) ; 2,9 (1H, dd, CH₂Ar) ; 2 (2H, s très large, NH₂)

### Intermédiaire 17 : L βcyclohexylalanine N-(2-phényl-1-éthyl)amide

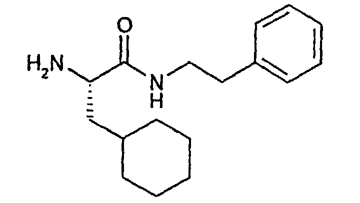

RMN (CDCl₃) : δ de 7,2 à 7,4 (6H, m, CONHCH₂ et H(Ar)) ; 3,55 (2H, m, H₂NCHCONHCH₂) ; 3,4 (1H, dd, CONHCH₂-) ; 2,85 (2H, m, CH₂Ar) ; de 0,9 à 1,9 (16H, 3 m, H₂NCH-CH₂-cyclohex)

### Intermédiaire 18 : D,L 2,4-dichlorophénylalanine N-méthylamide

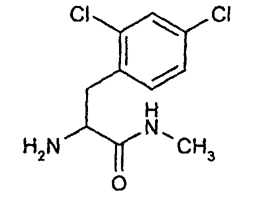

Les aminoacides racémiques ont été synthétisés par les méthodes connues dans l'état de l'art.

Solubiliser 1 g (4,27 mmoles) de D,L 2,4-dichlorophénylalanine dans 35 ml de MeOH. Ajouter 3,25 ml (25,6 mmoles) de TMSCI et chauffer une nuit à reflux.

Evaporer à sec. Reprendre avec 20 ml de MeOH. Glacer à -20°C. Ajouter 15 ml de méthylamine et agiter 3 h à + 20°C. Evaporer à sec. Reprendre par de l'eau. Neutraliser par NaHCO3 et extraire au chloroforme. Sécher. Evaporer. Récupérer 900 mg (soit 85 %) d'huile.
RMN (CDCl₃) : δ 7,4 (1H, s, H(Ar)) ; 7,2 (2H, s, H(Ar)) ; 7,15 (1H, m, CONH) ;
3,7 (1H, m, NH₂CHCO) ; 3,45 (1H, dd, CH₂Ar) ; 2,9 (1H, dd, CH₂Ar) ; 2,85 (3H, d, NHCH₃) ;
1,55 (2H, s large, NH₂)

De la même manière ont été synthétisés les intermédiaires **19** à **21** :

### Intermédiaire 19 : D,L 2,6-dichlorophénylalanine N-méthylamide

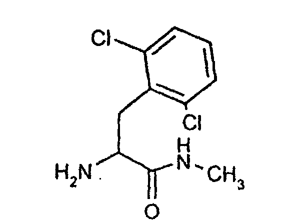

RMN (CDCl₃) : δ 7,3 (2H, d, H(Ar)) ; 7,15 (1H, t, H(Ar)) ; 3,75 (1H, m, NH₂CHCO) ;
3,65 (1H, dd, CH₂Ar) ; 3,15 (1H, dd, CH₂Ar) ; 2,85 (3H, d, NHCH₃)

### Intermédiaire 20 : D,L 3-chlorophénylalanine N-méthylamide

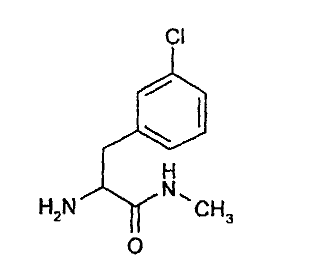

RMN (DMSO) : δ 8,1 (1H, q, CONHCH₃) ; 7,25 (3H, m, H(Ar)) ; 7,15 (1H, d, H(Ar)) ;
3,55 (1H, m, CHCONHCH₃) ; 2,95 (1H, dd, CH₂Ar) ; 2,75 (1H, dd, CH₂Ar) ; 2,55 (3H, d, NHCH₃)

### Intermédiaire 21 : D,L 2,5-dichlorophénylalanine N-méthylamide

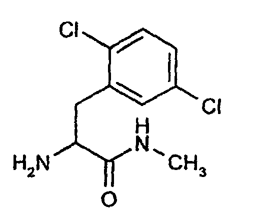

RMN (CDCl₃) : δ de 7,15 à 7,35 (4H, m, H(Ar), CONH) ; 3,7 (1H, dd, H₂NCHCO) ;
3,5 (1H, dd, CH₂Ar) ; 2,9 (4H, m, CH₂Ar et NHCH₃) ; 1,4 (2H, s très large, NH₂)

### Intermédiaire 22 : Acide 2(R)[1(S*)(4-(méthoxy)-benzylmercapto)éthyl] 2(R*)-hydroxy 4-méthylpentanoïque

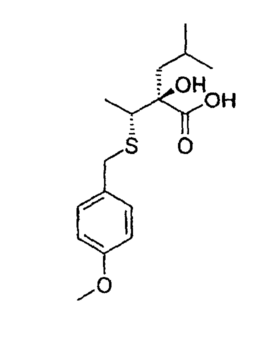

### a) Acide (E)-2[2(méthyl)propyl]but-2-énoïque éthylester

A 183, 54 g (0,494 moles) de bromure d'éthyltriphénylphosphonium dans 940 ml de THF, à température ambiante, ajouter 593 ml (0,593 mole) de bis-triméthylsilyle amidure de sodium (1 M dans le THF) puis 147 ml d'HMPA goutte à goutte.

Agiter à température ambiante 45 minutes puis additionner 62,57 g (0,395 mole) d'acide 4-méthyl-2-oxopentanoïque éthyl ester en solution dans 60 ml de THF en 1 heure à température ambiante. Agiter 1 heure à température ambiante et verser le milieu réactionnel sur 900 ml d'eau et glace. Extraire par 3 fois 800 ml d'éther éthylique et sécher sur sulfate de sodium puis évaporer sous vide à 30°C.

Purifier par flash chromatographie (étuant : heptane : Et₂O ; 99:1 puis pentane : Et₂O ; 97:3). Récupérer 43,71 g d'une huile jaune (65 %).
RMN (CDCl₃) : δ 6,9 (q, 1H, CH=) ; 4,2 (q, 2H, OCH₂CH₃) ; 2,2 (d, 2H, CH₂CH) ;
1,8 (d, 3H et m, 1H, CH₃CH= et CH₂CHCH₃) ; 1,3 (t, 3H, OCH₂CH₃) ; 0,9 (d, 6H, CH(CH₃)₂) ;
IR (CHCl₃) : νCO : 1701 cm⁻¹ ; νC=C : 1644 cm⁻¹

### b) Acide 2 (S)[1 (oxoéthyl)] 2-hydroxy 4-méthylpentanoïque éthyl ester

A 13,82 g (81, 2 mmoles) de produit **a)** dans 1,12 I d'acétone, 335 ml d'eau et 28,35 ml d'acide acétique à -10°C, ajouter par spatulées 22,32 g (141,2 mmoles) de KMnO₄.

Agiter 1h30 à -10°C puis filtrer.

Evaporer l'acétone du filtrat puis l'extraire par CH₂Cl₂ et sécher sur Na₂SO₄. Evaporer sous vide et purifier par flash chromatographie (éluant : heptane : CH₂Cl₂ : AcOEt ; 88:10:2).

Récupérer 3,53 g d'un liquide incolore (21 %).
RMN (CDCl₃) : δ 4,3 (q, 2H, OCH₂CH₃) ; 4,2 (s, 1H, OH) ; 2,3 (s, 3H, CH₃CO) ;
2,1 (dd, 1H, CH₂CH) ; 1,8 (m, 2H, CH₂CH) ; 1,3 (t, 3H, OCH₂CH₃) ; 0,9 (dd, 6H, CH(CH₃)₂).
IR (CHCl₃) : νOH : 3510 cm⁻¹ ; νCO (cétone et ester) : 1717 cm⁻¹.

### c) Acide 2 (S)[1(S*)-hydroxyéthyl] 2(S*)-hydroxy 4-méthylpentanoïque éthyl ester

A 3,31 g (16,4 mmoles) de produit b) dans 33 ml d'éthanol à 0°C, ajouter 0,62 g (16,4 mmoles) de borohydrure de sodium en 15 minutes. Agiter 5 minutes puis évaporer l'éthanol.

Le résidu est repris dans HCI 2N et extrait deux fois par Et₂O. Les phases éthérées sont séchées sur sulfate de sodium puis évaporées. Purifier par flash chromatographie (éluant : heptane : Et₂O : CH₂Cl₂ ; 70:20:10).

Récupérer 0,81 g du diastéréosiomère le moins polaire (25 %).
RMN (CDCl₃) : δ 4,3 (q, 2H, OCH₂CH₃) ; 3,8 (m, 1H, CH-OH) ; 3,5 (s, 1H, OH) ; 2,3 (d, 1H, OH) ; 1,9 (dd, 1H, CH₂-CH) ; 1,8-1,7 (m, 2H, CH₂CH, CH(CH₃)₂) ; 1,4 (t, 3H, OCH₂CH₃) ; 1,15 (d, 3H, CH-CH₃) ; 1 (d, 3H, CH(CH₃)₂) ; 0,85 (d, 3H, CH(CH₃)₂).
IR (CHCl₃): νOH :3528 cm⁻¹ ; νCO : 1721 cm⁻¹.

### d) Acide 2 (S)[1-(S*)-éthylméthanesulfonate] 2-(S*)-hydroxy 4-méthylpentanoïque éthyl ester

A 0,48 g (2,35 mmoles) du produit c) dissous dans 10 ml d'éther éthylique à 0°C, ajouter 0,36 ml (2,58 mmoles) de triéthylamine puis 0,2 ml (2,58 mmoles) du chlorure d'acide méthanesulfonique. Agiter 1 nuit à température ambiante.

Le milieu est ensuite lavé par H₂O puis de la soude 0,5 N puis de l'eau.

Sécher sur sulfate de sodium et évaporer.

Récupérer 0,57 g d'une huile visqueuse (86 %).
RMN (CDCl₃) : δ 4,8 (q, 1H, CH-OSO₂CH₃) ; 4,3 (m, 2H, OCH₂CH₃) ; 3,6 (s, 1K, OH) ;
2,2 (dd, 1H, CH₂CH); 1,9 (m, CH(CH₃)₂); 1,7 (dd, 1H, CH₂CH); 1,5 (d, 3H, CH-CH₃);
1,4 (m, 3H, OCH₂CH₃); 1 (dd, 6H, CH(CH₃)₂)
IR (CHCl₃) : νOH :3518 cm⁻¹ ; νCO : 1728 cm⁻¹.

### e) Acide 2 (R)[1(S*)-(4-(méthoxy)-benzylmercapto)éthyl]-2-(R*)-hydroxy 4-méthylpentanoïque éthyl ester

A 0,33 g (2,12 mmoles) de 4-(méthoxy)-benzylsulfure dans 1,3 ml d'éthanol, ajouter 0,97 ml (2,12 mmoles) d'éthylate de sodium (2,2 M dans l'éthanol). Agiter 5 minutes et additionner cette solution sur 0,54 g (1,91 mmoles) de produit d) dans 8,8 ml d'éthanol. Agiter 4 heures à 70°C puis 1 nuit à température ambiante.

Evaporer l'éthanol et reprendre le résidu dans AcOEt. Laver par H₂O, NaOH 1N puis de l'eau.

Sécher sur sulfate de sodium et évaporer.

Purifier par flash chromatographie (éluant : heptane : Et₂O ; 98:2 puis 97:3).

Récupérer 0,23 g (35 %).
RMN (CDCl₃) : δ 7,2 (d, 2H, CHAr) ; 6,8 (d, 2H, CHAr) ; 4,3 (q, 2H, OCH₂CH₃);
3,8 (s, 3H, OCH₃ et dd, 2H, CH₂S); 3,4 (s, 1H, OH) ; 2,8 (q, 1H, CHS) ; 2 (dd, 1H, CH₂CH); 1,7-1,5 (m,2H, CHCH₂); 1,3 (t, 3H, OCH₂CH₃); 1,25 (d, 3H, CH₃CH); 1 (d, 3H, CH(CH₃)₂) ; 0,9 (d, 3H,
CH(CH₃)₂).
IR (CHCl₃) : νOH :3526 cm⁻¹ ; νCO: 1723 cm⁻¹.

### f) Acide 2 (R) [1(S*) (4-(méthoxy) benzylmercapto)éthyl] 2 (R*) -hydroxy 4-méthylpentanoïque

A 0,31 g (0,91 mmole) de produit **d)** dans 6 ml d'éthanol ajouter 3,18 ml (3,18 mmoles) de soude 1N. Agiter 1 nuit à 80°C. Diluer avec H₂O et extraire par AcOEt. Acidifier la phase aqueuse par HCI 1N et extraire par CH₂Cl₂. Sécher sur sulfate de sodium et évaporer. Cristalliser en ajoutant au résidu de l'éther de pétrole et quelques gouttes d'éther éthylique.

Récupérer après filtration 0,123 g (43 %).
RMN (CDCl₃) : δ 7,3 (d, 2H, CHAr) ; 6,8 (d, 2H, CHAr) ; 3,7 (s, 3H, OCH₃ et dd, 2H, CH₂S) ; 3,3 (s, 1H, OH) ; 2,9 (q, 1H, CHCH₃) ; 2 (dd, 1H, CH₂CH) ; 1,8 (m, 1H, CH₂CH) ;
1,7 (dd, 1H, CH₂CH) ; 1,3 (d, 3H, CH₃CH) ; 1 (d, 3H, CH(CH₃)₂) ; 0,9 (d, 3H, CH(CH₃)₂).

### Intermédiaire 23 : Acide 2 (S) [1(S*)(o-benzylhydroxylamino)éthyl] 2(S)-hydroxy 4-méthylpentanoïque

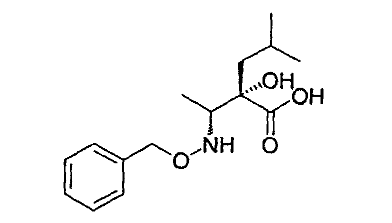

### a) Acide 2 (S)[(1-(R)-hydroxyéthyl)] 2-(S)-hydroxy 4-méthyl pentanoïque éthyl ester

A 21,84 g d'AD-Mix β dans 70 ml de tert-butanol et 70 ml d'eau agités 20 minutes, ajouter 1,16 g (12 mmoles) de méthane sulfonamide.

Refroidir à 0°C puis ajouter 2,08 g (12 mmoles) de produit **a)** de l'intermédiaire **22**.

Agiter 2 jours à température ambiante. Additionner 18 g de sulfite de sodium et agiter 2 heures. Extraire par AcOEt et laver la phase organique deux fois par KOH 2N.

Sécher sur sulfate de sodium et évaporer.

Purifier par flash chromatographie (éluant : CH₂Cl₂ : AcOEt : MeOH ; 95:4:1).

Récupérer 1,88 g (77 %).
RMN (CDCl₃) : δ 4,3 (q, 2H, OCH₂CH₃) ; 3,9 (m, 1H, CHOH) ; 3,4 (s, 1H, OH) ;
2,05 (d, 1H, OH) ; 1,7-1,5 (m, 3H, CH₂CH) ; 1,3 (t, 3H, OCH₂CH₃); 1,25 (d, 3H, CH₃CH) ;
1 (d, 3H, CH(CH₃)₂) ; 0,9 (d, 3H, CH(CH₃)₂).
IR (CHCl₃) : νOH :3518 cm⁻¹ ; νCO : 1724 cm⁻¹.

### b) Acide 2 (S) [1(oxoéthyl)] 2(S)-hydroxy-4-méthylpentanoïque éthyl ester

A 6,76 g (33,1 mmoles) de produit **a)** dans 200 ml de CH₂Cl₂ à 0°C, ajouter 9,4 ml (132 mmoles) de DMSO puis 18,7 g (132 mmoles) de P₂O₅.

Agiter 30 minutes à température ambiante puis additionner de nouveau 9,38 g de P₂O₅.

Agiter 16 h à température ambiante puis additionner 32,3 ml (231,7 mmoles) de triéthylamine en 15 minutes.

Agiter 1 heure à température ambiante puis additionner 200 ml d'HCI 1N à 0°C.

Décanter, laver la phase organique deux fois par HCI 1N. Purifier par flash chromatographie (éluant : heptane : AcOEt ; 95:5).

Récupérer 3,25 g d'huile (48 %).
RMN (CDCl₃) : δ 4,3 (q, 2H, OCH₂CH₃) ; 4,2 (s, 1H, OH) ; 2,3 (s, 3H, CH₃CO) ;
2,1 (dd, 1H, CH₂CH) ; 1,9 (dd, 1H, CH₂CH) ; 1,8 (m, 1H, CH₂CH) ; 1,3 (t, 3H, OCH₂CH₃) ;
0,95 (dd, 6H, CH(CH₃)₂).
IR (CHCl₃) : vOH :3504 cm⁻¹ ; νCO (ester + cétone) : 1716 cm⁻¹.

### c) Acide 2 (S) [1(o-benzylhydroxylimino)éthyl] 2-(S)-hydroxy 4-méthylpentanoïque éthyl ester

A 0,674 g (3,33 mmoles) de produit b) dans 10 ml d'éthanol additionner 0,585 g (3,66 mmoles) de chlorhydrate de o-benzyihydroxylamine et 0,27 ml (3,33 mmoles) de pyridine.

Agiter à 95°C pendant 3 heures.

Evaporer l'éthanol et reprendre le résidu par H₂O.

Extraire par 3 x 50 ml d'AcOEt.

Sécher la phase organique et évaporer sous vide.

Récupérer 1 g d'une huile incolore (98 %).
RMN (CDCl₃) : δ 7,3 (m, 5H, CHAr) ; 5,15 (s, 2H, CH₂Ar) ; 4,2 (q, 2H, OCH₂CH₃) ;
3,9 (s, 1H, OH) ; 1,9 (m, 5H, CH₃C=N et CH₂CH); 1,8 (m, 1H, CH₂CH) ; 1,3 (t, 3H, OCH₂CH₃); 0,95 (dd, 6H, CH(CH₃)₂).
IR (CHCl₃) : νOH :3514 cm⁻¹ ; νCO : 1726 cm⁻¹.

### d) Acide 2 (S) [1(o-benzylhydroxyamino) éthyl] 2 (S)-hydroxy 4-méthylpentanoïque éthyl ester

A 0,737 g (2,4 mmoles) de produit c) dans 20 ml de méthanol ajouter 1,35 g (21,6 mmoles) de cyanoborohydrure de sodium puis 5,13 ml de méthanol chlorhydrique goutte à goutte.

Agiter pendant 1 nuit et évaporer sous vide.

Le résidu est repris par HCl 1N et extrait trois fois par AcOEt.

Laver la phase organique par NaOH 1 N puis H₂O.

Purifier par flash chromatographie (éluant : heptane : AcOEt ; 9:1).

Récupérer 0,625 g d'une huile incolore (84 %).
RMN (DMSO) : δ 7,3 (m, 5H, HAr) ; 6,2 (d, 0,4H, NH) ; 6 (d, 0,6H, NH) ; 4,5 (dd, 2H, CH₂Ar);
4 (m, 2H, OCH₂CH₃) ; 3,25 (m, 0,6H, CHNH) ; 3 (m, 0,4H, CHNH) ; 1,7-1,4 (m, 3H, CH₂CH) ; 1,2 (t,
3H, OCH₂CH₃) ; 1,05 (dd, 2H, CH₃CH) ; 0,9 (dd, 3H, CH(CH₃)₂);
0,8 (dd, 3H, CH(CH₃)₂).
IR (CHCl₃) : νOH :3514 cm⁻¹ ; νCO : 1723 cm⁻¹.

### e) Acide 2 (S) [1 (o-benzylhydroxylamino) éthyl]2-(S)-hydroxy 4-méthylpentanoïque

A 0,6 g (1,95 mmoles) de produit **d)** dans 10 ml d'éthanol, ajouter 3,9 ml de soude 1N.

Agiter à 100°C pendant six heures. Evaporer l'éthanol et reprendre le résidu par H₂O. Neutraliser jusqu'à pH 6 par H₂SO₄ 1M.

Evaporer à sec et reprendre le résidu par du méthanol. Filtrer, évaporer.

Récupérer 0,55 g d'une mousse blanche (100 %).
RMN (DMSO): δ 7,3 (m, 5H, HAr) ; 6,6 (d, 0,4H, NH) ; 6,4 (d, 0,6H, NH) ; 4,9 (s, 0,4H, OH) ; 4,8 (s, 0,6H, OH) ; 4,55 (m, 2H, CH₂Ar): 2,9 (m, 1H, CHNH) ; 1,8-1,4 (m, 3H, CH₂CH);
1 (dd, 3H, CH₃CH) ; 0,9-0,8 (m, 6H, CH(CH₃)₂).
IR (CHCl₃) : νOH :3416 cm⁻¹ ; νCO : 1724 cm⁻¹.

### Exemple 1 : N-2(S)[(1-hydroxycarbamoyl)-1-éthyl]2-(S)hydroxy-4 méthylpentanoyl O-méthyltyrosine N methylamide

### a) N(1)[-2(S*) hydroxy-2(S*)(3méthyl) propyl 3(R*) méthyl pent-4-enoyl] O-methyltyrosine N-méthylamide

Solubiliser 4,83 g (25,9 mmoles) de l'intermédiaire 1 dans 100 ml de CH₂Cl₂ sec. Ajouter 5,40 g (25,9 mmoles) de o-méthyltyrosine N-méthylamide, 13,5 g (25,9 mmoles) de PyBop et 11,3 ml (64,8 mmoles) de diisopropyléthylamine et agiter 3 h à température ambiante. Laver par HCI N puis NaHCO₃. Sécher, évaporer.

Purifier par flash chromatographie sur 800 g de silice (éluant : heptane:AcOEt ; 50:50).

Récupérer 8,31 g (soit 85 %) mélange de diastéréoisomères.
RMN (CDCl3) : δ 7,3 (1H, m, CONH-) ; 7,15 (2H, d, CH(Ar)) ; 6,85 (2H, d, CHAr) ;
6,1 (1H, d, -CONH) ; 5,75 (1H, m, CH=) ; 5,1 (2H, m, CH₂=) ; 4,6 (1H, m, CH-α(Tyr)) ;
3,8 (3H, s, OCH₃) ; 3,05 (2H, m, CH₂Ar) ; 2,75 (3H, 2d, CONHCH₃) ; 2,5 (1H, m, C=C-CH-CH₃);
1,6 (3H, m, CH2-CH(CH₃)₂); 0,8 (9H, m, CH₃).

### b) N-2(S*)[1(hydroxycarbonyl)-1 éthyl]-2(S*) hydroxy-4-méthyl pentanoyl O-méthyltyrosine N-méthylamide

Solubiliser 8,05 g (21,4 mmoles) du produit **a)** dans 200 ml de tBuOH. Solubiliser 45,7 g (214 mmoles) de NalO₄ dans 845 ml d'eau. Ajouter 0,68 g (4,3 mmoles) de KMnO₄, 3,25 g (23,5 mmoles) de K₂CO₃. Agiter 1/2 h puis ajouter 200 ml de tBuOH, puis additionner la solution de produit **a)** et agiter 4 h à température ambiante.

Ajouter HCl N et extraire par 3 fois 500 ml d'AcOEt. Rassembler les phases organiques et les laver avec une solution acide de thiosulfate. Sécher sur Na₂SO₄, évaporer à sec.

Reprendre par NaOHN. Laver à l'éther éthylique. Acidifier par HCI 6N et extraire par AcOEt. Sécher, évaporer. Récupérer 5,5 g (soit 65 %) (mélange de diastéréoisomères).
RMN (DMSO) : δ 7,9 (1H, m, CONH); 7,6 (1H, m, CONH); 7,1(2H, m, CH(Ar)) ; 6,8 (2H, m, CH(Ar)) 4,95 (1H, d, OH) ; 4,45 (1H, m, CH α(Tyr)) ; 3,7 (3H, s, OCH₃) ; 2,9 (2H, m, CH₂-Ar) ;
2,55 (4H, m, CONHCH₃ et CHCOOH) ; de 1,7 à 1 (3H, m, CH₂-CH-(CH₃)₂) de 1 à 0,5 (9H, 3CH₃).

### c) N[2(S)[1(-phénylméthoxycarbamoyl)-1 éthyl]-2(S) hydroxy-4 méthyl pentanoyl] O-méthyl-L-tyrosine N-méthylamide

Disposer 5,5 g (13,9 mmoles) du composé b) dans 200 ml de THF sec. Ajouter 3,16 g (15,3 mmoles) de DCC, 2,07 g (15,3 mmoles) de HOBt, 4,45 g (27,9 mmoles) de O benzylhydroxylamine, chlorhydrate, 3,91 ml (27,9 mmoles) de Et₃N et agiter une nuit à température ambiante. Filtrer la DCU et évaporer à sec. Reprendre par CH₂Cl₂. Laver par HCI N puis NaHCO₃ saturé. Sécher, évaporer. Purifier par flash chromatographie sur 300 g de silice (injecter avec : CH₂Cl₂ ; éluer avec : CH₂Cl₂:MeOH:NH₄ ; 95:5:0,5).

Récupérer 3,34 g du diastéréoisomère le moins polaire pur (50 %).
RMN (CDCl₃) :
**diastéréoisomère le moins polaire :**
   δ 9,1 (1H, s, CONHO) ; 7,5 (1H, d, CONH) ; 7,4 (5H, s, Ar-CH2-O) ; 7,2 (2H, d, CH(Ar)) ; 6,8 (2H, d, CH(Ar)) ; 5,9 (1H, q, CONHCH₃); 4,9 (2H, s, ArCH₂O) ; 4,8 (1H, s, OH) ; 4,4 (1H, m, CH-α(Tyr)) ;
   3,8 (3H, s, OCH₃) ; 3,2 - 2,95 (2H, m, CH₂ Ar) ; 2,7 (3H, d, CONHCH₃) ; 2,3 (1H, q, ONHCOCHCH₃) ; de 1,2 à 1,4 (3H, m, CH₂CH(CH₃)₂); 0,95 - 0,8 - 0,7 (9H, 3d, 3CH₃).
**diastéréoisomère le plus polaire :**
   **N(2R)[1(R) phénylméthoxycarbamoyl)éthyl] 2(R) hydroxy 4-méthyl L-tyrosine N-méthylamide**

RMN (CDCl₃) : δ 10 (1H, s large, CONHOH); 7,7 (1H, d, CONH); 7,35 (5H, m, ArCH₂O) ;
7,1 (2H, d, H(Ar)) ; 6,8 (2H, d, H(Ar)) ; 6,2 (1H, q large, CONHCH₃);
4,9 (3H, s + m, Ar-CH₂-O + OH) ; 4,45 (1H, m, CH-α(Tyr)) ; 3,8 (3H, s, OCH₃) ; 3 (2H, m, CH₂Ar) ; 2,8 (3H, d, NHCH₃) ; 2,5 (1H, q, CHCH₃); 1,6 et 1,3 (3H, 2 m, CH₂CH);
1 - 0,85 et 0,7 (9H, 3d, 3CH₃).

### d) N-2-(S)[1(S) hydroxycarbamoyl)-1 éthyl]-2(S) hydroxy-4-méthyl pentanoyl O méthyl L-tyrosine N-méthylamide

3,28 g (6,8 mmoles) du diastéréoisomère le moins polaire **c)** sont solubilisés dans 40 ml d'éthanol absolu sous azote. Ajouter 300 mg de Pd/c à 10 %. Mettre sous atmosphère d'hydrogène et agiter 2 h à température ambiante.

Filtrer le catalyseur, le rincer à l'éthanol, évaporer à sec.

Purifier par flash chromatographie sur 200 g de silice (éluant : CH₂Cl₂:MeOH ; 95:5).

Récupérer 1,81 g (soit 65 %) de produit.
[α]_{D} = + 20,9° à t = 20°C (c =1, MeOH).
RMN(CDCl₃) : δ 10,75 (1H, s, HONHCO) ; 9,05 (1H, s, HONHCO) ; 7,9(1H, q, CONHCH₃) ;
7,55 (1H, d, CONH) ; 7,05 (2H, d, CH(Ar)) ; 6,75 (2H, d, CH(Ar)) ; 5,42 (1H, s, OH) ;
4,5 (1H, m, CHα(Tyr)) ; 3,7 (3H, s, OCH₃) ; 2,6 (3H, d, CONHCH₃) ; 2,3 (1H, q, HONHCOCH-CH₃) ; 1,55 (2H, m, CH₂CH(CH₃)₂) ; 1,22 (1H, m, CH₂-CH(CH₃)₂) ; 0,85 -0,7-0,6 (9H, 3d, 3CH3).

### Exemple 2 : N-2-(S)[1(S)(hydroxycarbamoyl) éthyl]-2(S) hydroxy-4-méthylpentanoyl 3-méthyl-(S) valine N-méthylamide

### a) N-[2(S)[1(S)((1,1 diméthyl) éthoxycarbonyl)éthyl]-2 (S) hydroxy 4-méthylpentanoyl] 3 méthyl (S) valine N-méthylamide

Solubiliser 500 mg (1,9 mmoles) de l'intermédiaire **4** dans 25 ml de CH₂Cl₂ sec. Ajouter 277 mg (1,9 mmoles) de (S) méthyl 3-valine N-méthylamide, 1,05 g de PyBop et 744 µl (4 mmoles) de diisopropyléthylamine. Agiter une nuit à température ambiante. Laver par HCI N puis NaHCO₃ saturé. Sécher, évaporer, Reprendre par AcOEt.

Filtrer sur un gel de silice. Evaporer.

Récupérer 598 mg (80 %) de produit.
RMN (CDCl₃) : δ 7,6 (1H, q, CONHCH₃) ; 6,1 (1H, d large, CONH) ; 4,2 (1H, s, OH) ; 4,15 (1H, d, NH-CH-tBu) ; 2,75 (4H, m, NHCH₃ et tBuOCOCH) ; 1,85 (1H, m, CH₂CH) ;
1,7 (1H, m, CH₂CH(CH₃)₂); 1,5 (9H, s, (CH₃)₃CO) ; 1,4 (1H, m, CH₂-CH) ; 1,15 (3H, d, COCHCH₃);
1,1 (9H, s, CH-C(CH₃)₃) ; 0,95 et 0,8 (6H, 2d, CH-(CH₃)₂).

### b) N 2(S) [1(S) (hydroxycarbonyl)éthyl) -2(S)hydroxy-4 méthylpentanoyl] 3 méthyl (S) valine N-méthylamide

Solubiliser 580 mg du composé a) dans 6 ml de CH₂Cl₂ sec. Ajouter 6 ml d'acide trifluoroacétique. Agiter 5 h à température ambiante. Evaporer à sec.

Reprendre par 3 fois avec 10 ml de CH₂Cl₂ et 10 ml d'heptane et évaporer à sec.

Récupérer 480 mg de produit pur (100 %).
RMN (DMSO) : δ 8,05 (1H, q, CONHCH₃) ;7,05 (1H, d, CONH); 5,2 (1H, s très large, COOH) ; 4,1 (1H, d, NHCHCO) ; 2,7 (1H, q, HO₂CCHCH₃) ; 2,55 (3H, d, NHCH₃) ; 1,6 (2H, m, CH₂-CH) ; 1,45 (1H, m, CH₂-CH) ; 1 (3H, d, CO-CH-CH₃) ; 0,9 (9H, s, C-(CH₃)₃) ;
0,8 et 0,65 (6H,2d, CH-(CH₃)₂).

### c) N-2-(S) [1(S) (hydroxycarbamoyl éthyl]-2 (S) hydroxy-4- méthyl pentanoyl 3 méthyl-(S) valine N-méthylamide

Solubiliser 430 mg (1,3 mmoles) du composé **b)** dans 9 ml de DMF. Ajouter 204 mg (1,74 mmoles) de O-THP hydroxylamine, 195 mg (1,45 mmoles) de HOBT, 160 µl (1,45 mmoles) de N-méthylmorpholine, 284 mg (1,48 mmoles) de WSC, HCl et agiter une nuit à température ambiante. Evaporer à sec.

Reprendre avec 10 ml de THF et 10ml d'HCI N et agiter 1 h à température ambiante.

Extraire par AcOEt. Sécher, évaporer.

Cristalliser dans l'éther éthylique. Récupérer 205 mg (soit 45 %).
RMN (DMSO) : δ 10,8 (1H, s large, HONHCO) ; 9,1 (1H, s large, HONHCO) ;
8,05 (1H, q, CONHCH₃) ; 7,45 (1H, d, CONH) ; 5,5 (1H, s, OH) ; 4,15 (1H, d, NHCHCO) ;
2,6 (3H, d, NHCH₃) ; 2,5 (1H, q, COCHCH₃) ; 1,6 (2H, m, CH₂CH(CH₃)₂);
1,3 (1H, m, CH₂CH(CH₃)₂) ; 1( 3H, d, CH₃CHCO) ; 0,9 (9H, s, C(CH₃)₃) ;
0,85et 0,65 (6H, 2d, -CH-(CH₃)₂).

### Exemple 3 : N-2 (S) [(S) hydroxy (S) hydroxycarbamoyl méthyl] 2(S) hydroxy 4-méthyl pentanoyl L-4-chlorophénylalanine N-méthylamide.

### a) N-2-(S) [(S) 1,1 diméthyléthoxycarbonyl (S) hydroxyméthyl] 2(S) hydroxy 4-méthyl pentanoyl L-4-chlorophénylalanine N-méthylamide

A 495 mg (1,9 mmoles) de l'intermédiaire **5** dans 5 ml de dichlorométhane sec, ajouter une quantité catalytique de DMAP, 1 ml (5,8 mmoles) de DIPEA puis 0,74 ml (5,8 mmoles) de TMSCI. Agiter 30 mn à 20°C puis ajouter 255 mg (1,9 mmoles) de HOBT, 1 g (2 mmoles) de PyBop, 400 mg (2 mmoles) de 4-chlorophénylalanine N-méthylamide et 1 ml (5,8 mmoles) de DIPEA. Agiter une nuit à 20°C sous azote.

Ajouter 385 mg (2 mmoles) d'acide citrique solubilisé dans 35 ml de méthanol et agiter 30 mn à 20°C. Evaporer à sec. Reprendre par AcOEt. Laver par HCl N puis NaHCO₃. Sécher, évaporer.

Purifier par flash chromatographie (éluant : heptane:AcOEt ; 40:60).

Récupérer 500 mg (58 %) de produit pur. PF : 168°C.
RMN (CDCl₃) : δ 7,3 (2H, d, H(Ar)) ; 7,2 (2H, d, H(Ar)) ; 6,9 (1H, d, NHCO) ; 6,4 (1H, q, CONHCH₃); 4,6 (1H, m, NHCHCO) ; 4,1 (1H, d, CHOH) ; 4 (1H, s, OH); 3,65 (1H, d, OH); 3,15 (2H, m, CH₂Ar); 2,75 (3H, d, NHCH₃) ; 1,65 (3H, m, CH₂CH); 1,55 ( 9H, s, (CH₃)₃C); 1 et 0,85 (6H, 2d, (CH₃)₂CH).

### b) N-2-(S)[(S)hydroxy (S) hydroxycarbonyl méthyl] 2(S) hydroxy 4-méthylpentanoyl L-4-chlorophénylalanine N-méthylamide

Solubiliser 500 mg du composé a) dans 5 ml de CH₂Cl₂ sec. Ajouter 1,25 ml d'acide trifluoroacétique et agiter une nuit à 20°C. Evaporer à sec.

Reprendre par un mélange CH₂Cl₂ 50 / heptane : 50 et évaporer à sec. Récupérer 440 mg soit 100 %.
RMN (CDCl₃) : δ 7,9 (1H, d, CONHCH); 7,3 (2H, d, CH(Ar)) ; 7,15 (2H, d, CH(Ar));
5,65 (1H, q, CONHCH₃) ; 4,55 (1H, m, NHCHCO) ; 4 (1H, s, CHOH); 3,1 (2H, m, CH₂Ar) ;
2,8 (3H, d, NHCH₃); 1,75 (1H, m, CH(CH₃)₂); 1,65 (2H, m, CH₂CH) ; 1 et 0,8 6H, 2d, CH(CH₃)₂).

### c) N-2-(S) [(S) hydroxy (S) hydroxycarbamoyl méthyl] 2(S) hydroxy 4-méthyl pentanoyl L-4 chlorophényl alanine N-méthylamide

Solubiliser 440 mg (1,1 mmoles) du composé b) dans 8 ml de DMF sec. Ajouter à 0°C 180 mg (1,5 mmotes d'hydroxylamine OTHP, 180 mg (1,3 mmoles) de HOBT, 150 µl (1,3 mmoles) de N-méthylmorpholine, 250 mg (1,3 mmoles) de WSC. Agiter une nuit à 20°C. Evaporer à sec. Reprendre avec 4 ml de THF et 4 ml d'HCl N et agiter 4h à 20°C.

Concentrer à sec. Purifier par flash chromatographie (éluant : CH₂Cl₂: MeOH:AcOEt ; 90:10:1). Récupérer 160 mg (35 %) de produit pur. PF : 185°C.
RMN (DMSO) : δ 10,7 (1H, s large, HONHCO) ; 9 (1H, s large, HONHCO) ; 8,15 (1H, q, CONHCH₃); 7,65 (1H, d, CONH) ; 7,25 (2H, d, H(Ar)) ; 7,20 (2H, d, H(Ar)) ; 6 et 5 (2H, 2 s très larges, OH) ;
4,45 (1H, m,COCHNH) ; 3,85 (1H, s, CHOH) ; 3,05 (1H, dd, CH₂Ar) , 2,95 (1H, dd, CH₂Ar) ;
2,5 (3H,d, NHCH₃) ; 1,55 (1H, dd, CH₂-CH) ; 1,4 (1H, m, CH₂CH) ; 1,25 (1H, dd, CH₂CH) ; 0,8 et 0,6 (6H, 2d, CH(CH₃)₂).

Les exemples 4 à 28 ont été synthétisés de la même manière que l'exemple 2, à l'exception de l'exemple 7, synthétisé de la même manière que l'exemple 1.

### Exemple 4 : N-2(S) [1(S) (hydroxycarbamoyl)éthyl] 2(S) hydroxy 4-méthyl pentanoyl L-phényl alanine N-méthylamide

P.F. : 174,2° (dec)
RMN (DMSO) : δ 10.75 (1H, s large, HONHCO); 9,05 (1H, s large. HONHCO) ;
7,95 (1H, q, CONHCH₃) ; 7,60 (1H, d, CONH) ; 7,20 (5H, m, H(Ar)) ; 5,40 (1H, s large, COH) ;
4,45 (1H, m, NHCHCO) ; 2,95 (2H, dd, CH₂Ar) ; 2,60 (3H, d, NHCH₃) ; 2,30 (1H, q, COCHCH₃) ; 1,60 (1H, ddd, CH(CH₃)) ; 1,55 (1H, dd, CH₂CH) ; 1,20 (1H, m, CH₂CH) ; 0,85 (3H, d, CH₃CH) ; 0,70 et 0,50 (6H, 2d, CH(CH₃)₂).

### Exemple 5 : N-2(S)[-1-(S)(hydroxycarbamoyl)-éthyl]-2(S)hydroxy-4-méthyl pentanoyl O méthyl-L-tyrosine phénylméthylamide

RMN (DMSO) : δ 10,75 (1H, s large, HONHCO) ; 9,05 (1H, s large, HONHCO) ; 8,5(1H, t, NHCH₂Ar)
; 7,65 (1H, d, CONH) ; 7,25 (2H, m, CH(Ar)) ; 7,1(5H, m, CH(Ar)) ; 6,8(2H, d, CH(Ar)) ;
5,4 (1H, s, OH) ; 4,6 (1H, m, NHCHCO) ; 4,25 (2H, m, NHCH₂Ar) ; 3,7 (3H, s, OCH₃);
2,9 (2H, m, CHCH₂Ar) ; 2,3 (1H, q, COCHCH₃) ; 1,55 (2H, m, CH₂CH) ; 1,2 (1H, dddd, CH(CH₃)₂);
0,85 - 0,7 et 0,55 (9H, 3d, CH₃CH et CH(CH₃)₂).

### Exemple 6 : N-2(S)[-1(S) (hydroxycarbamoyl) 1(S) éthyl]-2(S) hydroxy 4-méthyl pentanoyl leucinyl glycine méthyl ester

RMN (DMSO) : δ 10,8 (1H, s large, HONHCO) ; 9,1 (1H, s large, HONHCO) ; 8,5 (1H, t, CONH) ;
7,55 (1H, d, CONH); 5,45 (1H, s, OH) ; 4,45 (1H, m, CHCO) ; 3,85 (2H, d, NHCH₂CO) ;
3,60 (3H, s, OCH₃) ; 2,45 (1H, q, COCH-CH₃) ; de 1,8 à 1,35 (4H, m, CH₂-CH(CH₃)₂ et CH₂CH) ; de 1 à 0,6 (12H, m, -CH(CH₃)₂ 2 fois) ; 0,6 (3H, d, COCHCH₃).

### Exempte 7 : N-2(S) [-1-(S)hydroxycarbamoyl 1(S)éthyl] 2(S) hydroxy-3 méthyl butanoyl O-méthyltyrosine N-méthylamide

Synthétisé avec l'intermédiaire **9**.
RMN (DMSO) : δ 10,83 (1H, s large, OHNHCO) ; 9,05 (1H, s large, HONHCO) ; 7,9 (1H, q, NHCH₃); 7,4 (1H, d, NHCH); 7,1 (2H, d, H(Ar)) ; 6,8 (2H, d, H(Ar)) ; 5,60 (1H, s, OH) ; 4,48 (1H, m, NHCHCO) 3,7 (3H, s, OCH₃) ; 2,85 (2H, 2dd, CH₂Ar) ; 2,6 (1H, q, CHCH₃) ; 2,58 (3H, d, NHCH₃);
1,75 (1H, m, CH(CH₃)₂) ; 0,85 et 0,8 (6H, 2d, CH(CH₃)₂) ; 0,65 (3H, d, CHCH₃).

### Exemple 8 : N-2(S)[1(S)(Hydroxycarbamoyl)-1 éthyl] 2(S) hydroxy 4-méthyl pentanoyl L-4-nitrophénylalanine N-méthylamide

Déc. : 207°C (dec)
[α]_{D} = + 17,7° à t = 20°C (c = 1, MeOH).
RMN (DMSO) : δ 10,7 (1H, s large, HONHCO) ; 9,1 (1H, s large, HONHCO) ; 8,21 (2H, d, H(Ar)) ;
7,95 (1H, q, CONHCH₃) ; 7,7 (1H, d, CONH) ; 7,5 (2H, d, H(Ar)) ; 5,4 (1H, s, OH) ;
4,65 (1H, dd, NHCHCO) ; 3,1 (2H, d, CH₂Ar) ; 2,6 (3H, d, NHCH₃) ; 2,25 (1H, q, COCHCH₃); de 1,7 à 1,4 (2H, m, CH₂CH) ; 1,2 (1H, dd, CH₂CH) ; 0,85 (3H, d, CHCH₃) ;
0,75 et 0,45 (6H, 2d, CH(CH₃)₂).

### Exemple 9 : N-2(S)[1(S) (hydroxycarbamoyl) 1(S) éthyl]2-(S) hydroxy-4-méthyl pentanoyl L-4-amino phénylalanine N-méthylamide

RMN (DMSO) : δ 7,82 (1H, q, CONHCH₃) ; 7,50 (1H, d, CONH) ; 6,80 (2H, d, H(Ar)) ; 6,40 (2H, d, H(Ar)); 4,85 (2H, s large, NH₂) ; 4,40 (1H, dd, NHCHCO) ; 2,70 (2H, d, CH₂Ar) ; 2,55-(3H, d, CH₃NH) ; 2,2 (1H,q, COCHCH₃) ; de 1,7 à 1,45 (2H, m, CH₂CH) ; 1,25 (1H, m, CH₂CH) ;
0,85 (3H, d, CHCH₃) ; 0,70 (6H, d, CH(CH₃)₂).

### Exemple 10 : N-2(S) [-1(S) (hydroxycarbamoyl)-1 éthyl]-2(S) hydroxy-4-méthyl pentanoyl L-4-chlorophénylalanine N-méthylamide

[α]_{D} = + 17,8° à t = 20°C (c = 1, MeOH)
RMN (DMSO): δ 10,75 (1H, s large, HONHCO) ; 9,05 (1H, HONHCO) ; 7,9 (1H, q, CONHCH₃) ;
7,6 (1H, d, CONH) ; 7,25 (2H, d, 2HAr) ; 7,2 (2H, d, 2H(Ar)) ; 5,4 (1H, s large, OH) ;
4,5 (1H, dd, COCHNH) ; 2,9 (2H, d, CH₂Ar) ; 2,55 (3H, d, NHCH₃) ; 2,25 (1H, q, CHCH₃);
1,55 (1H, m, CH₂CH) ; 1,5 (1H, m, CH₂CH) ; 1,2 (1H, m, CH₂CH) ; 0,85 (3H, d, CHCH₃);
0,65 et 0,5 (6H, 2d, CH(CH₃)₂).

### Exemple 11 : N-2(S)[-1(S)(hydroxycarbamoyl)-1 éthyl]-2(S) hydroxy 4-méthyl pentanoyl L-4-bromophénylalanine N-méthylamide

PF : 214°C (déc.)
RMN (DMSO) : δ 10,75 (1H, s large, OHNHCO) ; 9,05 (1H, s large, HONHCO) ;
7,95 (1H, q, CONHCH₃) ; 7,65 (1H, d, CONH) ; 7,4 (2H, d, H(Ar)) ; 7,1(2H, d, 2H(Ar)) ;
5,4 (1H, s, OH) ; 4,5 (1H, dd, NHCHCO) ; 2,9 (2H, d, CH₂(Ar)) ; 2,55 (3H, d, NHCH₃) :
2,25 (1H, q, CHCH₃) ; 1,5 (2H, m, CH₂CH) ; 1,2 (1H, m, CH₂CH) ; 0,8 (3H, d, CHCH₃) ; 0,65 et 0,5 (6H, 2d, CH(CH₃)₂).

### Exemple 12a : N-2(S)[-1(S) hydroxycarbamoyl-1(S) éthyl]-2(S) hydroxy-4-méthyl pentanoyl L-4-chlorophénylalanine N-(2-(4-morpholino)1-éthyle) -amide

PF : 179°C (déc.)
RMN (DMSO) : δ 10,75 (1H, s large, OHNHCO) ; 9,05 (1H, s large, HONHCO) ;
7,95 (1H, t large, CONHCH₂₋) ; 7,65 (1H, d, CONH) ; 7,25 (4H, dd, H(Ar)) ; 5,4 (1H, s, OH) ;
4,5 (1H, m, CHCO) ; 3,55 (4H, m, (CH₂)₂-O) ; 3,15 (2H, q, CONHCH₂) ; 2,9 (2H, dd, CH₂Ar) ;
2,35 (4H, m, N(CH₂)₂) ; 2,3 (3H, m, CONH-CH₂-CH₂, CHCH₃) ; 1,55 (2H, m, CH₂CH) ; 1,2 (1H, dd, CH₂CH) ; 0,85 (3H, d, CHCH₃) ; 0,7 et 0,55 (6H, 2d, CH(CH₃)₂).

### Exemple 12b : Chlorhydrate de l'exemple 12a

RMN (DMSO) : δ 10,8 (s, 1H, HONH) ; 10,7 (s large, 1H, (CH₂)₃N⁺H, Cl⁻) ;
9,05 (s, 1H, HONH) ; 8,5 (m, 1H, CONHCH₂) ; 7,65 (d, 1H, CONH) ; 7,3 (m, 4H, H(Ar)) ;
5,4 (s, 1H, OH) ; 4,55 (1H, m, NHCHCO) ; 4-3,6 (m, 4H, (CH₂)₂-O) ;
3,5-2,9 (m, 10H, CONHCH₂ et (CH₂)₃N⁺H, Cl⁻ et CH₂Ar) ; 2,25 (q, 1H, CHCH₃) ;
1,6 (m, 2H, CH₂CH) ; 1,2 (m, 1H, CH₂CH) ; 0,85 (d, 3H, CHCH₃) ;
0,7 et 0,5 (2d, 6H, CH(CH₃)₂).

### Exemple 13 : N-2(S) [-1-(S) (Hydroxycarbamoyl)-1 éthyl]-2(S) hydroxy-4-méthyl pentanoyl L-4-chlorophénylalanine N-(2 méthylthio-1-éthyle) amide

RMN (DMSO) : δ 10,7 (1H, s large, HONHCO) ; 9,05 (1H, s, OHNHCO) ; 8,2 (1H, t, CONH) ;
7,6 (1H, d, CONH) ; 7,25 (4H, q, H(Ar)) ; 5,35 (1H, s, OH) ; 4,6 (1H, m, NHCHCO) ;
3,2 (2H, dd, CH₂-Ar) ; 2,9 (2H, m, NHCH₂) ; 2,45 (2H, m, CH₂S) ; 2,25 (1H, q, CHCH₃);
2,05 (3H, s, SCH₃) ; 1,55 (2H, m, CH₂CH) ; 1,15 (1H, m, CH₂CH) ; 0,85 (3H, d, CHCH₃);
0,65 et 0,5 (6H, 2d, CH(CH₃)₂).

### Exemple 14 : N-2(S)[1(S) hydroxycarbamoyl-1éthyl]-2(S) hydroxy 4-méthyl pentanoyl L-4-iodophénylalanine N-méthylamide

RMN (DMSO) : δ 10,75 (1H, sl, OHNHCO) ; 9,05 (1H s large, HONHCO) ; 7,95 (1H, q, CONHCH₃) ; 7,63 (1H, d, CONH) ; 7,6 (2H, d, H(Ar)) ; 7 (2H, d, HAr) ; 5,4 (1H, s, OH) ; 4,55 (1H, m, COCHNH) ; 2,88 (2H,dd, CH₂Ar) ; 2,55 (3H, d, NHCH₃) ; 2,25 (1H, q, CHCH₃) ; 1,55 (2H, m, CH₂CH) ;
1,2 (1H, m, CH₂CH) ; 0,85 (3H, d, CHCH₃) ; 0,7 et 0,5 (6H, 2d, CH(CH₃)₂).

### Exemple 15 : N-2(S)[1(S) hydroxycarbamoyl-1 éthyl]2(S) hydroxy 4-méthylpentanoyl L-4-iodophénylalanine N-(2 méthylthio-1-éthyl) amide

RMN (DMSO) : δ 10,7 (1H, s large, OHNHCO) ; 9,1 (1H, s large, HONHCO) ;
8,2 (1H, dd, CONHCH₂) ; 7,6 (3H, d, H(Ar)et d, CONH) ; 7,05 (2H, d, H(Ar)) ; 5,3 (1H, s, OH) ;
4,6 (1H, m, NHCHCO) ; 3,3 (2H, m, CH₂NH) ; 2,9 (2H, m, CH₂Ar) ; 2,5 (3H, CH₂S et CHCH₃) ;
2,1 (3H, s, SCH₃); 1,6 (2H, m, CH₂CH) ; 1,2 (1H, m, CH₂CH) ; 0,9 (3H, d, CH-CH₃) ;
0,7 et 0,5 (6H, 2d, CH(CH₃)₂).

### Exemple 16 : N-2(S) [1(S) hydroxy carbamoyl-1 éthyl]-2(S) hydroxy 4-méthylpentanoyl L-4-fluorophénylalanine N-méthylamide

P.F. : 186.5°C
RMN (DMSO) : δ 10,75 (1H, s, HONHCO) ; 9,05 (1H, s large, HONHCO) ; 7,9 (1H, q, CONHCH₃); 7,65 (1H, d, CONHCH) ; 7,2 (2H, dd, H(Ar)) ; 7,05 (2H, dd, H(Ar)) ; 5,4 (1H, s, COH) ;
4,55 (1H, m, COCHNH) ; 2,9 (2H, d, CH₂Ar) ; 2,6 (3H, d, NHCH₃) ; 2,3 (1H, q, CHCH₃) ;
1,6 (2H, m, CH₂CH) ; 1,2 (1H, m, CH₂CH) ; 0,85 (3H, d, CHCH₃) ; 0,7 et 0,55 (6H, 2d, CH(CH₃)₂).

### Exemple 17 : N-2(S) hydroxy 3(S) hydroxycarbamoyl 2(S) 1(2-méthyl) propyl hexanoyl L-4-chlorophénylalanine N-méthyl amide

RMN (DMSO) : δ 7,9 (1H, q, CONHCH₃) ; 7,6 (1H, d, CONH) ; 7,2 (4H, m, H(Ar)) ;
5,3 (1H, s large, OH) ; 4,5 (1H,m, NHCHCO) ; 2,9 (2H, d, CH₂Ar) ; 2,6 (3H, d, NHCH₃) ;
2,15 (1H, q, CHCH₃) ; de 1,5 à 0,8 (7H, m, CH₂CH et CHCH₂CH₂CH₃) ;
0,8 et 0,7 (9H , 1 t et 2d, 3CH₃).

### Exemple 18 : N-2(S)[1(S)hydroxy carbamoyl-1 éthyl]-2 (S) hydroxy 4-méthylpentanoyl L-3,4-dichlorophénylalanine N-méthylamide

PF : 216°C (Déc)
RMN (DMSO) : δ 10,7 (1H, s, HONHCO) ;9,05 (1H, s, HONHCO) ; 7,95 (1H, q, CONHCH₃) ;
7,7 (1H, d, CONH) ; 7,5 (2H, 1d et 1s, H(Ar)) ; 7,2 (1H, d, H(Ar)) ; 5,45 (1H, s, OH) ;
4,55 (1H, m, NHCHCO) ; 2,9 (2H, d, CH₂Ar)) ; 2,6 (3H, d, CONHCH₃) ; 2,25 (1H, q, CHCH₃) ;
1,55 (2H, m, CH₂CH) ; 1,2 (1H, m, CH₂CH) ; 0,85 (3H, d, CHCH₃) ; 0,6 et 0,5 (6H, 2d, CH(CH₃)₂).

### Exemple 19 : N-2(S)[1(S)hydroxy carbamoyl-1 éthyl]-2 (S) hydroxy 4-méthylpentanoyl L-3.4-dichlorophénylalanine N-hydroxyéthyloxyethylamide

RMN (DMSO) : δ 8,1 (1H, t, NHCH₂) ; 7,7 (1H, d, CONH) ; 7,5 (2H, dd, H(Ar)) ; 7,25 (1H, dd, H(Ar)) ; 5,5 (1H, s large, COH) ; 4,6 (1H, m, COCHNH) ; 3,5 (2H, t, CH₂OH) ; 3,4 (4H, m, CH₂-O-CH₂)) ;
3,2 (2H, m, NHCH₂) ; 3 (2H, d, CH₂Ar)) ; 2,3 (1H, q, CHCH₃) ; 1,5 (2H, m, CH₂CH) ;
1,2 (1H, m, CH₂CH) ; 0,85 (3H, d, CHCH₃) ; 0,7 et 0,5 (6H, 2d, CH(CH₃)₂).

### Exemple 20 : N-2(S)[1(S)hydroxy carbamoyl-1 éthyl]-2 (S) hydroxy 4-méthylpentanoyl L-2,4-dichlorophénylalanine N-méthylamide

Les diastéréoisomères ont été séparés à l'étape a (diastéréoisomère le moins polaire)
PF: 193,5°C
RMN (DMSO) : δ 10,8 (1H, s, CONHOH) ; 9,1 (1H, s, CONHOH) ; 7,9 (1H,q, CONHCH₃) ;
7,7 (1H, d, CONH) ; 7,5 (1H, s, H(Ar)) ; 7,4 (2H, 2d, H(Ar)) ; 5,4 (1H, s, OH) ; 4,6 (1H, m, COCHNH) ; 3,1 (2H, m, CH₂(Ar)) ; 2,6 (3H, d, NHCH₃) ; 2,3 (1H, q, CHCH₃) ; 1,5 (2H, m, CH₂CH) ;
1,2 (1H, m, CH₂CH) ; 0,8 (3H, d, CHCH₃) ; 0,7 et 0,5 (6H, 2d, CH(CH₃)₂).

### Exemple 21 : N-2(S)[1(S) hydroxycarbamoyl-1 éthyl]-2(S) hydroxy-4-méthylpentanoyl L-3.4-dichlorophénylalanine N-(2-méthylthio-1-éthyle)amide

RMN (DMSO) : δ 10,75 (1H, s, HONHCO) ; 9,05 (1H, s, HONH) ; 8,15 (1H, t, CONHCH₂) ;
7,7 (1H, d, CONHCH) ; 7,5 (1H, m, H(Ar)) ; 7,2 (1H, m, H(Ar)) ; 5,4 (1H, s, OH) ;
4,6 (1H, m, NHCHCO) ; 3,25 (2H, q, CONHCH₂) ; 2,95 (2H, m,CH₂Ar) ; 2,4 (2H, m, CH₂S) ;
2,28 (1H, q, COCHCH3) ; 2,05 (3H, s, SCH₃) ; 1,55 (2H, m, CH₂CH(CH₃)₂) ;
1,2 (1H, m, CH₂CH(CH₃)₂) ; 0,85 (3H, d, COCHCH₃) ; 0,65 et 0,5 (6H, 2d, CH(CH₃)₂

### Exemple 22 : N-2(S)-[1(S) hydroxycarbamoyl-1 éthyl]-2(S) hydroxy-4-méthylpentanoyl L 3-chlorophénylalanine N-méthylamide

Les diastéréoisomères ont été séparés à l'étape **a** (diastéréoisomère le moins polaire)
RMN (DMSO) : δ 10,75 (1H, s, HONHCO) ; 9,1 (1H, s, HONHCO) ; 7,95 (1H, q, CONHCH₃) ;
7,7 (1H, dd, CONHCH) ; de 7,3 à 7,1 (4H, m, H(Ar)) ; 5,4 (1H, s, COH) ; 4,55 (1H, q, NHCHCO) ;
2,9 (2H, d, CH₂Ar) ; 2,6 (3H, d, NHCH₃) ; 2,24 (1H, q, COCHCH₃) ;
de 1,7 à 1,45 (2H, m, CH₂CH(CH₃)₂) ; 1,2 (1H, m, CH₂CH(CH₃₎₂₎ ; 0,85 (3H, d, COCHCH₃) ;
0,7 et 0,5 (6H, 2d, CH(CH₃)₂)

### Exemple 23 : N-2(S) [1(S)hydroxycarbamoyl-1 éthyl]-2(S) hydroxy 4-méthyl pentanoyl L 3,4-dichlorophénylalanine N-(2-(2-méthoxyéthoxy)1-éthyl)amide

RMN (DMSO) : δ 10,75 (1H, s très large, OHNHCO) ; 9,05 (1H, s large, HONHCO) ;
8,15 (1H, m, CONHCH₂) ; 7,72 (1H, d, CONHCH) ; 7,5 (1H, d, H(Ar)) ; 7,48 (1H, dd, H(Ar)) ;
7,25 (1H, dd, H(Ar)) ; 5,45 (1H, s, COH) ; 4,65 (1H, q, NHCHCO) ;
de 3,5 à 3,2 (11H, m, CONHCH₂CH₂OCH₂CH₂OCH₃) ; 2,92 (2H, d, CH₂Ar) ;
2,25 (1H, m, COCHCH₃) ; 1,55 (2H, m, CH₂CH(CH₃)₂) ; 1,2 (1H, m, CH₂CH(CH₃)₂) ;
0,85 (3H, d, COCHCH₃) ; 0,7 et 0,5 (6H, 2d, CH(CH₃)₂)

### Exemple 24 : N-2(S)[1(S)hydroxycarbamoyl-1 éthyl]-2(S)hydroxy-4-méthylpentanoyl L-2,6- dichlorophénylalanine N-méthylamide

Les diastéréoisomères ont été séparés à l'étape **a** (diastéréoisomère le moins polaire)
RMN (DMSO) : δ 10,75 (1H, s, HONHCO) ; 9,1 (1H, s, HONHCO) ; 8,1 (1H, q, CONHCH₃) ;
7,65 (1H, d, CONHCH) ; 7,4 (2H, d, H(Ar)) ; 7,2 (1H, t, H(Ar)) ; 5,45 (1H, s, COH) ;
4,75 (1H, m, NHCHCO) ; 3,3 (1H, dd, CH₂Ar) ; 3,1 (1H, dd, CH₂Ar) ; 2,6 (3H, d, NHCH₃) ;
2,2 (1H, q, COCHCH₃) ; 1,55 (2H, m, CH₂CH(CH₃)₂) ; 1,2 (1H, m, CH₂CH(CH₃)₂) ;
0,85 (3H, d, COCHCH₃) ; 0,6 et 0,3 (6H, 2d, CH(CH₃)₂)

### Exemple 25 : N-2(S)[1(S) hydroxycarbamoyl-1 éthyl]-2(S)hydroxy-4-méthylpentanoyl L-2,5-dichlorophénylalanine N-méthylamide

Les diastéréoisomères ont été séparés à l'étape **a** (diastéréoisomère le moins polaire)
PF : 209-212°C
RMN (DMSO) : δ 10,7 (1H, s, HONHCO) ; 9.1 (1H, s, HONHCO) ; 7,8 (1H, q, CONHCH₃) ;
7,7 (1H, d, CONHCH) ; 7,4 (2H, m, H(Ar)) ; 7,3 (1H, dd, H(Ar)) ; 5,4 (1H, s, COH) ;
4,6 (1H, m, NHCHCO) ; 3,0 (2H, m, CH₂Ar) ; 2,6 (3H, d, NHCH₃) ; 2,3 (1H, m, COCHCH₃) ;
1,5 (2H, m, CH₂CH(CH₃)₂) ; 1,2 (1H,m, CH₂CH(CH₃)₂) ; 0,9 (3H, d, COCHCH₃) ;
0,7 et 0,5 (6H, 2d. CH(CH₃)₂)

### Exemple 26 : N-2(S)[1(S) hydroxycarbamoyl-1 éthyl]-2(S)hydroxy-4-méthylpentanoy L-tryptophan N-méthylamide

RMN (DMSO) : δ 10,75 (1H, s, HONHCO) ; 9,1 (1H, s, HONHCO); 7,95 (1H, q, NHCH₃) ;
7,65 (1H, d, CONHCH) ; 7,5 (2H, m, H(Ar)) ; 7,25 (1H, d, H(Ar)) ; de 7,15 à 6,9 (3H, m, H(Ar)) ;
5,4 (1H, s, COH) ; 4,5 (1H, m, COCHNH) ; 3 (2H, m, CH₂Ar) ; 2,55 (3H, d, NHCH₃) ;
2,2 (1H, q, COCHCH₃) ; de 1,65 à 0,9 (3H, m, CH₂CH(CH₃)₂) ; 0,75 (3H, d, COCHCH₃) ;
0,6 et 0,4 (6H, 2d, CH(CH₃)₂)

### Exemple 27 : N-2(S)[1(S) hydroxycarbamoyl-1 éthyl]-2(S)hydroxy-4-méthylpentanoyl L

### 4-chlorophénylalanine N-(2-cyano-1-éthyl) amide

IR : ν CN 2254 cm⁻¹
RMN (DMSO) : δ 10,75 (1H, s, HONHCO) ; 9,08 (1H, s large, HONHCO) ; 8,5 (1H, q, CONHCH₃) ; 7,65 (1H, d, CONHCH) ; 7,25 (4H, dd, H(Ar)) ; 5,4 (1H, s, COH) ; 4,6 (1H, m, COCHNH) ;
3,2 (2H, m, CONHCH₂CH₂) ; 2,95 (2H, m, CH₂Ar) ; 2,6 (2H, t, CH₂CN) ; 2,25 (1H, q, COCHCH₃) ; 1,55 (1H, m, CH₂CH(CH₃)₂) ; 1,25 (2H, m, CH₂CH(CH₃)₂) ; 0,95 (3H, d, COCHCH₃) ;
0,65 et 0,5 (6H, 2d, CH(CH₃)₂)

### Exemple 28 : N-2(S)[1(S) hydroxycarbamoyl-1 éthyl]-2(S)hydroxy-4-méthylpentanoyl L-4-chlorophénylalanine N-oximino-2-amino-2-éthylamide

Ce composé a été préparé à partir de l'exemple **27**.

Solubiliser 130 mg (0,29 mmoles) de composé de l'exemple **27** dans 10 ml de n-butanol, ajouter 1,01 ml d'une solution d'hydroxylamine base 0,56 M (soit 0,57 mmoles) et chauffer une nuit à 80°C sous atmosphère d'azote; Evaporer à sec. Purifier par flash chromatographie sur 10 g de silice (injection sèche sur 1 g de SiO₂). Eluer CH₂Cl₂ 90 : MeOH 10. Récupérer 74 mg soit 53 % de mousse.
RMN (DMSO) : δ 10,7 (1H, s large, HONHCO) ; 9 (1H, s, HONHCO) ; 8,8 (1H, s, C=NOH) ;
8 (1H, m CONHCH₂) ; 7,62 (1H, m, CONHCH) ; 7,25 (2H, m, H(Ar)) ; 7,15 (2H, m, H(Ar)) ;
5,35 (2H, s large, H₂NC=NOH) ; 4,6 (1H, q, COCHNH) ; 4 (1H, s, COH) ;
3,15 (2H, m, CONHCH₂-) ; 2,85 (2H, m, CH₂Ar) ; de 2,2 à 2,02 (3H, m, COCHCH₃ et CH₂-C=NOH); de 1,7 à 1 (3H, m, CH₂CH(CH₃)₂) ; 0,8 (3H, d, COCHCH₃) ; 0,65 et 0,55 (6H, 2d, CH(CH₃)₂)

### Exemple 29 : N-2-(S) [(S) hydroxy-(S)hydroxycarbamoylméthyl)-2(S) hydroxy-4-méthyl]pentanoyl O-méthyl tyrosine N-méthylamide

Synthétisé de la même manière que l'exemple **3**.

PF : 199°C (déc.)
RMN (DMSO) : δ 10,72 (1H, s, HONH) ; 9 (1H, s, HONH) ; 8,2 (1H, q, CONHCH₃) ; 7,6 (1H, d, CONH) ; 7,05 (2H, d, CH(Ar)) ; 6,8 (2H, d, CHAr) ; 6,1 (1H, d, OH) ; 4,85 (1H, s, OH) ; 4,38 (1H, m, NHCHCO) ; 3,9 (1H, d, CH-O) ; 3,7 (3H, s, OCH₃) ; 2,92 (2H, 2dd, CH₂Ar) ;
2,6 (3H, d, NHCH₃) ; 1,55 (1H, dd, CH₂CH) ; 1,45 (1H, m, CH(CH₃)₂) ; 1,28 (1H, dd, CH₂CH₂) ;
0,8 et 0,65 (2d, CH(CH₃)₂).

### Exemple 30 : N-2(S)[1(S) hydroxycarbamoylméthyl]-2(S)hydroxy-4-méthylpentanoyl L-3,4- dichlorophénylalanine N-méthylamide

PF: 194,1°C
RMN (DMSO) : δ 10,7 (1H, s, large, HONHCO) ; 9,0 (1H, s, HONHCO) ; 8,25 (1H, q, CONHCH₃) ; 7,7 (1H, d, CONHCH) ; 7,5 (1H, d, H(Ar)) ; 7,45 (1H, m, H(Ar)) ; 7,2 (1H, dd, H(Ar)) ;
6,05 (1H, d, COCHOH) ; 4,9 (1H, s, COH) ; 4,5 (1H, m, COCHNH) ; 3,9 (1H, d, COCHOH) ;
de 3,15 à 2,2 (2H, m, CH₂Ar) ; 2,6 (3H, d, CONHCH₃) ; de 1,5 à 1,25 (3H, m, CH₂CH(CH₃)₂) ;
0,8 et 0,6 (6H, 2d, CH(CH₃)₂)

### Exemple 31 : N-2(S)[(S)hydroxycarbamoyl(S) méthoxy]méthyl 2(S) hydroxy-4 méthyl pentanoyl O-méthyl tyrosine N-méthylamide

Synthétisé de la même manière que l'exemple **2** à partir de l'intermédiaire **6**.
RMN (DMSO): δ 10,9 (1H, s large, OHNHCO) ; 9,2 (1H, s large, HONHCO) ;
8,05 (1H, q, CONHCH₃) ; 7,55 (1H, d, CONH) ; 7,05 (2H, d, CH(Ar)) ; 6,8 (2H, d, CH(Ar)) ;
5,05 (1H, s large, OH) ; 4,4 (1H, m, NHCHCO) ; 3,7 (3H, s, OCH₃) ; 3,6 (1H, s, CHOCH₃);
3,2 (3H, s, CHOCH₃) ; 2,9 (2H, m, CH₂Ar) ; 2,6 (3H, d, NHCH₃) ; 1,55 (3H, m, CH₂CH) ;
0,8 et 0,65 (6H, 2d, CH(CH₃)₂).

### Exemple 32 : N-2(S) [1(S)(hydroxycarbamoyl)éthyl]2(S) hydroxy 5-phényl pentanoyl L-3-cyclohexylalanine 2 phényléthylamide

Synthétisé à partir de l'intermédiaire **8**.
RMN (DMSO) : δ 10,8 (1H, s, HONHCO) ; 9,1 (1H, s, HONHCO) ; 8,05 (1H, t, CONHCH₂),
7,5 (1H, d, CONH) ; de 7,3 à 7 (10H, m, H(Ar)) ; 5,45 (1H, s, OH) ; 4,35 (1H, m, NHCHCO);
3,18 (2H,q, NHCH₂CH₂) ;2,6 (2H, t, CH₂CH₂Ar) ; 2,5 (3H, m, CHCH₃ et CH₂Ar) ;
de 1,8 à 0,7 (17 H, m : H cyclohex, CH₂-cyclohex ; C(OH)CH₂CH₂) ; 1 (3H, d, CHCH₃).

### Exemple 33 : N-2(S)[1(S)(hydroxycarbamoyl)éthyl] 2(S) hydroxy 5-phénylpentanoyl 1-3.4-dichlorophénylalanine N-méthylamide

Synthétisé à partir de l'intermédiaire **8**.
RMN (DMSO) : δ 10,7 (1H, s, CONHOH) ; 9 (1H, s, CONHOH) ; 7,9 (1H, q, CONHCH3) ; 7,7 (1H, d, CONH) ; 7,5 (2H, 1d et 1s. H(Ar)) ; de 7,2 à 7 (6H, m, H(Ar)) ; 5,4 (1H, s, OH) ; 4,5 (1H, m, COCHNH) ; 2,9 (2H, m, CH₂Ar) ; 2,7 (1H, q, CHCH₃) ; de 2,5 à 2,3 (5H, m, CH₂CH₂Ar et NHCH₃) ; de 1,6 à 1,2 (4H, m, CH₂CH₂CH₂Ar) ; 0,5 (3H, d, CHCH₃).

### Exemple 34 : N-2-(S)[(S)-hydroxy-(S)hydroxycarbamoylméthyl)-2(S)-hydroxy-4-méthyl]pentanoyl-o-méthyl-3-méthyl-(S) valine N-méthylamide

Synthétisé de la même manière que l'exemple **3**.
RMN (DMSO) : δ 9 (s, 1H, HONH) ; 8,05 (q, 1H, CONHCH₃) ; 7,5 (d, 1H, CONH);
5,65 (d, 1H, OH) ; 5,35 (s, 1H, OH) ; 4,1 (d, 1H, NHCHCO) ; 3,9 (d, 1H, CHOH) ;
2,55 (d, 3H, NHCH₃); 1,6, (m, 2H, CH₂CH) ; 1,25 (m, 1H, CH₂CH) ;
0,9 et 0,7 (2d, 6H, CH(CH₃)₂).

### Exemple 35a : N-2 (S)[1(S)-hydroxycarbamoyl-1 (S) éthyl]-2 (S)-hydroxy-4-méthyl pentanoyl L-(3,4)-dichlorophénylalanine N-(2-(4-morpholino)1-éthyl) amide

Synthétisé de la même manière que l'exemple **2**.
RMN (DMSO) : δ 10,7 (s, 1H, HONH) ; 9,05 (s, 1H, HONH) ; 8 (m, 1H, CONHCH₂) ;
7,7 (d, 1H, CONH) ; 7,5 (m, 2H, H(Ar)) ; 7,2 (dd, 1H, H(Ar)) ; 5,4 (s, 1H, OH) ;
4,6 (m, 1H, NHCHCO) ; 3,5 (m, 4H, (CH₂)₂-O) ; 3,2 (m, 2H, CONHCH₂) ;
2,9 (d, 2H, CH₂Ar) ; 2,4-2,2 (m, 7H, N(CH₂)₂ et CONHCH₂CH₂ et CHCH₃) ;
1,55 (m,2H, CH₂CH) ; 1,2 (m, 1H, CH₂CH) ; 0,8 (d, 3H, CHCH₃) ;
0,7 et 0,5 (2d, 6H, CH(CH₃)₂).

### Exemple 35b : Chlorhydrate de l'exemple 35a

RMN (DMSO) : δ 10,8 (m, 1H, (CH₂)₃N⁺H, Cl⁻) ; 10,7 (s, 1H, HONH) ;
9 (s large, 1H, HONH) ; 8,5 (m, 1H, CONHCH₂) ; 7,8 (d, 1H, CONH) ; 7,5 (m, 2H, H(Ar)) ;
7,2 (dd, 1H, H(Ar)) ; 5,4 (s large, 1H, OH) ; 4,55 (m, 1H, NHCHCO) ;
4-3,4 (m, 6H, (CH₂)₂-O et CONHCH₂) : 3,1-2,9 (m, 8H, (CH₂)₃N⁺H, Cl- et CH₂Ar);
2,2 (q, 1H, CHCH₃) ; 1,5 (m, 2H, CH₂CH); 1,2 (m, 1H, CH₂CH) ; 0,8 (3H, d, CHCH₃);
0,65 et 0,45 (2d, 6H, CH(CH₃)₂).

### Exemple 36 : N-2(R)-[(1-thioéthyl)] 2(R) hydroxy 4-méthyl pentanoyl -o-méthyl-L-tyrosine méthylamide

### a) 2(R)-[1(4(méthoxy)benzylmercapto)éthyl]2(R)hydroxy 4-méthylpentanoyl-o-méthyl-L-tyrosine méthylamide.

Synthétisé de la même manière que le composé **a)** de l'exemple **2** à partir de l'intermédiaire **22** et de o-méthyl-L-tyrosine méthylamide.

RMN (CDCl₃) : δ 7,3-7,15 (m, 5H, HAr et CONH) ; 6,8 (m, 4H, HAr) ; 6 (m, 1H, CONH);
4,7-4,5 (m, 1H, NHCHCO) ; 3,8 (2s et dd, 8H, 2OCH₃ et CH₂S) ; 3,05 (m, 2H, CH₂Ar) ;
2,9 (q, 1H, CHS) 2,7-2,6 (2d, 3H, CONHCH₃) ; 1,75 (d, 2H, CH₂CH) ; 1,65 (m, 1H, CH₂CH) ;
1-0,75 (m, 9H, CHCH₃ et CH(CH₃)₂).

### b) N-2(R)-[(1-thioéthyl)] 2(R) hydroxy 4-méthyl pentanoyl -o-méthyl-L-tyrosine méthylamide

A 0,2 g (0,39 mmole) de composé a) dans 6,3 ml d'ammoniac à -60°C, ajouter 56 mg (2,4 mmoles) de sodium . Agiter 10 minutes puis additionner du chlorure d'ammonium. Laisser l'ammoniac s'évaporer en remontant à température ambiante. Le résidu est repris dans H₂O et extrait par CH₂Cl₂. La phase organique est séchée sur sulfate de sodium et évaporée.

Les diastéréoisomères sont séparés par flash chromatographie (éluant : CH₂Cl₂ : AcOEt ; 80:20). Récupérer 28 mg du diastéréoisomère le moins polaire (28 %).
RMN (CDCl₃) : δ 7,3 (d, 1H, CONH) ; 7,2(d, 2H, HAr) ; 6,8 (d, 2H, HAr) ;
5,95 (m, 1H, CONHCH₃) ; 4,5(m, 1H, NHCHCO) ; 3,8 (s, 3H, OCH₃); 3,2 (m, 1H, CHS) :
3,05 (m, 2H, CH₂Ar) ; 2,7 (d, 3H, NHCH₃) ; 2,65 (s, 1H, OH) ; 1,8-1,6 (m, 3H, CH₂CH);
1,35 (d, 1H, SH) ; 1-0,8 (ddd, 9H, CH₃CH et CH(CH₃)₂).
IR (CHCl₃) : νOH :3619 cm⁻¹ ; νCO : 1675 cm⁻¹.

### Exemple 37 : N-2(R)-[1-thioéthyl] 2(R) hydroxy 4-méthyl pentanoyl-L-(3,4)-dichlorophénylalanine

### a) 2(R)-[1(4(méthoxy)benzylmercapto)éthyl]2(R)-hydroxy 4-méthylpentanoyl-L-(3,4)-dichlorophénylalanine

Synthétisé comme l'exemple 34 a).
RMN (CDCl₃) : δ 7,3-6,9 (m, 8H, HAr et CONH) ; 6,3 (m, 0,7H, CONHCH₃) ;
6,1 (m, 0,3H, CONHCH₃) ; 4,6 (m, 1H, NHCHCO) ; 3,9 (2s, 3H, OCH₃) ; 3,8 (m, 2H, CH₂S) ; 3,2-2,8 (m, 2H, CH₂Ar) ; 2,75 (2d, 3H, NHCH₃) ; 1,7-1,5 (m, 3H, CH₂CH) ;
1,3 (m, 3H, CH₃CH) ; 1-0,7 (m, 6H, CH(CH₃)₂).

### b) N-2 (R)-[1-thioéthyl] 2(R) hydroxy 4-méthyl pentanoyl -L-(3,4)-dichlorophénylalanine

Synthétisés de la même manière que le composé 35 b).
RMN (CDCl₃) : δ 7,4 (d, 1H, CONH) ; 7,3 (m, 3H, HAr) ; 6,1 (m, 1H, NHCH₃) ;
4,6 (m, 1H, NHCHCO) ; 3,2-3 (m, 3H, CHS et CH₂Ar) ; 2,7 (d, 3H, NHCH₃) ;
1,8-1,6 (m, 3H, CH₂CH) ; 1,3 (d, 1H, SH) ; 1-0,8 (m, 9H, CH₃CH, CH(CH₃)₂).

### Exemple 38 : N-2(S)-[1(N-hydroxy-N-formyl) éthyl] 2(S)-hydroxy 4-méthylpentanoyl-o-méthyl L-tyrosine méthylamide

### a) N-2(S)-[1(o-benzylhydroxylamino)éthyl]2(S)-hydroxy-4-méthylpentanoyl-o-méthyl-L-tyrosine méthylamide

Synthétisé de la même manière que le composé a) de l'exemple 2 sauf pour la purification réalisée par flash chromatographie (éluant : heptane : AcOEt ; 20:80 puis 30:70).
RMN (DMSO) : δ 7,9 (m, 1H, CONH) ; 7,6 (d, 1H, CONH) ; 7,25 (m, 5H, HAr) ;
7,05 (dd, 2H, HAr) ; 6,75 (dd, 2H, HAr) ; 6,1 (d, 0,5H, NH) ; 5,95 (d, 0,5H, NH) ;
4,9 (s, 0,5H, OH) ; 4,85 (s, 0,5H, OH) ; 4,55 (s, 1H, OCH₂Ar) ; 4,5 (s, 1H, OCH₂Ar) ;
4,45 (m, 1H, NHCHCO) ; 3,7 (s, 3H, OCH₃) ; 3,1 (m, 0,5H, CHNH) ; 3 (m, 0,5H, CHNH) ;
2,7 (m, 2H, CHCH₂Ar) ; 2,55 (dd, 2H, NHCH₃) ; 1,7-1,3 (m, 3H, CH₂CH) ;
0,95-0,6 (m, 9H, CHCH₃ et CH(CH₃)₂).
IR (CHCl₃) : vOH :3454 cm⁻¹ ; νNH : 3401 cm⁻¹ ; νCO : 1675 cm⁻¹.

### b) N-2(S)-[1-(N-formyl (o-benzylhydroxylamino))éthyl] 2 (S)-hydroxy-4-méthylpentanoyl-o-méthyl-L-tyrosine méthylamide

A une solution d'acide formique (85 µl ; 2,24 mmoles) et d'anhydride acétique (0,22 ml ; 2,24 mmoles) chauffée à 40°C pendant 45 minutes, additionner 0,264 g (0,56 mmoles) de composé **a)** dissous dans 11 ml de CH₂Cl₂.

Agiter à température ambiante pendant 1h30. Laver par une solution de NaHCO₃ à 80 % puis par H₂O. Sécher sur sulfate de sodium et évaporer sous vide.

Récupérer 0,267 g (95 %).

Le produit est un mélange de diastéréoisomères ≈ 50-50. Chaque diastéréoisomère existe sous la forme de deux conformères en RMN.
RMN (DMSO) : δ 8-7,6 (m, 3H, CHO et CONH et COCHCH₃) ; 7,4 (m, 5H, HAr) ;
7 (m, 2H, HAr) ; 6,8-6,6 (m, 2H, HAr) ; 5,6-5,2 (m, 1H, OH) ;
5-4,5 (m, 3H, OCH₂Ar et NHCHCO) ; 3,7-3,6 (2s, 3H, OCH₃) ; 2,8 (m, 2H, CH₂Ar) ;
2,6 (m, 3H, NHCH₃) ; 1,6 et 1,4 (m, 3H, CH₂CH) ; 1,2 et 1 (d, 3H, CHCH₃) ;
0,8 et 0,65 (m, 6H, CH(CH₃)₂).
IR (CHCl₃) : νOH :3454 cm⁻¹ ; νNH : 3393 cm⁻¹ ; νCO : 1661 cm⁻¹.

### c) N-2(S)-[1(N-hydroxy-N-formyl) éthyl] 2(S)-hydroxy 4-méthylpentanoyl-o-méthyl L-tyrosine méthylamide

A une suspension de Pd/C 10 % (0,266 g) dans 5 ml de MeOH, additionner 0,266 g de composé b) (0,53 mmoles) en solution dans 25 ml de MeOH.

Agiter sous hydrogène pendant 1 heure. Filtrer sur célite et évaporer sous vide.

Purifier par flash chromatographie (éluant CH₂Cl₂ : MeOH ; 97:3).

Récupérer 0,118 g (54 %).

Le produit est un mélange de diastéréoisomères ≈ 50-50. Chaque diastéréoisomère existe sous la forme de deux conformères en RMN.
RMN (DMSO + D₂O) : δ 8,2 et 8,1 et 7,8 et 7,7 (4s, 1H, CHO) ; 7 (m, 2H, HAr) ;
6,7 (m, 2H, HAr) ; 4,5 (m, 1H, NHCHCO) ; 4,2 et 3,7 (m, 1H, CHCH₃) ; 3,8 (s, 3H, OCH₃) ;
2,8 (m, 2H, CH₂Ar) ; 2,6 (2d, 3H, NHCH₃) ; 1,6-1,25 (m, 3H, CH₂CH) ;
1,2-0,6 (m, 9H, CHCH₃ et CH(CH₃)₂)
IR (CHCl₃) : νOH :3450 cm⁻¹ ; νNH : 3397 cm⁻¹ ; νCO : 1664 cm⁻¹.

### Biochimie

### Activité sur collagénase

L'activité anticollagénasique des composés selon la présente invention a été déterminée sur la collagénase de la lignée monocytaire humaine U937 selon le mode opératoire décrit par Cawston et

Barrett (Anal. Biochem. 99, 340-345, 1979).

La collagénase a été incubée à 27°C durant 16 heures avec du collagène radiomarqué et son activité a été déterminée par la libération des peptides solubles produits par la dégradation enzymatique du collagène. La concentration inhibitrice CI50 pour chaque composé a été déterminée par mesure de l'activité de l'enzyme en présence de l'inhibiteur à une concentration comprise entre 10⁻⁷ et 10⁻¹² M (l'inhibiteur est dissout à une concentration de 10⁻² M dans le DMSO puis dilué successivement au dixième dans le tampon : Tris, HCI 150 mM, pH 7,5 ; NaCl 0,15 M ; CaCl₂ 10 mM).

### Activité sur stromélysine

L'activité antistromélysine des composés selon la présente invention a été déterminée sur la stromélysine d'origine humaine, selon le mode opératoire décrit par Cawston T.E., Galloway, W.A., Mercier, E., Murphy, G., Reynolds J.J. (Biochem. J., 195, 159-165, 1981).

La stromélysine a été incubée à 37°C durant 16 heures avec de la transferrine radiomarquée et son activité a été déterminée par la libération des peptides solubles produits par la dégradation enzymatique de la transferrine. La concentration inhibitrice Cl50 pour chaque composé a été déterminée par mesure de l'activité de l'enzyme en présence de l'inhibiteur à une concentration comprise entre 10⁻⁷ et 10⁻¹² M (l'inhibiteur est dissout de la même manière que pour la collagénase).

### Activité sur gélatinases

L'activité antigélatinasique des composés selon la présente invention a été déterminée sur la gélatinase de 72 kDa d'origine humaine, selon le mode opératoire décrit par Knight, C.G., Willenbrock, F.. Murphy,G (FEBS Letters, 296 (3), 263-266, 1992).

La gélatinase a été incubée à 37°C durant 10 minutes et son activité a été déterminée par l'augmentation de fluorescence du produit de dégradation Mca-Pro-Leu du substrat synthétique Mca-Pro-leu-Gly-Leu-Dpa-Ala-Arg-NH₂.

La concentration inhibitrice CI50 pour chaque composé a été déterminée par mesure de l'activité de l'enzyme en présence de l'inhibiteur à une concentration comprise entre 10⁻⁷ et 10⁻¹² M (l'inhibiteur est dissout à une concentration de 10⁻² M dans le DMSO puis dilué puis dilué successivement au dixième dans le DMSO jusqu'à 10⁻⁴ M puis dans le tampon : Tris, HCl 50 mM + 0,05 % Brij 35 ; CaCl₂ 10 mM ; NaCl 0,15 M).

L'activité antigélatinasique des composés selon la présente invention a aussi été déterminée sur la gélatinase de 92 kDa ou 72 kDa d'origine humaine, selon le mode opératoire décrit par Harris, E.D., Krane, S.M; (Biochem. Biophys. Acta, 258, 566-576, 1972).

La gélatinase a été incubée à 37°C durant 16 heures avec de la gélatine radiomarquée et son activité a été déterminée par la libération des peptides solubles produits par la dégradation enzymatique de

la gélatine. La concentration inhibitrice CI50 pour chaque composé a été déterminée par mesure de l'activité de l'enzyme en présence de l'inhibiteur à une concentration comprise entre 10⁻⁷ et 10⁻¹² M (l'inhibiteur est dissout de la même manière que pour la collagénase).

### Inhibition de la production de TNF in vitro

La capacité des composés de la présente invention à inhiber la libération de TNF a été déterminée à partir de macrophages péritonéaux de souris stimulés par du LPS (Lang, F., Robert, J.M., Boucrot, P. , Welin, K; Petit, J.Y., J. Pharmacol. Exp. Ther., 275, 171-176, 1995).

Après une phase d'adhérence de 2 heures, les cellules ont été traitées durant 1 heure par le composé à tester à des concentrations comprises entre 10⁻⁶ à 10⁻⁹ M (l'inhibiteur est dissout dans le DMSO à 10⁻² M puis à 5.10⁻³ M dans du RPMI 1640 avec 5 % SVF puis dilué successivement au dixième dans le même milieu), puis stimulées par l'ajout de LPS (100 ng/ml en concentration finale).

Trois heures plus tard, l'activité biologique du TNF dans les surnageants a été déterminée par un essai de cytotoxicité utilisant la lignée murine L929 selon la procédure opératoire décrite par Bandara, G., Lin, C.W., Georgescu, H.I., Evans, C.H. (Bioch. Biophys. Acta, 1134, 309-318, 1982)

La concentration en pg/ml du TNF des échantillons a été déterminée à partir d'une courbe standard de TNF α murin permettant ainsi de définir la concentration inhibitrice CI50 des composés étudiés.

Les résultats sur collagénase, gélatinase, stromélysine et TNF sont regroupés dans le tableau I.

### Inhibition de la dégradation du cartilage in vivo

Les activités des composés de l'exemple 1 et de l'exemple 10 comparativement au composé BB16 ont été déterminées dans un modèle de dégradation du cartilage selon le mode opératoire décrit par Bottomley, K.M.K, Griffith, S.R.J., Rising, T.G., Steward, A. (Brit. J. Pharmacol. 21, 287-289, 1988). Du cartilage de tête fumorale de rat enrobé de coton stérile est implanté en sous-cutané sur le dos de souris. Après 21 jours, les souris sont euthanasiées, les cartilages récupérés et pesés. Les animaux sont traités chaque jour, 2 fois par jour par une dose de 10 mg/kg i.p.,

Le composé de l'exemple 10 inhibe la perte de poids du cartilage de 65 % tandis que le composé de l'exemple 1 inhibe cette dernière de la même façon que le comparateur 1 : BB16 à savoir 42 % (P < 0,05).

### Inhibition du choc septique

Le choc septique est induit chez des souris femelles Balb/c selon la procédure décrite par Mohler, K.M. et al. (Nature, 370, 218-220, 1994). Brièvement, le choc est induit par l'injection i.p. de 20 mg de D-galactosamine et i.v. de 20 ng de LPS. La survie des animaux est appréciée durant les 48 heures suivantes.

Les produits à tester sont injectés i.p. 30 minutes avant l'induction du choc. Les composés, selon l'invention, protègent du choc de 10 à 50 mg/kg. A titre d'exemple, le composé de l'exemple 10 protège à 50 mg/kg.

### Inhibition de la production de TNFα in vivo

L'injection de LPS à des souris (100 µg/souris i.p.) induit la production de TNFα dont la concentration plasmatique est maximale à 60-90 minutes (Sekut, L., Menius, J.A., Brackeen M.F., Connolly K.M., J. Lab. Clin. Med., 124, 6, 613-820, 1994).

Le TNF α est dosé par sa cytotoxicité sur des cellules L929 (Flick, D.A., Gilford, G.E., J. Immunol. Methods, 68, 167-175, 1984).

Les produits à tester sont administrés i.p. ou p.o. avant l'injection de LPS.

Les composés, selon l'invention, inhibent la production de TNFα entre 1 et 50 mg/kg.

### Inhibition de la libération de TGFα

Les composés de la présente invention ont été testés selon les méthodes des exemples 2 ou 3 décrites dans le brevet WO 96/25156 et inhibent la libération du TGFα.

### Détermination de la biodisponibilité

Les composés de la présente invention ont été administrés i.p. ou p.o. entre 1 et 50 mg/kg à des souris ; les taux sanguins ont ensuite été déterminés par un bioessai ex-vivo selon Wang X. et al. (Cancer Res., 54, 4726-4728, 1994). Les résultats sont présentés dans le tableau 2. L'introduction d'un groupement OH augmente systématiquement la biodisponibilité des produits.

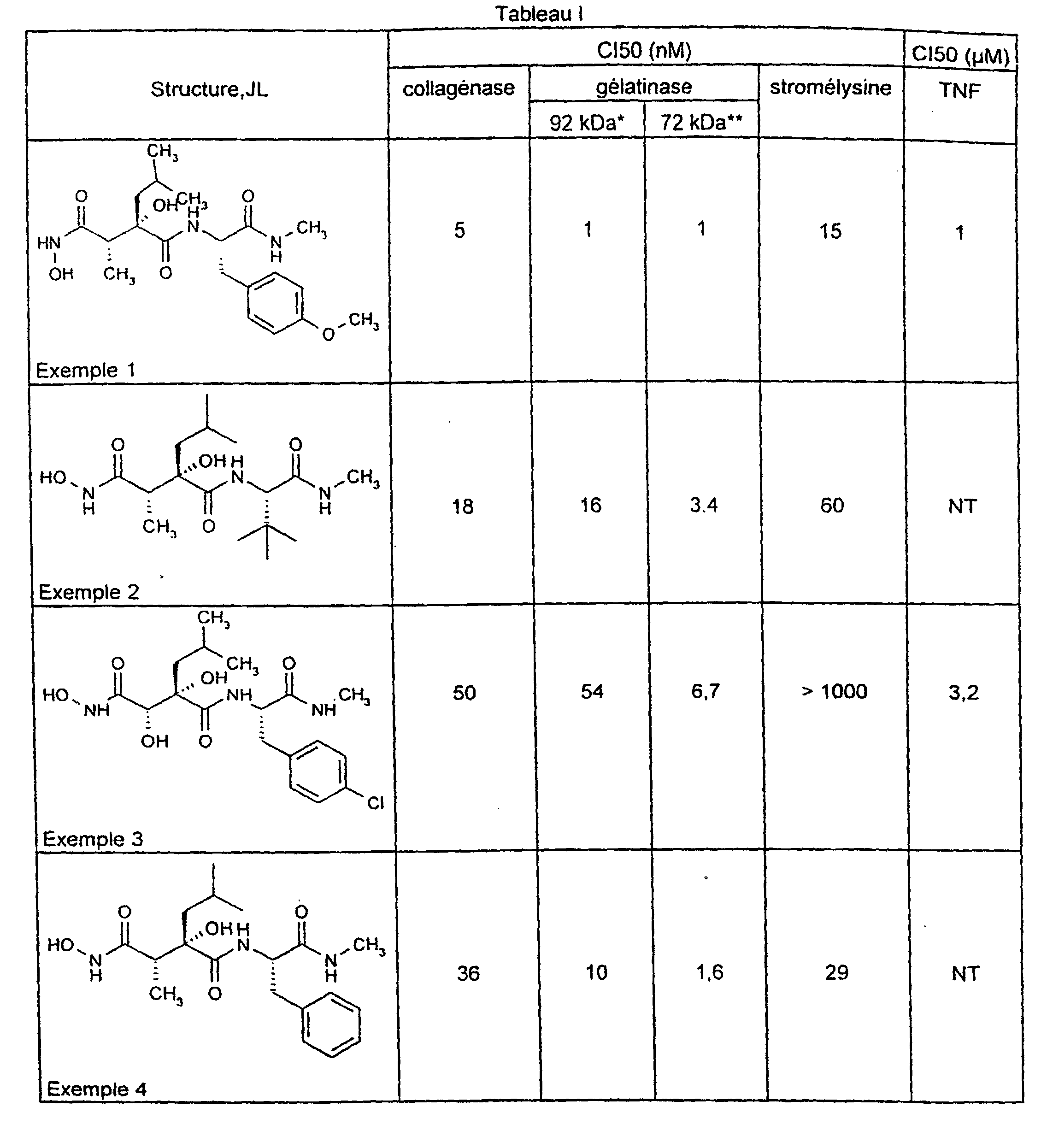

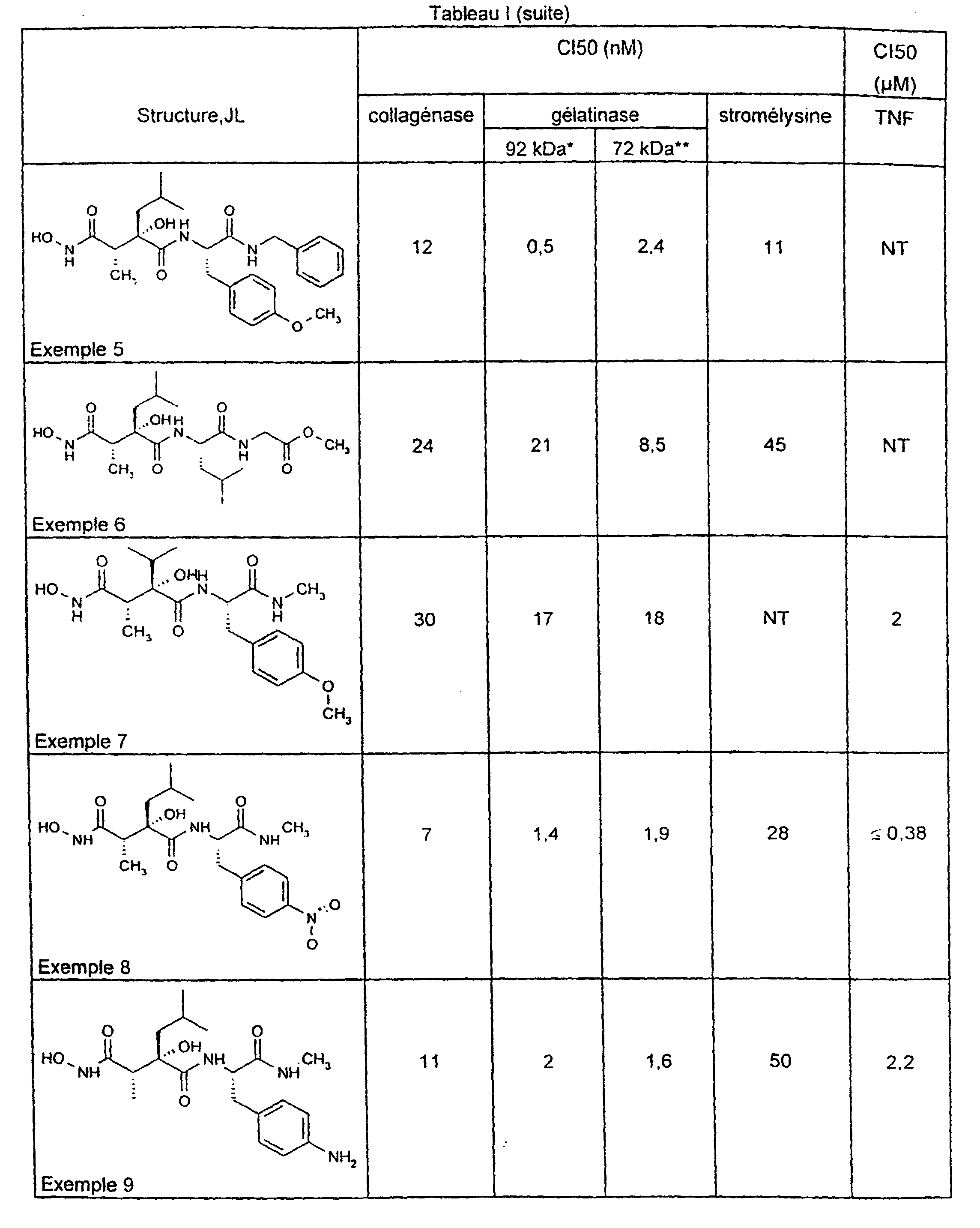

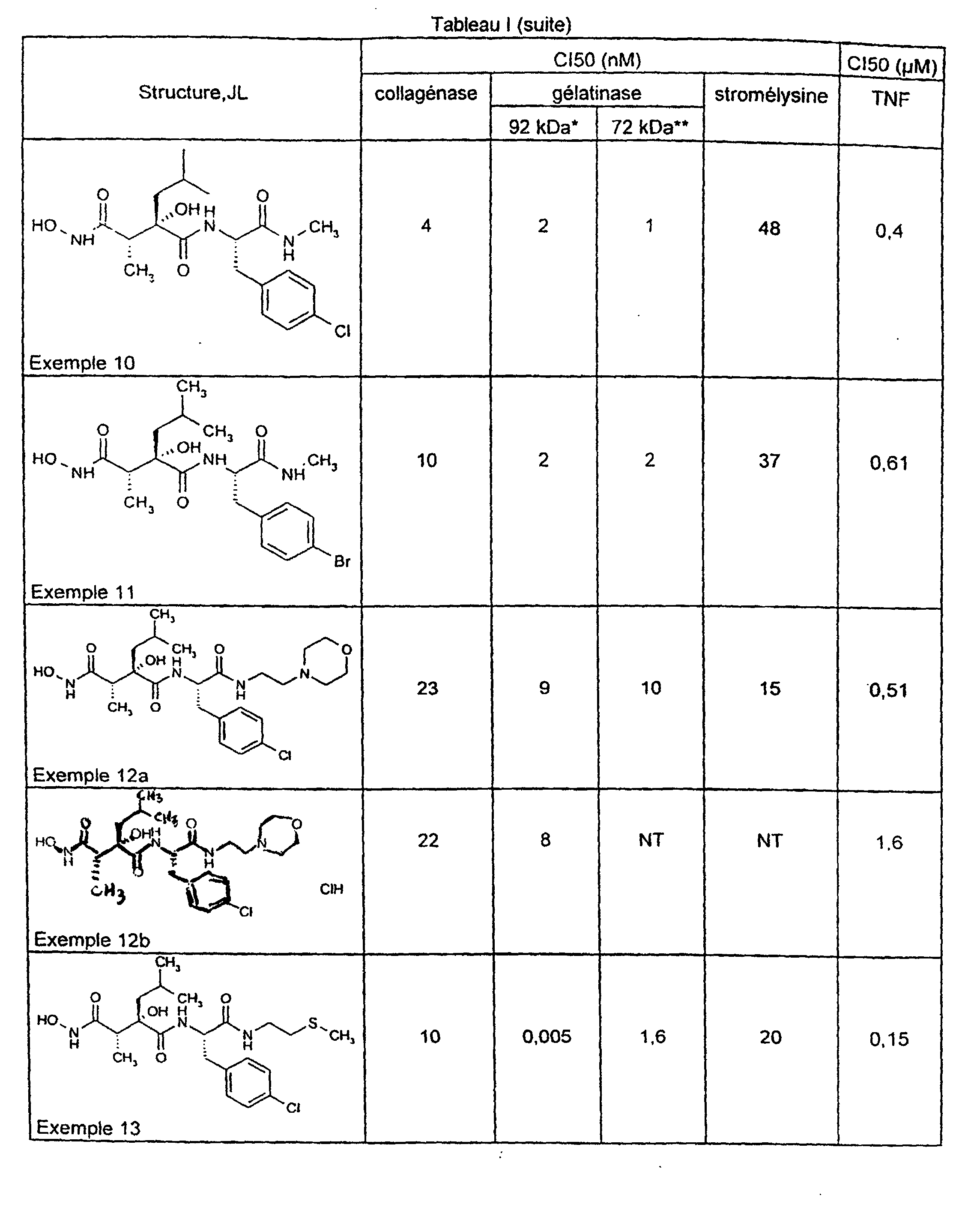

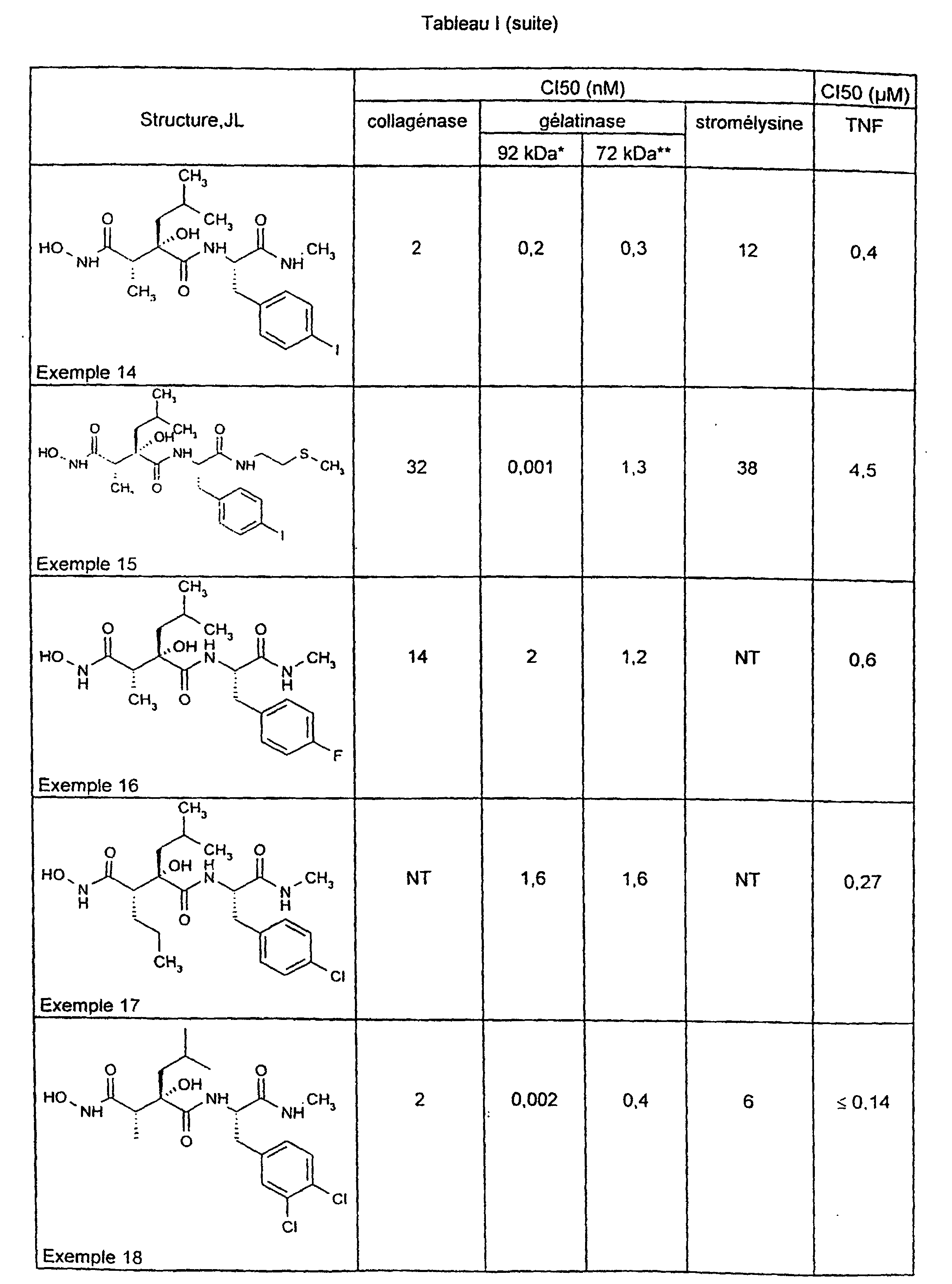

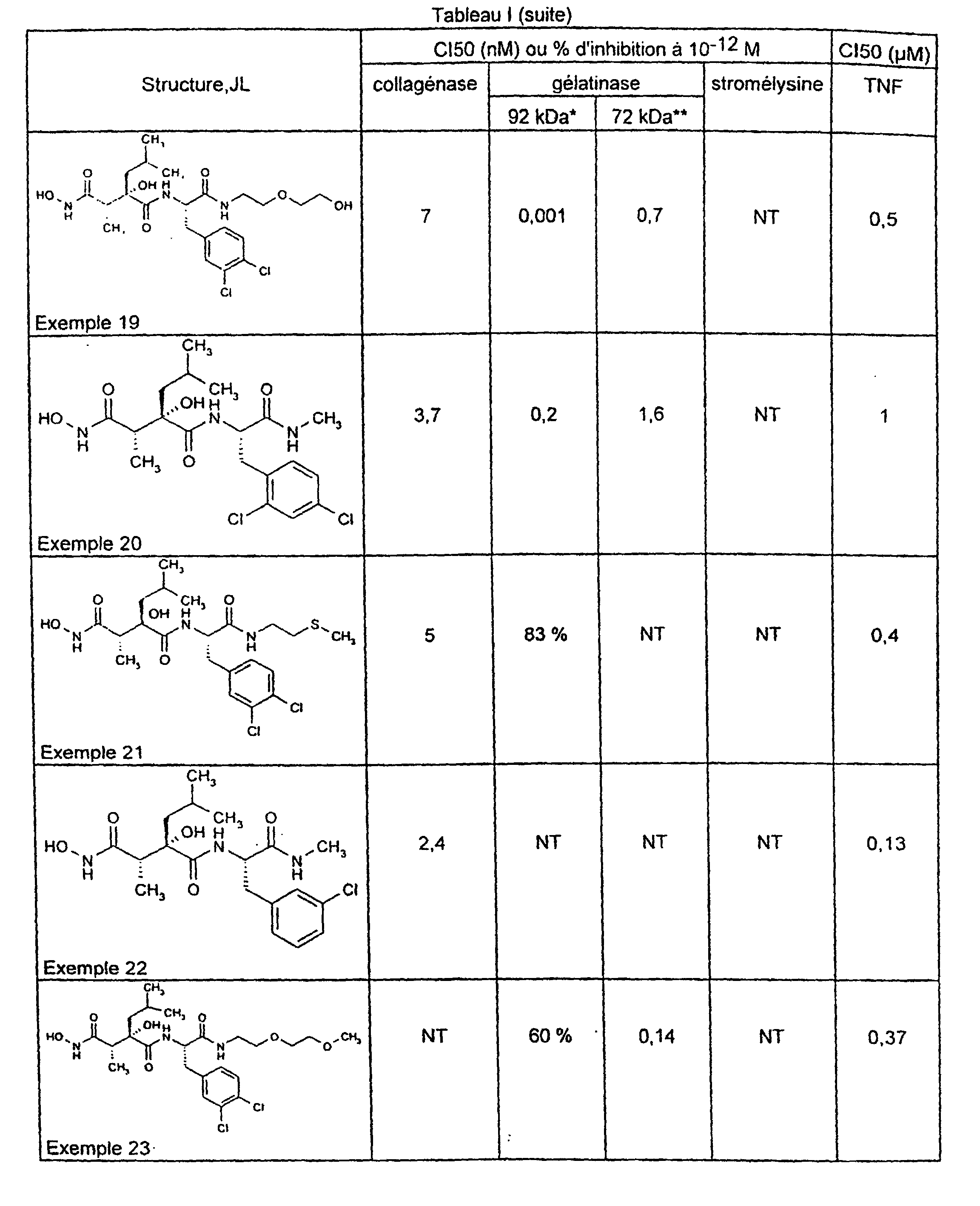

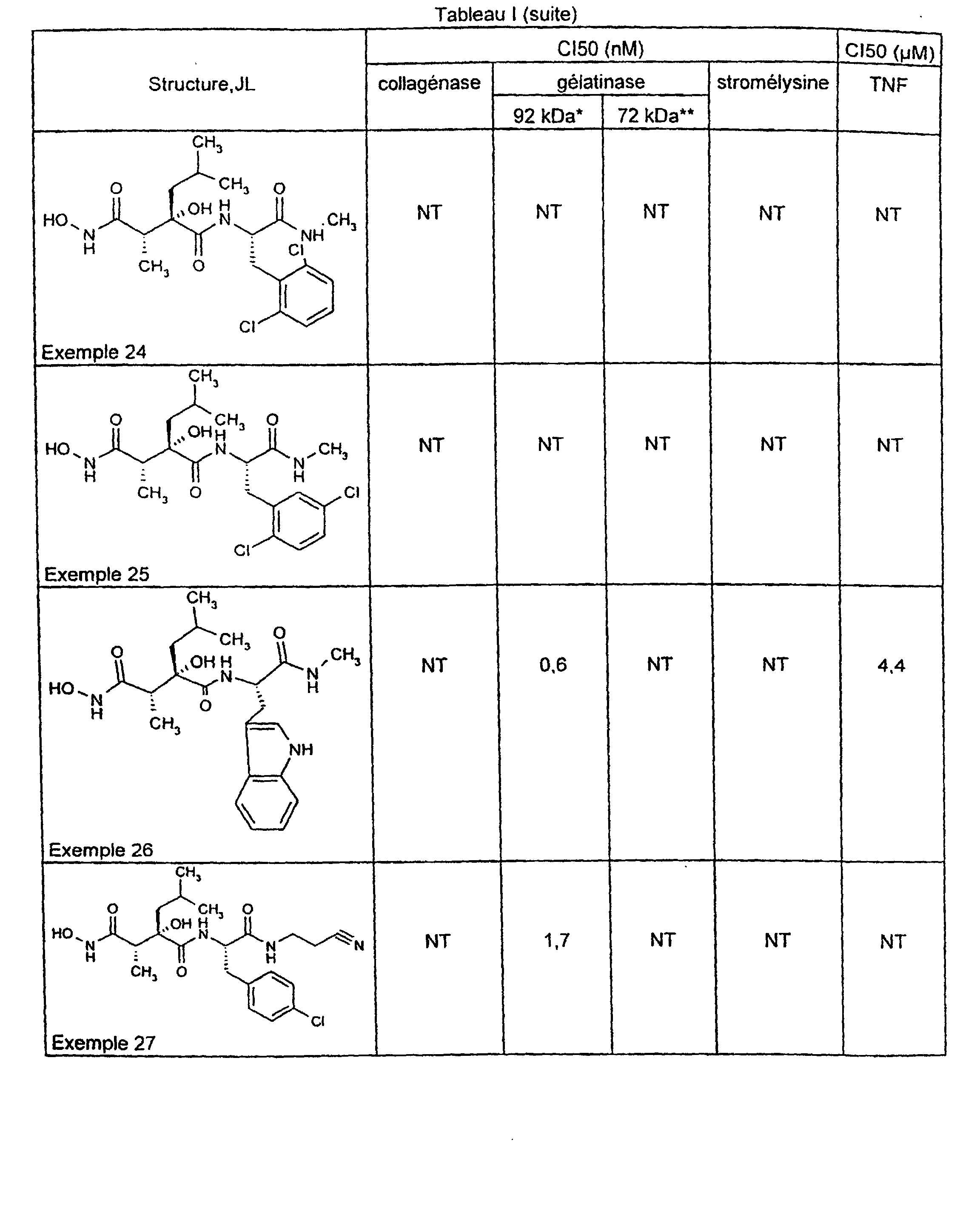

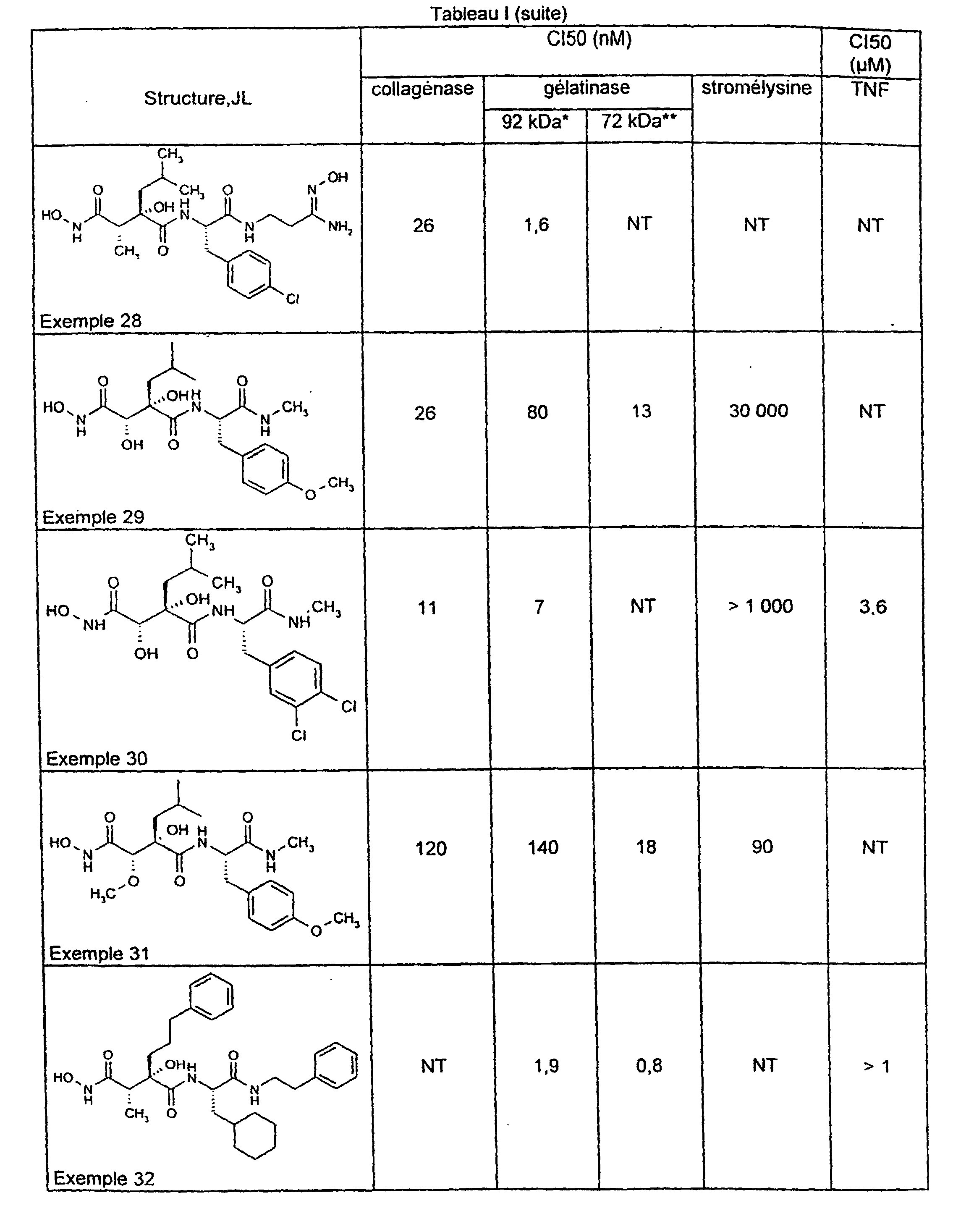

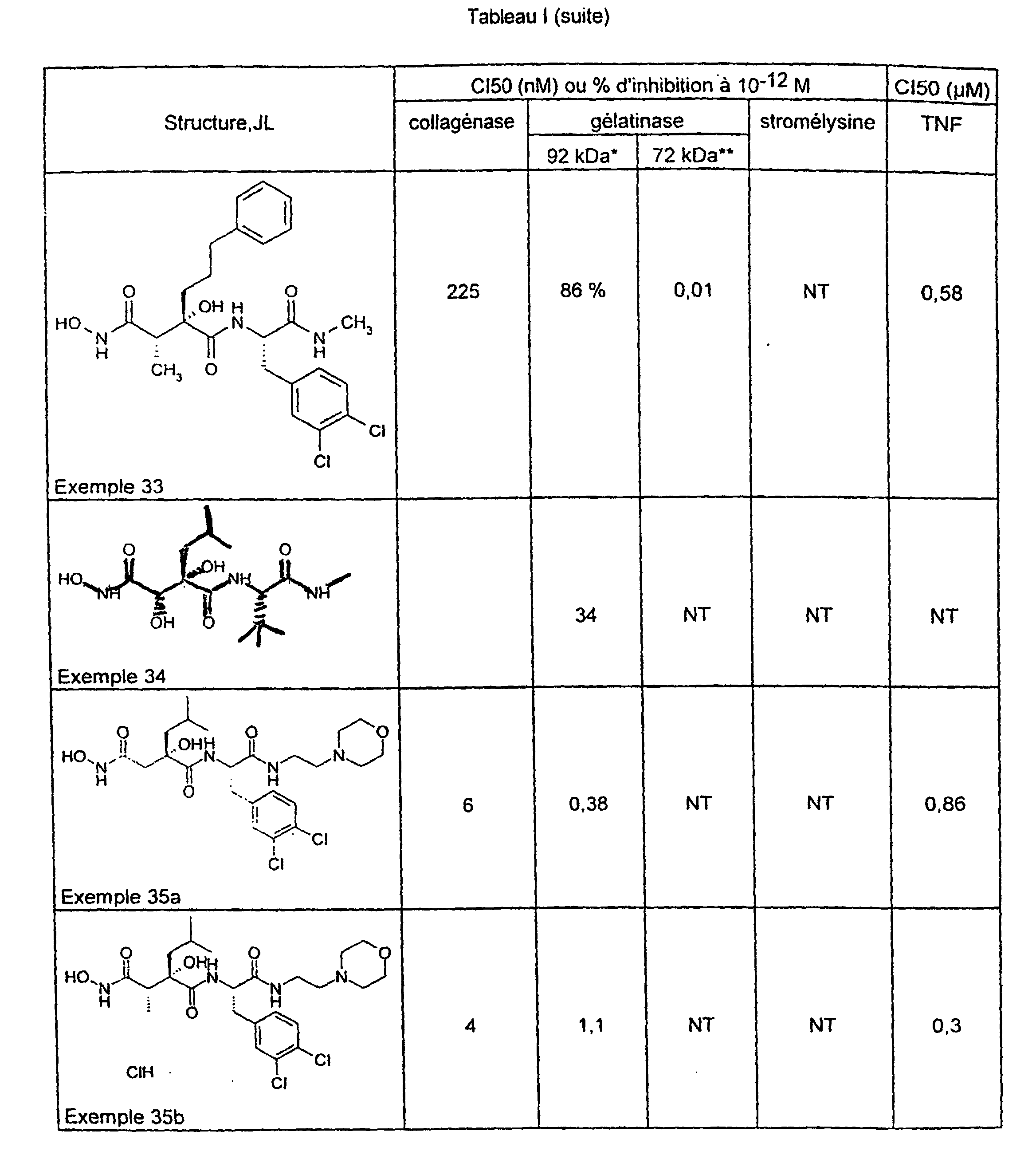

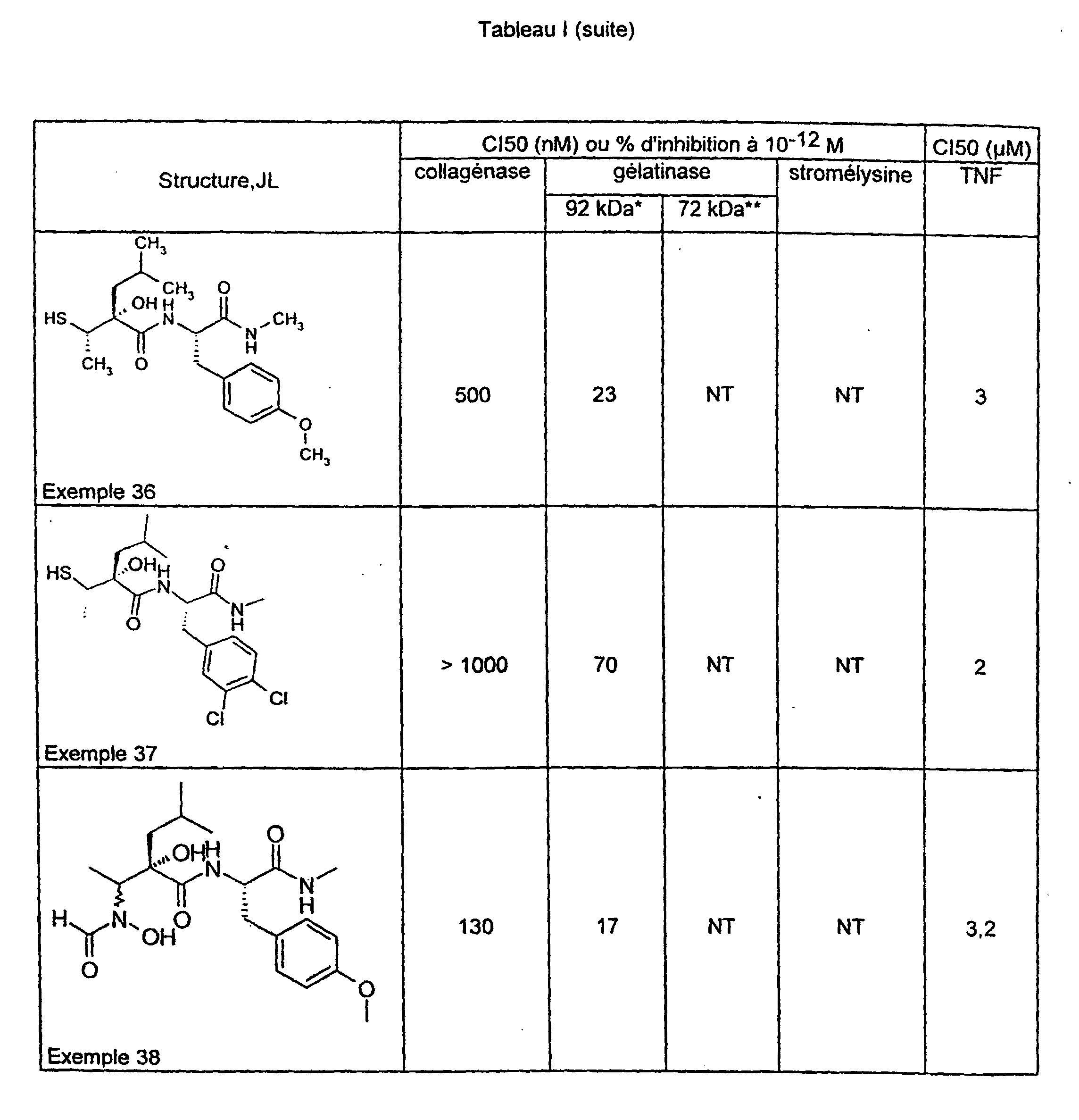

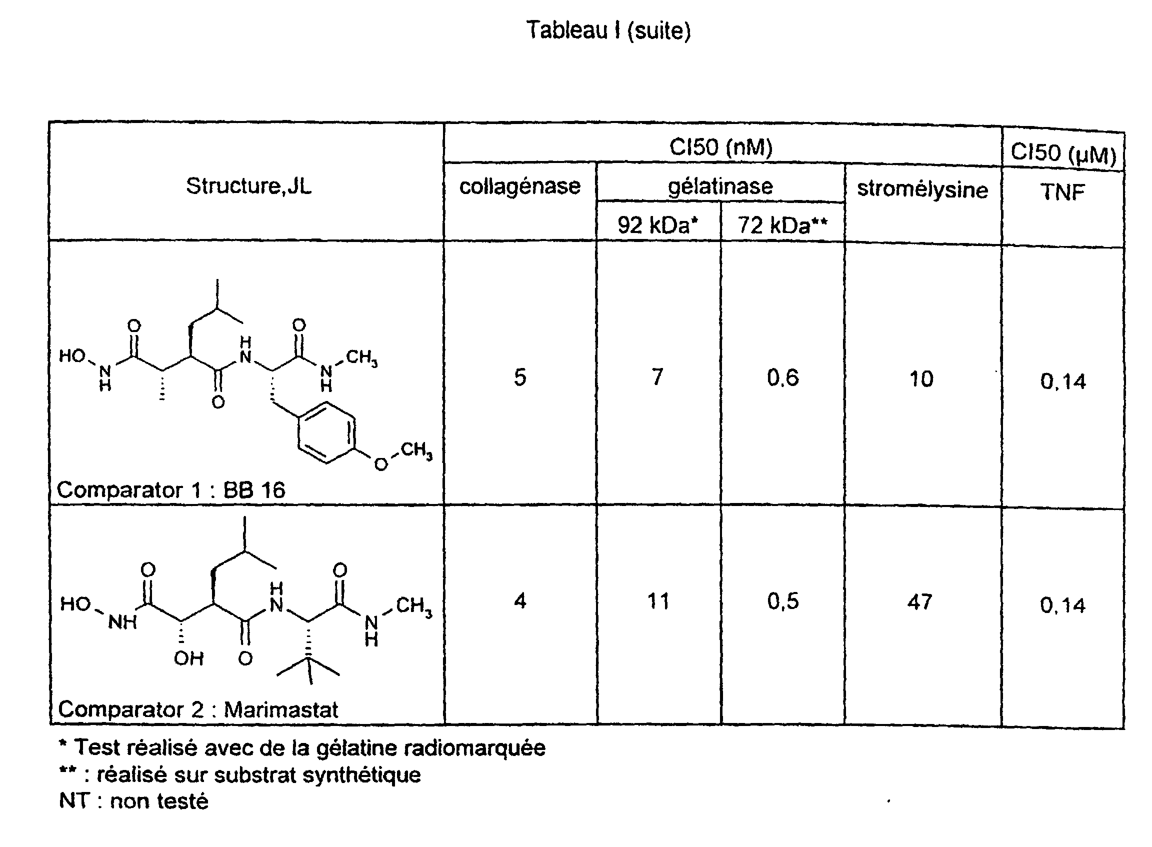

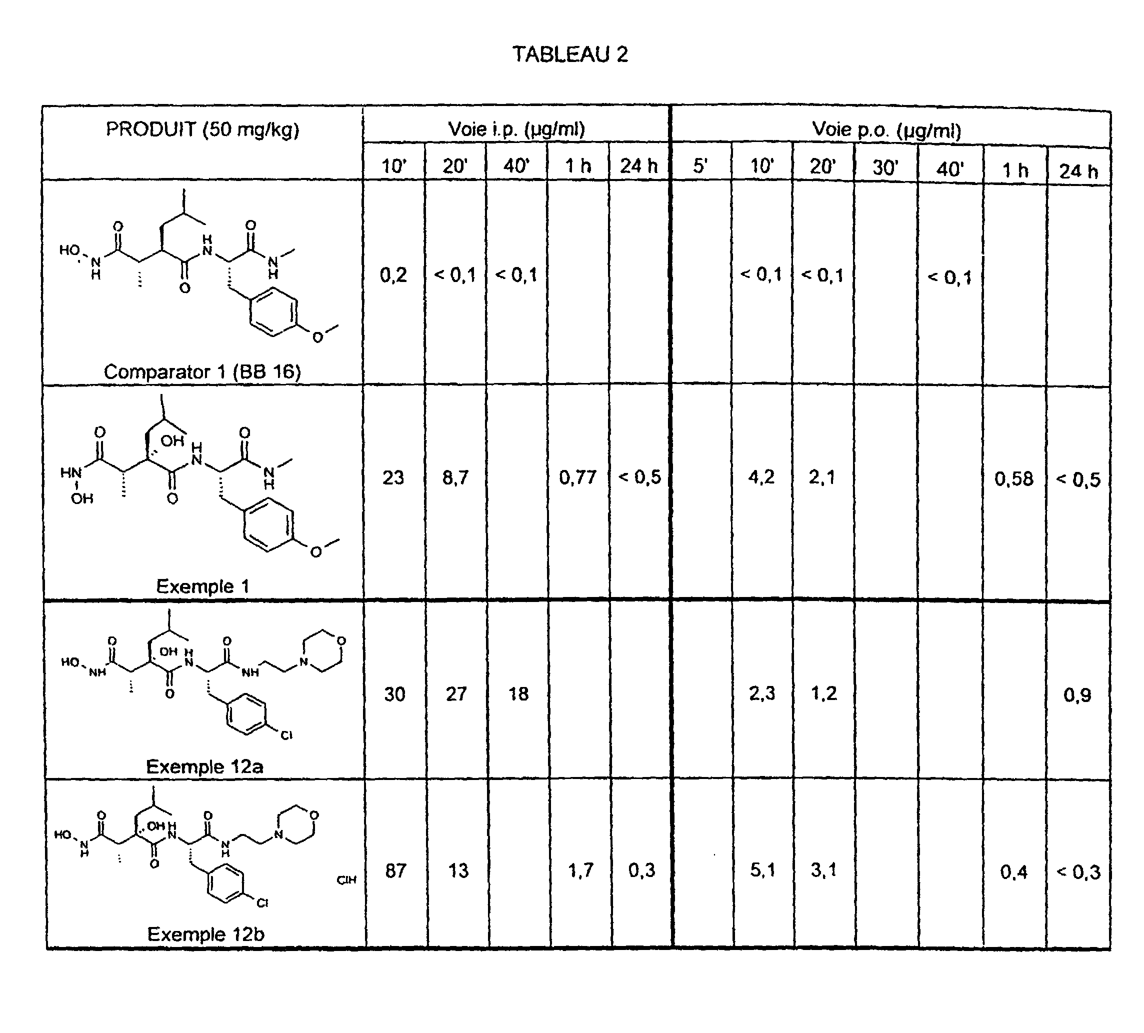

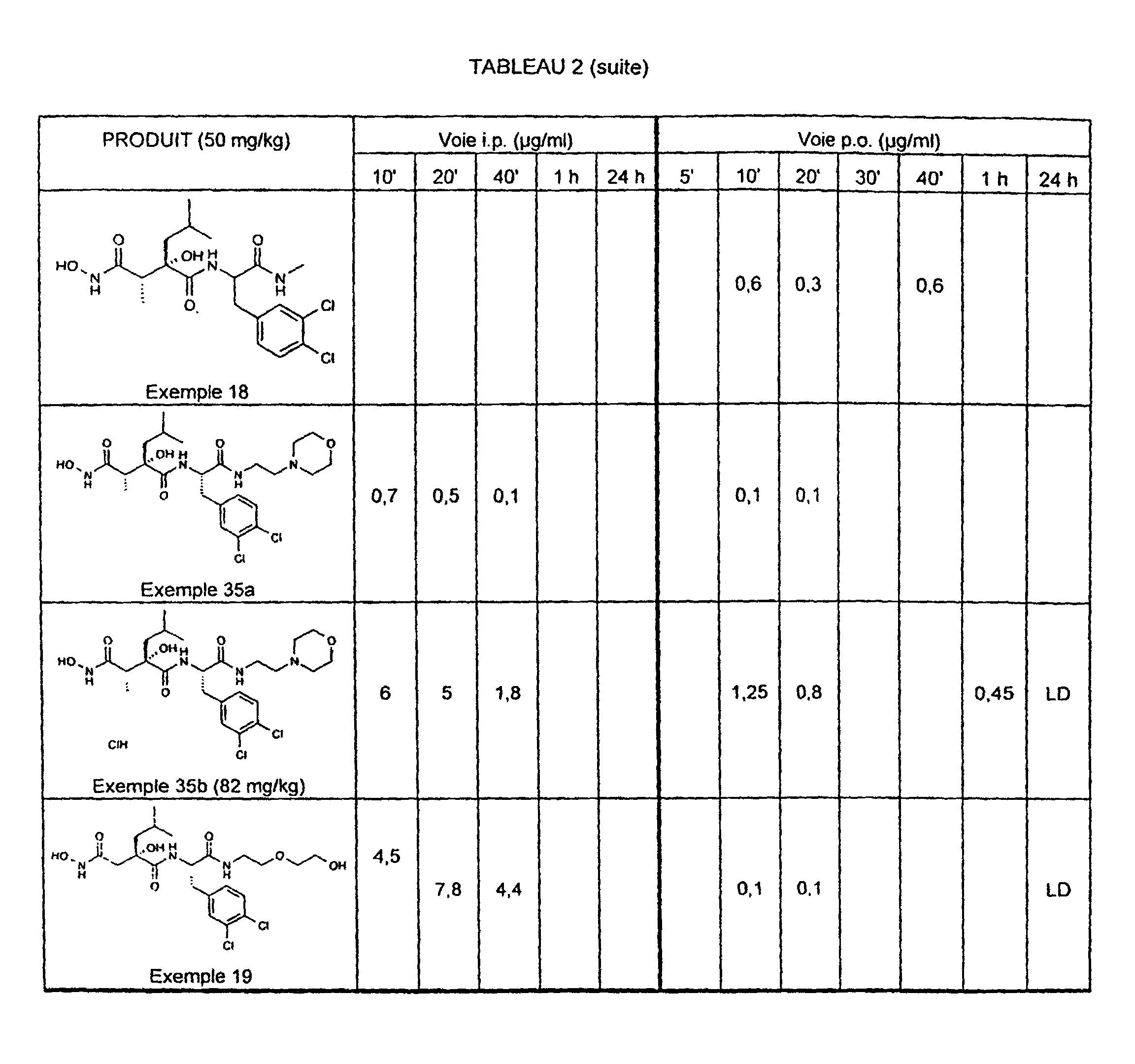

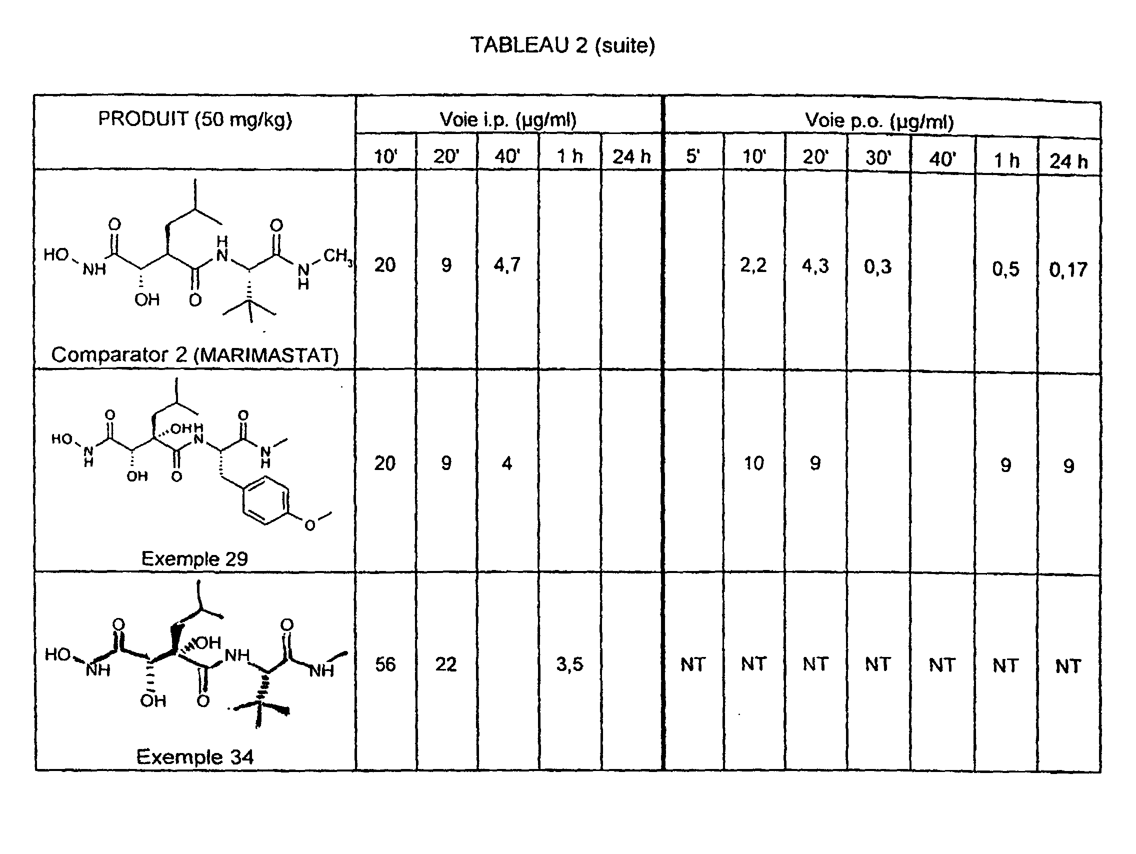

## Revendications

1. Composés de formule générale (X) suivante : 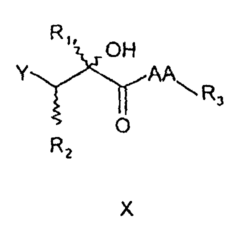 dans laquelle :
- Y représente :
• - CONHOH, ou
• - SH, ou
• un groupe de formule 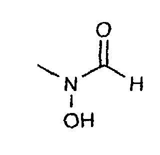 ou
• un groupe de formule 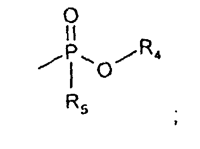 dans lequel :
• • R₄ représente -H, ou un groupe alkyle en C₁ à C₆, ou un groupe phénylalkyle dans lequel le groupe alkyle est en C₁ à C₆,
• • R₅ représente un groupe de formule 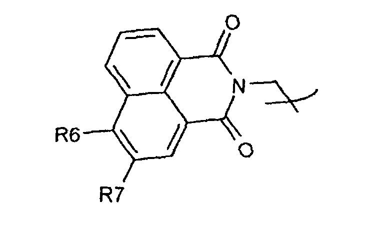
dans lequel :
• •• R₆ représente -H, ou un groupe alkoxy en C₁ à C₆, où un groupe benzyloxy,
• •• R₇ représente -H, ou un atome d'halogène tel que -Cl ou -Br,
- R₁ représente :
• une chaîne alkyle linéaire ou ramifiée en C₃ à C₁₆, ou cyclisée en C₃ à C₆, ladite chaîne comportant le cas échéant un hétéroatome tel que O, S ou N,
• un groupe phénoxyalkyle, ou phénylalkyle, substitués ou non, ou un groupe hétéroarylalkyle, le groupe alkyle étant en C₂ à C₅,
- R₂ représente :
• un atome d'hydrogène, ou,
• un groupe alkyle en C₁ à C_{5,} ou alkylidène en C₂ à C₅, ou
• un hydroxyle, un alkoxy en C₁ à C₆ ou un benzyloxy, à condition que Y représente -CONHOH lorsque R₂ représente un hydroxyle, ou
• un groupe hydroxyméthyle, ou alkoxyméthyle en C₁ à C₆, ou
• un groupe arylalkyle dans lequel la partie alkyle est en C₁ à C₆, un groupe aryloxyméthyle, un groupe arylthiométhyle, un groupe hétéroarylthiométhyle, dans lesquels aryle désigne un reste phényle éventuellement substitué, notamment par -OH, -OCH₃, un groupe alkyle en C₁ à C₃ linéaire ou ramifié, un halogène tel que -Cl ou -Br, un groupe aminé tel que -NH₂, -NHCOCH₃, -NHCOOR₁₀, R₁₀ représentant un groupe alkyle en C₁ à C₃ linéaire ou ramifié, ou
• un groupe phtalimido alkyle dans lequel la partie alkyle est en C₁ à C₆, ou
• un groupe alkoxycarbonylméthyle (alkoxy désignant méthoxy, éthoxy), un benzyloxycarbonylméthyle, un acétylméthyle, à condition dans ces trois cas que Y représente -SH,
- AA représente un acide aminé, ou un enchaînement d'acides aminés, ces acides aminés étant naturels ou non, et avantageusement de configuration absolue S, notamment un acide aminé de formule 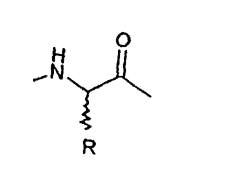 dans laquelle R représente :
• une chaîne alkyle linéaire ou ramifiée en C₁ à C₄,
• un groupe -CH₂-Y dans lequel Y représente un cycle de 4 à 6 atomes de carbone dans le cycle, comportant le cas échéant un ou plusieurs hétéroatomes tels que O, S ou N, ledit cycle étant aromatique ou non, le cas échéant substitué notamment par un ou plusieurs groupes -OCH₃, -NO₂, -NH₂, ou par un ou plusieurs atomes d'halogène notamment choisis parmi -Cl, -Br, -F et -I,
• un groupe de formule 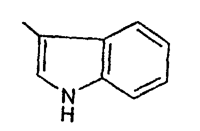
- R₃ représente un groupe de formule -NH-(R₈)ₙ-R₉ dans laquelle :
• n représente 0 ou 1,
• R₈ représente une chaîne alkyle linéaire ou ramifiée, de 1 à 8 atomes de carbone, comportant le cas échéant un ou plusieurs hétéoratomes tels que O ou S,
• R₉ représente un atome d'hydrogène ou un groupe méthyle, nitrile, morpholino, phényle, méthoxy, hydroxyle, thiométhyle, ou un groupe de formule -CH(NH₂) = N-OH, ou un groupe -N(CH₃)₂.

2. Composés selon la revendication 1, **caractérisés par** la formule générale (Xa) suivante : 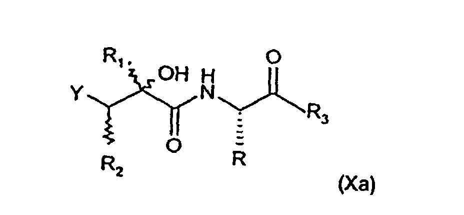 dans laquelle :
- Y représente :
• - CONHOH,
- R₁ représente :
• - CH(CH₃)₂,
• - CH₂- CH(CH₃)₂,
• 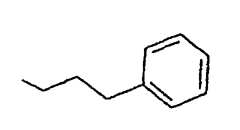
- R₂ représente :
• un groupe alkyle de 1 à 5 atomes de carbone, notamment un groupe méthyle, ou propyle,
• un hydroxyle, ou
• un groupe alkoxy de 1 à 5 atomes de carbone, notamment un groupe méthoxy,
- R représente :
• - C(CH₃)₃,
• - CH₂ - CH(CH₃)₂,
• un groupe 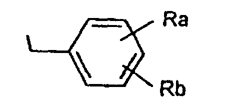 aromatique ou non, dans lequel Rₐ et R_{b}, indépendamment l'un de l'autre, représentent -H, -Cl, -Br, -I, -F, -OCH3, -NO₂, -NH₂,
• un groupe de formule 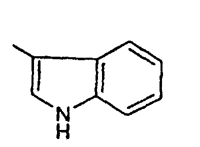
- R₃ représente un groupe -NH - (CH₂)ₙ₁ - R₉ dans lequel :
• n₁ représente 0, 1 ou 2,
• R₉ représente -CH₃, -C≡N, -COOCH₃, -SCH₃, -O-(CH₂)₂-OH, -O-(CH₂)₂-OCH₃, -CH(NH₂)=N-OH,

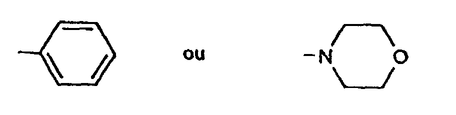

3. Composés selon la revendication 1 ou la revendication 2, **caractérisés en ce qu'**ils possèdent une stéréochimie telle que les substituants R₁ et R₂ sont positionnés en anti par rapport au reste succinique selon la formule (XI.1) suivante:

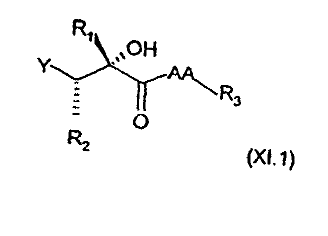

4. Composés selon l'une des revendications 1 à 3, **caractérisés en ce que** R représente un groupe de formule 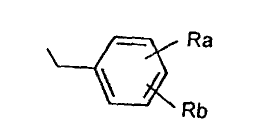 dans laquelle Rₐ et R_{b} représentent un atome d'halogène, notamment un atome de chlore.

5. Composés selon l'une des renvendications 1 à 4, **caractérisés en ce que** R₃ représente un groupe de formule
-NH -(CH₂)₂ -SCH₃

6. Mélange **caractérisé en ce qu'**il comprend des composés de formule (XI. 1) suivante : 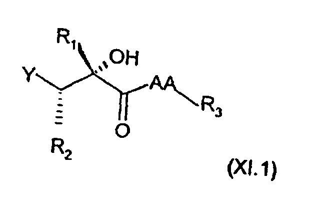 dans laquelle Y, R₁ et R₂, AA et R₃ sont tels que définis dans l'une des revendications 1 à 5.
et des composés de formule (XI.2) suivante : 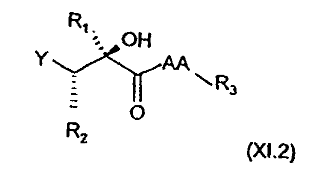 dans laquelle Y, R₁, R₂, AA et R₃ ont la signification indiquée ci-dessus,
la proportion des composés (XI.1) et (XI.2) dans le mélange étant avantageusement d'environ 50 % à environ 99 % pour le composé de formule (XI.1) et d'environ 50 % à environ 1 % pour le composé de formule (XI.2).

7. Composés selon l'une des revendications 1 à 5, **caractérisés par** les formules suivantes :

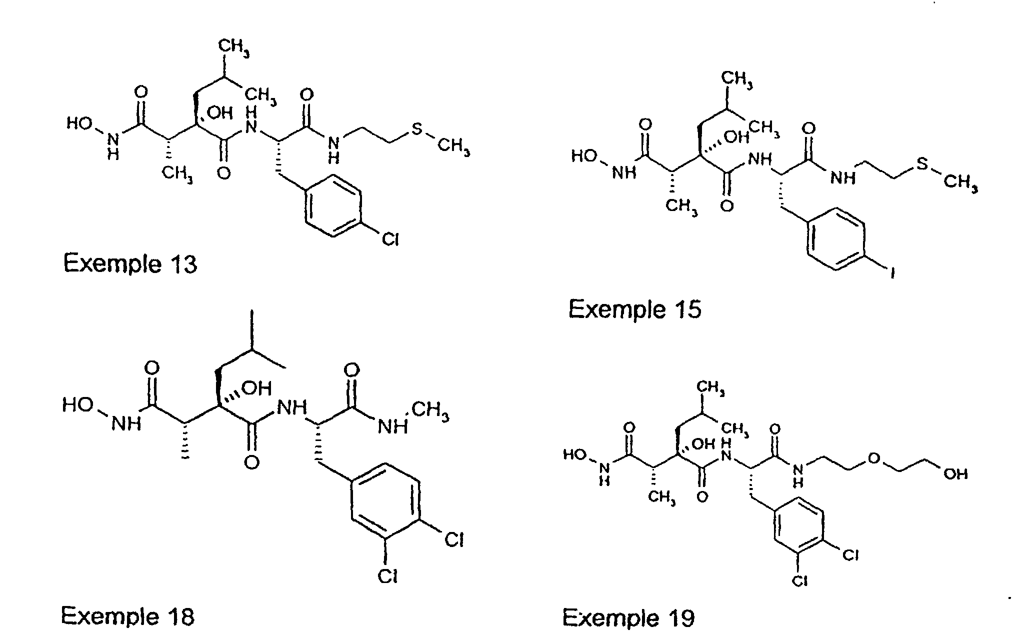

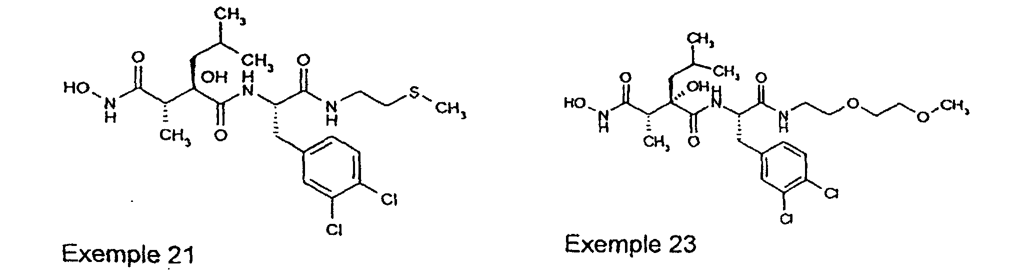

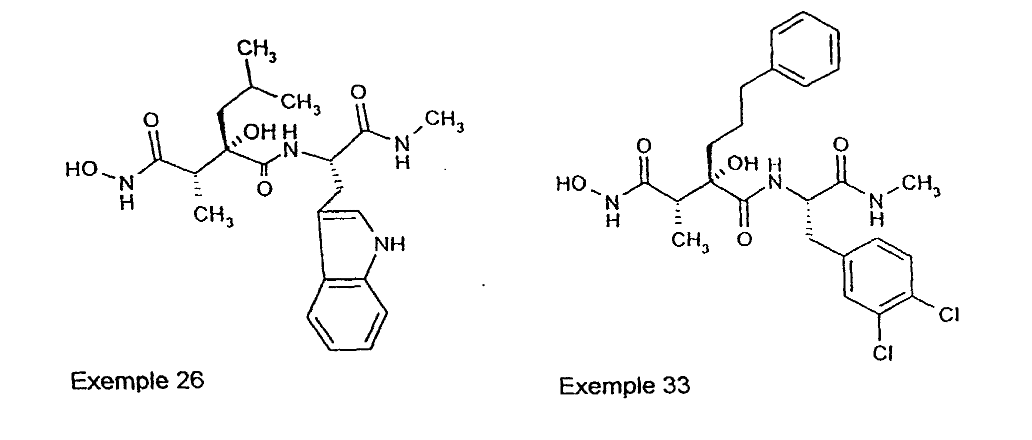

8. Composition pharmaceutique **caractérisée en ce qu'**elle comprend, à titre de principe actif, un (ou plusieurs) composé(s) et/ou un (ou plusieurs) mélange(s), selon l'une des revendications 1 à 5, en association avec un véhicule pharmaceutiquement acceptable.

9. Composition pharmaceutique selon la revendication 8, **caractérisée en ce qu'**elle se présente sous une forme administrable par voie orale, ou parentérale, ou rectale.

10. Composition pharmaceutique selon la revendication 8 ou la revendication 9, **caractérisée en ce que** la posologie en principe actif est d'environ 0,1 à environ 500 mg/kg/jour, de préférence de 1 à 300 mg/kg/jour par voies orale et rectale, et d'environ 0,1 µg/kg/jour à 1 mg/kg/jour par voie parentérale.

11. Composition pharmaceutique selon l'une des revendications 8 à 10, **caractérisée en ce qu'**elle se présente sous une forme administrable par voie orale, en dose unitaire de 1 mg à 250 mg de principe actif par prise, et de préférence de 10 mg à 250 mg de principe actif par prise, à raison de 1 à 4 prises par jour.

12. Composition pharmaceutique selon l'une des revendications 8 à 10, **caractérisée en ce qu'**elle se présente sous une forme administrable par voie parentérale, en dose unitaire de 1µg à 50 mg de principe actif par injection, à raison de 1 à 2 injections par jour.

13. Utilisation d'un (ou plusieurs) composé(s) de l'une des revendications 1 à 5, et/ou d'un (ou plusieurs) mélange(s) selon la revendication 6, pour la préparation d'un médicament destiné au traitement de pathologies humaines ou animales dans lesquelles sont impliqués les métalloprotéases, et/ou le TNFα, et/ou le TGFα.

14. Utilisation selon la revendication 13, pour la préparation d'un médicament ayant la propriété d'inhiber l'action des métalloprotéases impliquées dans la dégradation de la matrice extracellulaire, telles que les collagénases, gélatinases et stromélysines, ce médicament étant destiné au traitement de pathologies humaines ou animales liées à cette action des métalloprotéases, notamment au traitement :
- de l'arthrite rhumatoïde,
- de l'ostéoarthrite.
- de l'ostéoporose,
- de l'ulcération de la cornée.
- des périodontites,
- des gingivites,
- des invasions tumorales,
- de la prolifération métastatique,
- de l'athérosclérose,
- du SIDA,
- des maladies inflammatoires chroniques de l'intestin,
- des maladies neurodégénératives telles que la maladie d'Alzheimer, la sclérose en plaques.

15. Utilisation selon la revendication 13, pour la préparation d'un médicament ayant la propriété d'inhiber la libération de TNFα à partir de son précurseur inactif, ce médicament étant destiné au traitement de pathologies humaines ou animales, dans lesquelles le TNFα est impliqué, notamment au traitement des pathologies inflammatoires, immunologiques, infectieuses ou malignes, telles que :
- l'arthrite rhumatoïde,
- la maladie de Crohn,
- la sclérose en plaques,
- le choc septique,
- le cancer,
- la cachexie associée à une immunodéficience.

16. Utilisation selon la revendication 13, pour la préparation d'un médicament ayant la propriété d'inhiber la production de TGFα, ce médicament étant destiné au traitement de pathologies humaines ou animales dans lesquelles le TGFα est impliqué, telles que :
- le cancer,
- le psoriasis,
- l'eczéma,
- la formation de chéloïdes.
- la rétinopathie diabétique,
- l'athérosclérose,
- les maladies inflammatoires.

17. Procédé de préparation des composés de formule X tels que définis dans la revendication 1, dans laquelle Y représente -CONHOH (encore désignés ci-après composés de formule XV), **caractérisé en ce qu'**il est effectué selon le schéma suivant: 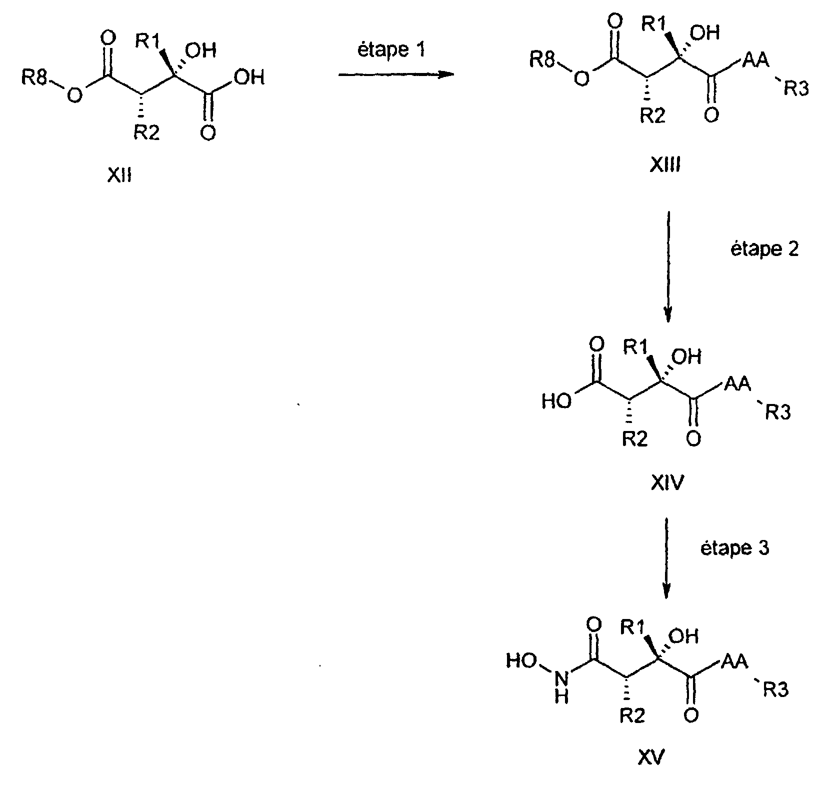 dans lequel :
- l'étape I consiste à condenser l'acide α-hydroxysuccinique XII (où R₈ est un groupe protecteur compatible avec les différents éléments de la molécule tel que t-butyle ou benzyle) avec un reste AA-R₃ où AA et R₃ sont tels que définis dans la revendication 1, par une méthode de couplage utilisée en synthèse peptidique et de préférence le PyBop à température ambiante durant 1 à 24 h (dans le cas où R₂ = OH, les alcools peuvent être protégés au préalable avec par exemple un dérivé silylé),
- l'étape 2 consiste à hydrolyser l'ester XIII obtenu à l'étape précédente en acide carboxylique XIV avec de l'acide trifluoroacétique, notamment à température ambiante dans un solvant tel que CH₂Cl₂ durant 1 à 10 h lorsque R₈ est t-butyle, ou à hydrogénolyser l'ester XIII en acide XIV avec par exemple H₂ Pd/C lorsque R₈ est benzyle (notamment sous pression atmosphérique dans un solvant polaire tel que l'éthanol durant 30 minutes à 10 h),
- dans l'étape 3, l'acide hydroxamique XV est formé par réaction de l'hydroxylamine, l'hydroxylamine O protégée ou l'hydroxylamine N,O diprotégée, préférentiellement avec l'hydroxylamine O-THP ou l'hydroxylamine O-benzyle (lorsque R₂ est différent d'alkylidène, aryloxyméthyle et hétéroarylthiométhyle) en présence d'un réactif de couplage tel que DCC/HOBT ou WSC/HOBT à température ambiante dans un solvant tel que THF, CH₂Cl₂, ou DMF pendant 1 à 24 h (lorsque R₂ = OH, les alcools sont protégés au préalable avec par exemple TMSCI) ; les hydroxylamines O ou N-O (di)protégées sont ensuite déprotégées suivant la nature du groupe protecteur par exemple en milieu acide pour l'hydroxylamine O-THP (notamment à température ambiante dans un mélange THF-H₂O pendant 1 à 24 h) ou H₂ Pd/C pour l'hydroxylamine O-benzyle (notamment sous pression atmosphérique dans un solvant polaire tel que l'éthanol pendant 30 minutes à 10 h).

18. Procédé de préparation des composés de formule X tels que définis dans la revendication 1, dans laquelle Y représente -CONHOH (encore désignés ci-après composés de formule XV), sous réserve que R1 n'est pas un groupe hétéroarylalkyle, et que R2 n'est pas un groupe hétéroarylthiométhyle, **caractérisé en ce qu'**il comprend :
- une réaction d'aldolisation à partir d'un céto-ester XXVII et d'un alcène XXVIII (notamment en présence d'un acide de Lewis tel que SnCl₄ à -80°C dans un solvant tel que CH₂Cl₂ durant 5 minutes à 2 h), ou à partir d'un céto-acide (sous forme de sel de sodium ou triéthylamine) XXX et un d'alcène XXXI (notamment à température ambiante entre 1 et 10 h dans un mélange THF-H₂O) selon le schéma suivant : 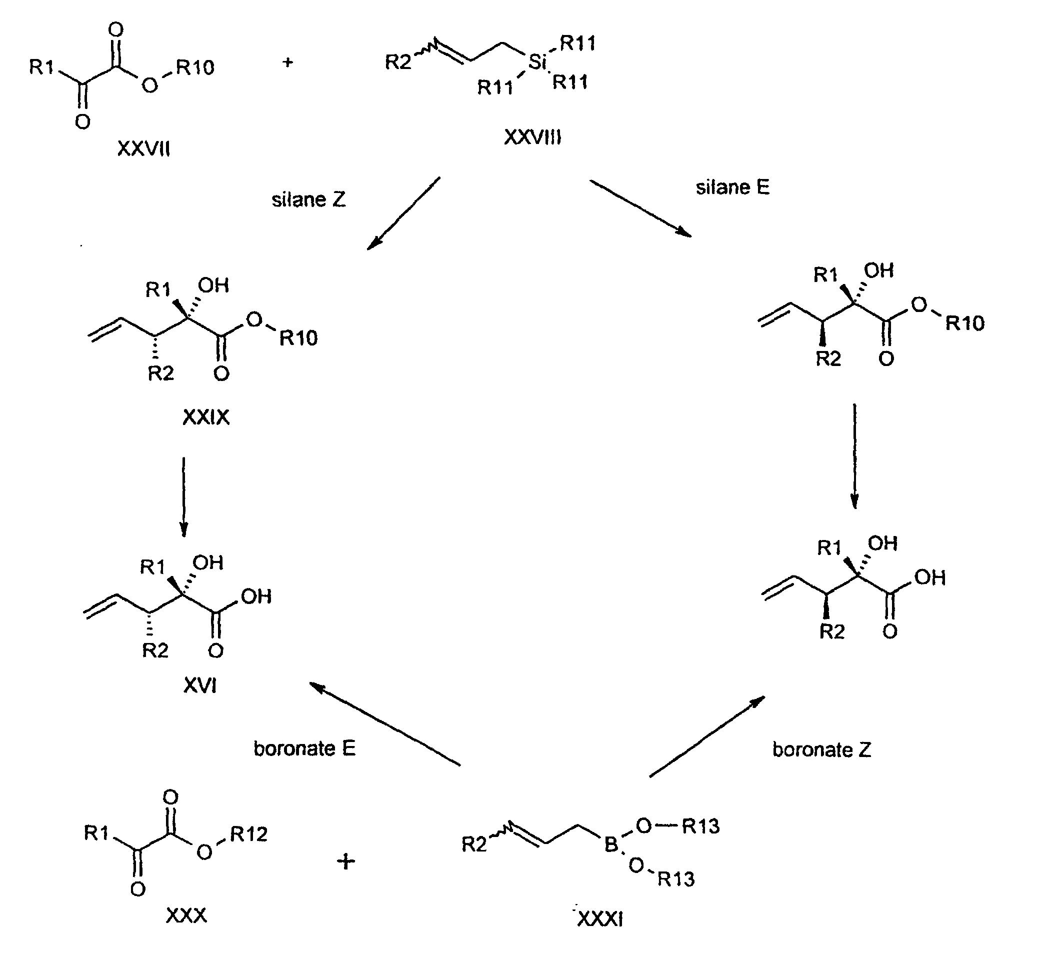
dans lequel :
- R₁ et R₂ ont la signification indiquée ci-dessus,
- R₁₀ est un alkyle éventuellement ramifié en C₁-C₁₂, un benzyle ou un composé optiquement pur tel que l'acide mandélique estérifié par un alkyle en C₁-C₃ linéaire ou ramifié, ou un benzyle,
- R₁ est un alkyle en C₁-C₃ linéraire ou ramifié, ou un chlore,
- R₁₂ est le sodium ou la triéthylamine,
- R₁₃ est l'hydrogène, un alkyle en C₁-C₃ linéaire ou ramifié ; R₁₃ peut également représenter un enchaînement formant un cycle avec l'atome de bore tel que par exemple le di-isopropyltartrate,
- les réactions sont diastéréosélectives et conduisent aux dérivés de stéréochimie XVI si la double liaison est de géométrie Z pour les composés XXVIII et de géométrie E pour les composés XXXI.
- l'obtention des composés de formule XV susmentionnés étant ensuite effectuée selon le schéma réactionnel suivant : 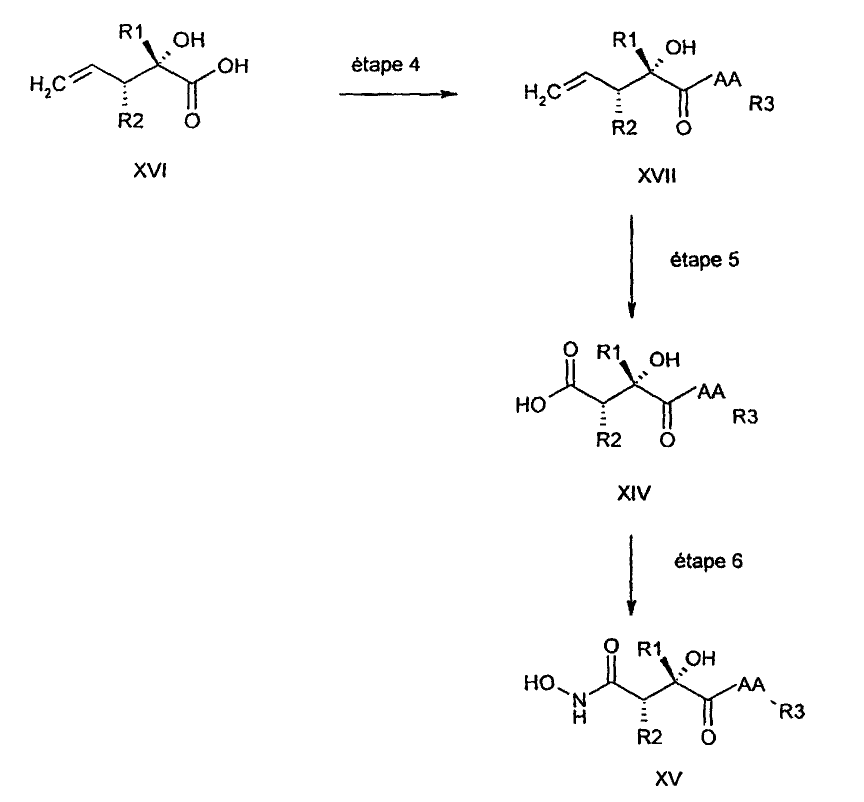
dans lequel :
- les étapes 4 et 6 sont réalisées comme dans les étapes 1 et 3 du schéma réactionnel de la revendication 17 respectivement, et à partir des composés XVI et XIV, ce qui conduit aux composés de formule XVII et XV respectivement,
- l'étape 5 consiste à oxyder la double liaison éthylénique du composé de formule XVII en acide notamment par ozonolyse (par exemple à -60°C dans CH₂Cl₂ jusqu'à obtention d'une coloration bleue persistante) puis oxydation (notamment à température ambiante avec NaClO₂ et NaH₂PO₄ dans tBuOH-H₂O durant 15 h) ou directement par KMnO₄/NalO₄ (notamment à température ambiante dans un mélange tBuOH-H₂O durant 1 à 10 h), ce qui conduit au composé de formule XIV.

19. Procédé de préparation des composés de formule X tels que définis dans la revendication 1, dans laquelle Y représente :
• -SH, ou
• un groupe de formule 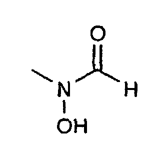
ou
• un groupe de formule 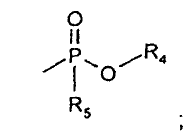
dans lequel R4 et R5 sont tels que définis dans la revendication 1,
ledit procédé étant effectué selon le schéma suivant : 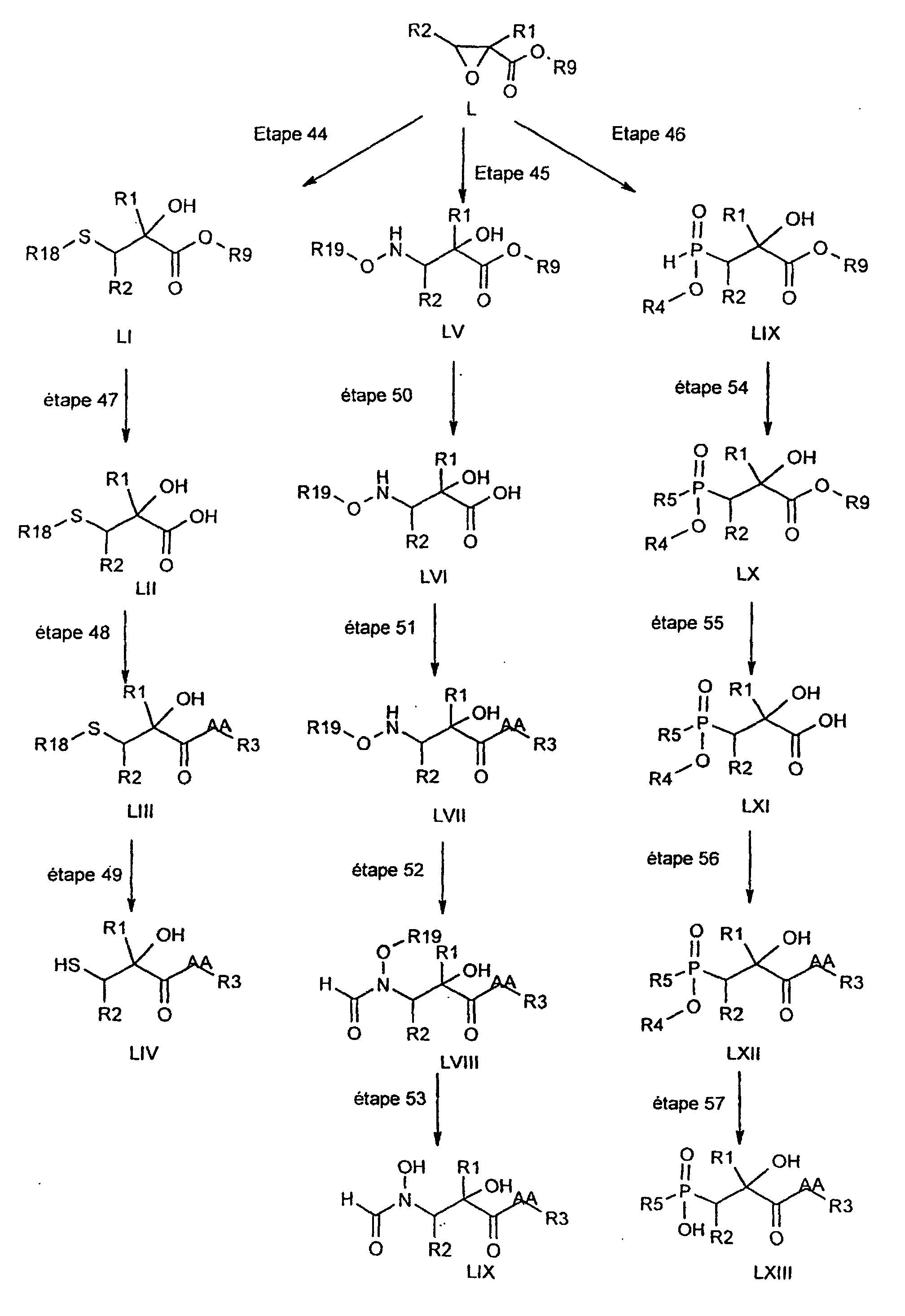
- l'étape 44 consiste à ouvrir un époxyde de formule L dans laquelle R₁ et R₂ sont tels que définis dans la revendication 1, et R₉ est un groupe protecteur d'acide carboxylique, notamment un reste benzyle sensible à l'hydrogenolyse catalytique, cette ouverture de l'epoxyde L étant effectuée par un nucléophile par exemple un thiol protégé par un groupe R₁₈ compatible avec R₉, par exemple un benzyle dans le méthanol durant 1 h à 60°C,
- l'étape 47 consiste à déprotéger l'ester LI, par exemple avec de l'acide triffuoroacétique comme précédemment,
- l'étape 48 est identique à l'étape 1 du schéma réactionnel de la revendication 17, et réalisée à partir du compose LII obtenu à l'étape précédente,
- l'étape 49 consiste à déprotéger le soufre par exemple avec du sodium dans l'ammoniac liquide, notamment à -60°C durant 5 à 15 minutes,
- l'étape 45 consiste à ouvrir l'époxyde L par de l'hydroxylamine protégée telle que définie dans l'étape 3 du procédé selon la revendication 17 (avec, par exemple, R₁₉ = benzyle ou THP),
- l'étape 50 consiste à déprotéger l'ester LV par une méthode compatible avec R₁₉,
- l'étape 51 est identique à l'étape 48, et réalisée à partir du composé LVI obtenu à l'étape précédente,
- l'étape 52 consiste à faire réagir l'hydroxylamine LVII avec de l'acide formique et de l'anhydride acétique, notamment à une température d'au moins 100°C durant 1 à 15 h,
- l'étape 53 consiste à cliver R₁₉ sur le composé LVIII avec par exemple H₂ Pd/C ou HCI 1N dépendant de la structure de R₁₉ comme précédemment,
- l'étape 46 consiste à ouvrir un époxyde L par de l'acide hypophosphoreux de formule H₃PO₂ puis esterification avec un agent de couplage tel que DCC et un groupe R₄OH dans lequel R₄ est tel que défini dans la revendication 1, en présence par exemple de triméthylorthoformate et de tétraméthylguanidine à température ambiante pendant 5 h,
- l'étape 54 consiste à traiter le composé LIX avec un composé de formule : 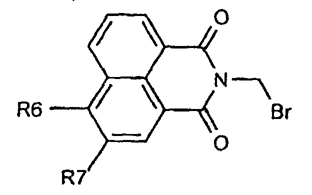 (préparé selon les méthodes décrites dans la littérature) dans laquelle R₆ et R₇ sont tels que définis ci-dessus, notammant dans CH₂Cl₂ en présence de bis triméthylsilyle acétamide à température ambiante pendant 5 h, ce qui conduit à l'obtention du composé de formule LX dans laquelle R₅ représente : 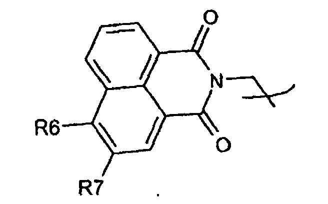
- l'étape 55 consiste à cliver l'ester R₉ par une méthode compatible avec R₄ comme précédemment.
- l'étape 56 est identique à l'étape 48, et réalisée à partir du composé LXI obtenu à l'étape précédente.
- l'étape 57 consiste à cliver le groupe R₄ du composé LXII obtenu à l'étape précédente, par exemple, à l'aide de Nal dans l'acétone au reflux durant 15 h.

## Patentansprüche

1. Verbindung der nachstehenden allgemeinen Formel (X) : 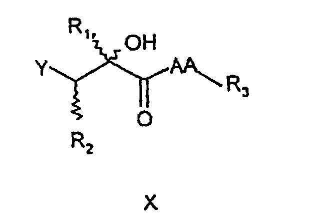 in der:
- Y einen der nachfolgenden Reste darstellt:
• -CONHOH oder
• -SH oder
• einen Rest der Formel 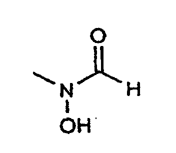 oder
• einen Rest der allgemeinen Formel 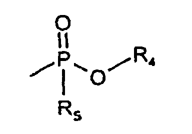 in der:
• der Rest R₄ ein Wasserstoffatom oder einen C₁-C₆-Alkylrest oder einen Phenylalkylrest mit einem C₁-C₆-Alkylrest darstellt und
• der Rest R₅ einen Rest der allgemeinen Formel 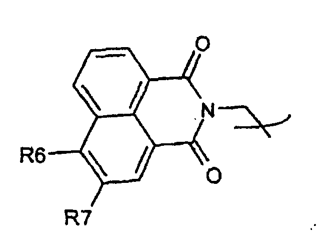 darstellt, in der
• der Rest R₆ ein Wasserstoffatom oder einen C₁-C₆-Alkoxyrest oder einen Benzyloxyrest darstellt und
• der Rest R₇ ein Wasserstoffatom oder ein Halogenatom, wie -Cl oder -Br darstellt,
- R₁ einen der nachfolgenden Reste darstellt:
• einen linearkettigen oder verzweigtkettigen C₃-C₁₆-Alkylrest oder cyclischen C₃-C₆-Alkylrest, wobei die Kette ggf. ein Heteroatom, wie O, S oder N, aufweist, oder
• einen substituierten oder unsubstituierten Phenoxyalkylrest oder Phenylalkylrest oder einen Heteroarylalkylrest mit einem C₂-C₅-Alkylrest,
- R₂ einen der nachfolgenden Reste darstellt:
• ein Wasserstoffatom oder
• einen C₁-C₅-Alkylrest oder C₂-C₅-Alkylidenrest oder
• eine Hydroxylgruppe, einen C₁-C₆-Alkoxyrest oder einen Benzyloxyrest, unter der Maßgabe, dass der Rest Y den Rest -CONHOH darstellt, wenn der Rest R₂ eine Hydroxylgruppe ist oder
• einen Hydroxymethyl- oder Alkoxymethylrest mit 1-6 C-Atomen oder
• einen Arylalkylrest mit einem C₁-C₆-Alkylrest, einen Aryloxymethylrest, einen Arylthiomethylrest, einen Heteroarylthiomethylrest, in denen der Arylrest einen Phenylrest darstellt, der gegebenenfalls subsituiert ist, und zwar insbesondere mit -OH, -OCH₃, einem linearen oder verzweigtkettigen C₁-C₃-Alkylrest, einem Halogenatom, wie -Cl oder -Br, einer Aminogruppe, wie NH₂, -NHCOCH₃ und -NHCOOR₁₀, wobei der Rest R₁₀ einen linearen oder verzweigtkettigen C₁-C₃-Alkylrest darstellt, oder
• einen Phtalimidoalkylrest mit einem C₁-C₆-Alkylrest oder
• einen Alkoxycarbonylmethylrest, wobei der Alkoxyrest der Methoxy- oder Ethoxyrest ist, einen Benzyloxycarbonylmethylrest, einen Acetylmethylrest, unter der Maßgabe, dass im Falle dieser drei Reste der Rest Y gleich -SH ist,
- AA eine Aminosäure oder eine Aminosäuresequenz darstellt, wobei die Aminosäuren natürliche oder künstliche Aminosäuren sind und vorzugsweise in der absoluten S-Konfiguration vorliegen, insbesondere eine Aminosäure der allgemeinen Formel ist: 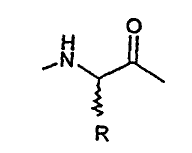 in der der Rest R einen der folgenden Reste darstellt:
• einen linearkettigen oder verzweigtkettigen C₁-C₄-Alkylrest oder
• einen -CH₂-Y' Rest, in dem der Rest Y' einen Ring mit 4 bis 6 C-Atomen im Ring darstellt und ggf. ein oder mehrere Heteroatome, wie O, S oder N, enthält, wobei der Ring ein aromatischer oder nicht-aromatischer Ring ist, der ggf. insbesondere durch eine oder mehrere der Gruppen -OCH₃, -NO₂, -NH₂ oder durch ein oder mehrere Halogenatome, insbesondere ausgewählt aus -Cl, -Br, -F und - I substituiert ist, oder
• eine Gruppe der Formel 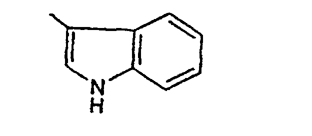
- R₃ einen Rest der allgemeinen Formel -NH-(R₈)ₙ-R₉ darstellt, in der
• n 0 oder 1 ist,
• der Rest R₈ einen linearkettigen oder verzweigtkettigen C₁-C₈-Alkylrest darstellt, der ggf. ein oder mehrere Heteroatome, wie O öder S, aufweist und
• der Rest R₉ ein Wasserstoffatom oder eine Methyl-, Nitril-, Morpholino-, Phenyl-, Methoxy-, Hydroxyl-, Thiomethylgruppe oder einen Rest der Formel -CH(NH₂) = N-OH oder -N(CH₃)₂ darstellt.

2. Verbindung nach Anspruch 1, **gekennzeichnet durch** die nachstehende allgemeine Formel (Xa) : 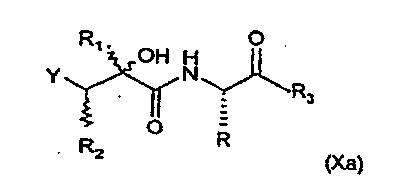 in der
- Y den Rest -CONHOH darstellt,
R₁ ausgewählt ist aus
• -CH(CH₃)₂,
• -CH₂-CH(CH₃)₂, oder
• 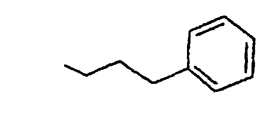
- R₂ ausgewählt ist aus:
• einem Alkylrest mit 1 bis 5 C-Atomen, insbesondere einer Methyl- oder Propylgruppe,
• einer Hydroxylgruppe, oder
• einer Alkoxygruppe mit 1 bis 5 Kohlenstoffatomen, insbesondere einer Methoxygruppe,
- R ausgewählt ist aus
• -C(CH₃)₃,
• -CH₂-CH(CH₃)₂,
• einem allgemeinen Rest der Formel 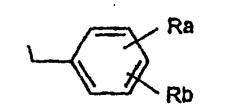 der aromatisch oder nicht-aromatisch ist, in der die Reste Rₐ und R_{b} unabhängig voneinander -H, -Cl, -Br, -I, -F, -OCH₃, -NO₂ und -NH₂ darstellen, und
• einem Rest der Formel 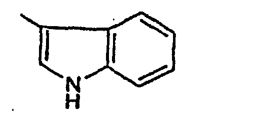 und
- R₃ einen allgemeinen Rest der Formel -NH-(CH₂)ₙ₁-R₉ darstellt, in der
• n₁ 0,1 oder 2 ist und
• der Rest R₉ -CH₃, -C≡N, -COOCH₃, -SCH₃, -O-(CH₂)₂-OH, -O-(CH₂)₂-OCH₃, -CH(NH₂)=N-OH, 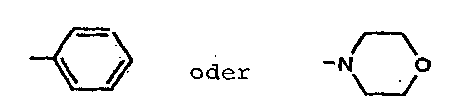 darstellt.

3. Verbindung nach Anspruch 1 oder 2, **dadurch gekennzeichnet, dass** sie eine Stereochemie; derart, dass die Substituenten R₁ und R₂ in Antistellung zu dem Bernsteinsäurerest angeordnet sind, gemäß der nachstehenden allgemeinen Formel (XI.1) aufweist:

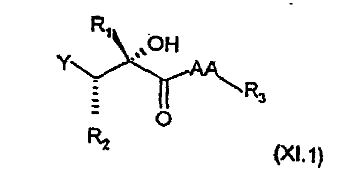

4. Verbindung nach einem der Ansprüche 1 bis 3, **dadurch gekennzeichnet, dass** der Rest R einen Rest der allgemeinen Formel 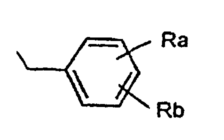 darstellt, in der die Reste Rₐ und R_{b} ein Halogenatom, insbesondere ein Chloratom darstellen.

5. Verbindung nach einem der Ansprüche 1 bis 4, **dadurch gekennzeichnet, dass** der Rest R₃ einen Rest der Formel
-NH- (CH₂)₂-SCH₃
darstellt.

6. Zusammensetzung, **dadurch gekennzeichnet, dass** sie Verbindungen der nachstehenden allgemeinen Formel (XI.1) 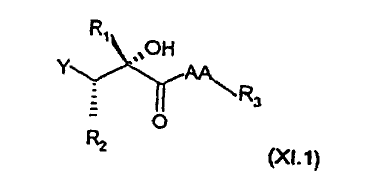 in der die Reste Y, R₁, R₂, AA und R₃ wie in einem der Ansprüche 1 bis 5 definiert sind, und Verbindungen der nachstehenden allgemeinen Formel (XI.2) 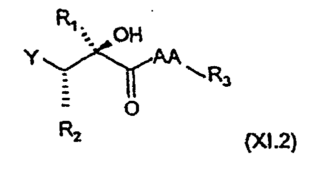 enthält, in der die Reste Y, R₁, R₂, AA und R₃ die vorstehend angegebene Bedeutung haben, wobei der Anteil der Verbindungen (XI.1) und (XI.2) in der Zusammensetzung vorzugsweise etwa 50% bis etwa 99% für die Verbindung der allgemeinen Formel (XI.1) und etwa 50% bis etwa 1% für die Verbindung der allgemeinen Formel (XI.2) beträgt.

7. Verbindung nach einem der Ansprüche 1 bis 5, **gekennzeichnet durch** eine der nachstehenden Formeln:

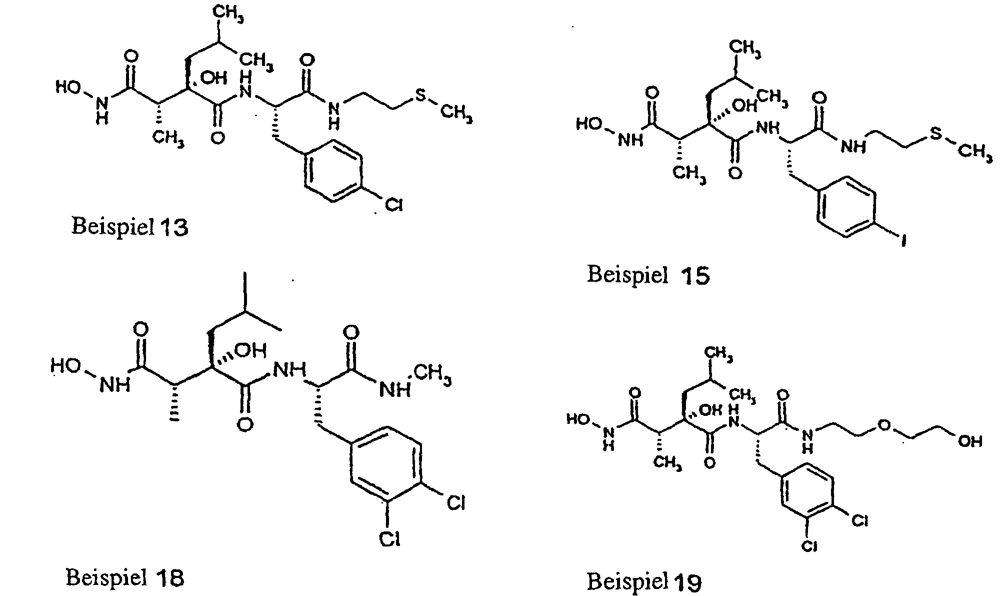

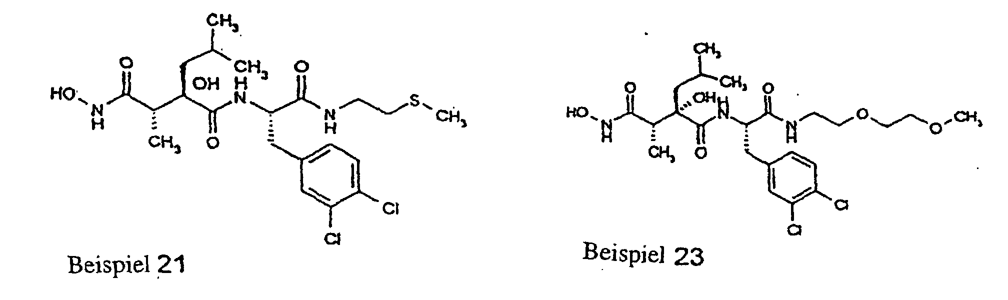

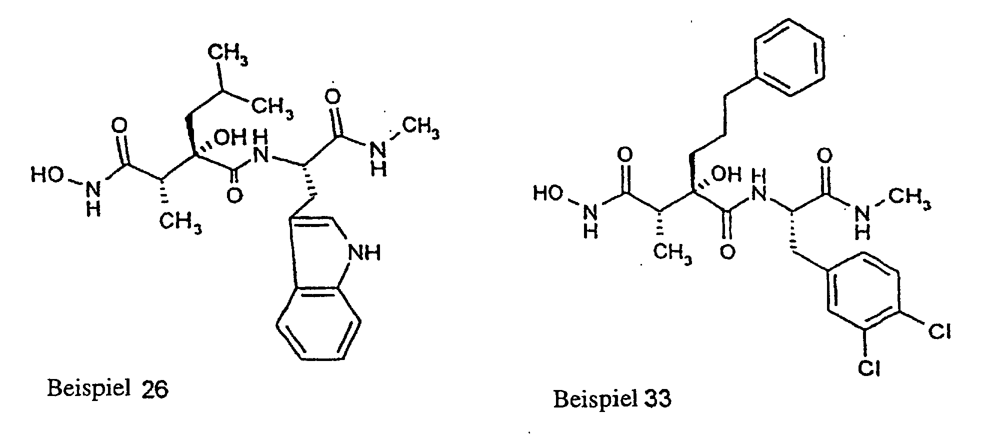

8. Pharmazeutische Zubereitung, **dadurch gekennzeichnet, dass** sie als aktiven Wirkstoff eine oder mehrere der Verbindungen nach einem der Ansprüche 1 bis 5 und/oder eine oder mehrere der Zusammensetzungen derselben zusammen mit einem pharmazeutisch verträglichen Trägerstoff enthält.

9. Pharmazeutische Zubereitung nach Anspruch 8, **dadurch gekennzeichnet, dass** sie in einer peroral, parenteral oder rektal verabreichbaren Form vorliegt.

10. Pharmazeutische Zubreitung nach Anspruch 8 oder 9, **dadurch gekennzeichnet, dass** die Dosierung des Wirkstoffs etwa 0,1 bis etwa 500 mg/kg/Tag und vorzugsweise 1 bis etwa 300 mg/kg/Tag bei der peroralen und rektalen Verabreichung beträgt und etwa 0,1 µg/kg/Tag bis 1 mg/kg/Tag bei der parenteralen Verabreichung beträgt.

11. Pharmazeutische Zubereitung nach einem der Ansprüche 8 bis 10, **dadurch gekennzeichnet, dass** sie in einer peroral zu verabreichenden Form mit einer Einzeldosis von 1 mg bis 250 mg Wirkstoff pro Einnahme, vorzugsweise 10 mg bis 250 mg Wirkstoff pro Einnahme bei 1 bis 4 Einnahmen pro Tag vorliegt.

12. Pharmazeutische Zubereitung nach einem der Ansprüche 8 bis 10, **dadurch gekennzeichnet, dass** sie in einer parenteral zu verabreichenden Form mit einer Einzeldosis von 1 µg bis 50 µg Wirkstoff pro Injektion bei 1 bis 2 Injektionen pro Tag vorliegt.

13. Verwendung einer oder mehrerer der Verbindungen nach einem der Ansprüche 1 bis 5 und/oder einer oder mehreren Zusammensetzung(en) nach Anspruch 6 zur Herstellung eines Medikaments zur Behandlung von Erkrankungen des Menschen oder Tieres, in denen Metalloproteasen und/oder α-TNF und/oder α-TGF eine Rolle spielen.

14. Verwendung nach Anspruch 13, zur Herstellung eines Medikaments zur Inhibierung der Wirkung von Metalloproteasen, die bei der Zersetzung der extrazellulären Matrix eine Rolle spielen, wie die Collagenasen, Gelatinasen und Stromelysine, wobei das Medikament zur Behandlung von Erkrankungen des Menschen oder Tieres, die mit dieser Wirkung der Metalloproteasen in Verbindung stehen, vorzugsweise zur Behandlung von
- Gelenkrheumatismus,
- Osteoarthritis,
- Osteoporose,
- Ulkusbildung der Hornhaut (des Auges),
- Periodontitis,
- Gingivitis,
- tumoröse Invasionen,
- metastatische Proliferation,
- Atheriosklerose,
- Aids,
- chronischen Entzündungserkrankungen des Darms und
- neurodegenerativen Erkrankungen, wie die Alzheimer Krankheit und Multiple Sklerose, bestimmt ist.

15. Verwendung nach Anspruch 13 zur Herstellung eines Medikaments zur Inhibierung der Freisetzung von α-TNF ausgehend von seiner inaktiven Vorstufe, wobei das Medikament zur Behandlung von Erkrankungen des Menschen oder Tieres dient, bei denen α-TNF eine Rolle spielt, vorzugsweise bei der Behandlung von Entzündungserkrankungen, immunologischen Erkrankungen, infektiösen Erkrankungen und malignen Erkrankungen, wie
- Gelenkrheumatismus,
- Crohnsche Krankheit,
- Multiple Sklerose,
- septischer Schock,
- Krebs,
- Kachexie in Verbindung mit einer Immunschwäche.

16. Verwendung nach Anspruch 13 zur Herstellung eines Medikaments zur Inhibierung der Produktion von α-TGF, wobei das Medikament zur Behandlung von Erkrankungen des Menschen und Tieres dient, in denen α-TGF eine Rolle spielt, wie
- Krebs,
- Psoriasis,
- Ekzeme,
- die Bildung von Keloiden,
- diabetische Retinopathie,
- Atherosklerose, und
- Entzündungserkrankungen.

17. Verfahren zur Herstellung von Verbindungen der Formel (X), wie in Anspruch 1 definiert, in der Y den Rest -CONHOH darstellt (nachstehend auch als Verbindung der Formel XV bezeichnet), **gekennzeichnet durch** die nachstehenden Schritte: 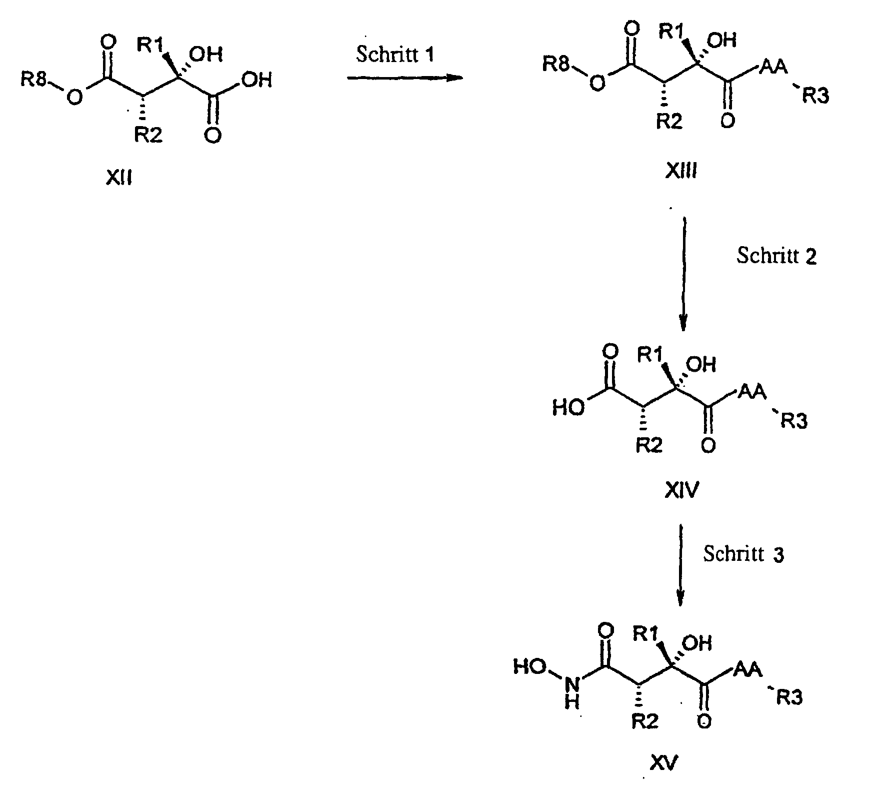 wobei:
- Schritt 1 darin besteht, die α-Hydroxybernsteinsäure XII (in der der Rest R₈ eine Schutzgruppe ist, die mit den verschiedenen Elementen des Moleküls kompatibel ist, wie Tertiärbutyl oder Benzyl) mit einem Rest AA-R₃ zu kondensieren, in dem AA und R₃, wie sie im Anspruch 1 definiert sind, mittels eines Verknüpfungsverfahrens, wie es bei der Synthese von Peptiden verwendet wird, und vorzugsweise dem PyBop bei Raumtemperatur für 1 bis 24 h (für den Fall, dass R₂ = OH können die Hydroxylgruppen zuvor z.B. mit einem Silylderivat geschützt werden),
- Schritt 2 darin besteht, den in dem vorhergehenden Schritt erhaltenen Ester XIII mit Trifluoressigsäure zu der Carbonsäure XIV zu hydrolisieren, vorzugsweise bei Raumtemperatur in einem Lösungsmittel, wie CH₂Cl₂ innerhalb von 1 bis 10 h, wenn der Rest R₈ der Tertiärbutylrest ist, oder den Ester XIII **durch** Hydrogenolyse in die Säure XIV unter Verwendung von z.B. H₂ Pd/C zu überführen, wenn R₈ der Benzylrest ist (vorzugsweise unter Atmosphärendruck in einem polaren Lösungsmittel, wie Ethanol, für 30 Minuten bis 10 Stunden),
- im Schritt 3 die Hydroxamsäure XV gebildet wird **durch** Umsetzung mit Hydroxylamin, einem O-geschützten Hydroxylamin oder einem N,O-digeschützten Hydroxylamin, vorzugsweise mit dem Hydroxylamin-O-THP oder Hydroxylamin-O-benzyl (wenn der Rest R₂ verschieden ist von einem Alkyliden-, Aryloxymethylund Heteroarylthiomethylrest) in Gegenwart eines Kupplungsreagens, wie DCC/HOBT oder WSC/HOBT, bei Raumtemperatur in einem Lösungsmittel, wie THF, CH₂Cl₂ oder DMF binnen 1 bis 24 h (wenn der Rest R₂ = OH werden die Hydroxylgruppen zuvor mit z.B. TMSCl geschützt), wobei die O- oder N,O-(di)geschützten Hydroxylamine anschließend entschützt werden, abhängig von der Natur der Schutzgruppe z.B. im Säuremilieu im Falle von Hydroxylamin-O-THP (vorzugsweise bei Raumtemperatur in einem Gemisch aus THF/H₂O binnen 1 bis 24 h) oder **durch** H₂ Pd/C im Falle von Hydroxylamin-O-benzyl (vorzugsweise unter Atmosphärendruck in einem polaren Lösungsmittel, wie Ethanol, binnen 30 Minuten bis 10 h).

18. Verfahren zur Herstellung von Verbindungen der allgemeinen Formel (X), wie im Anspruch 1 definiert, in der Y den Rest -CONHOH darstellt (nachstehend auch als Verbindung der Formel XV bezeichnet), unter der Maßgabe, dass der Rest R₁ kein Heteroarylalkylrest ist und der Rest R₂ kein Heteroarylthiomethylrest ist, **gekennzeichnet durch**
- eine Aldolreaktion ausgehend von dem Ketoester XXVII und dem Alken XXVIII (vorzugsweise in Gegenwart einer Lewissäure, wie SnCl₄ bei -80°C in einem Lösungsmittel, wie CH₂Cl₂ binnen 5 Minuten bis 2 Stunden), oder ausgehend von einer Ketosäure (in Form ihres Natrium- oder Triethylaminsalzes) XXX und einem Alken XXXI (vorzugsweise bei Raumtemperatur binnen 1 bis 10 h in einem Gemisch aus THF-H₂O) gemäß dem nachstehenden Reaktionsschema: 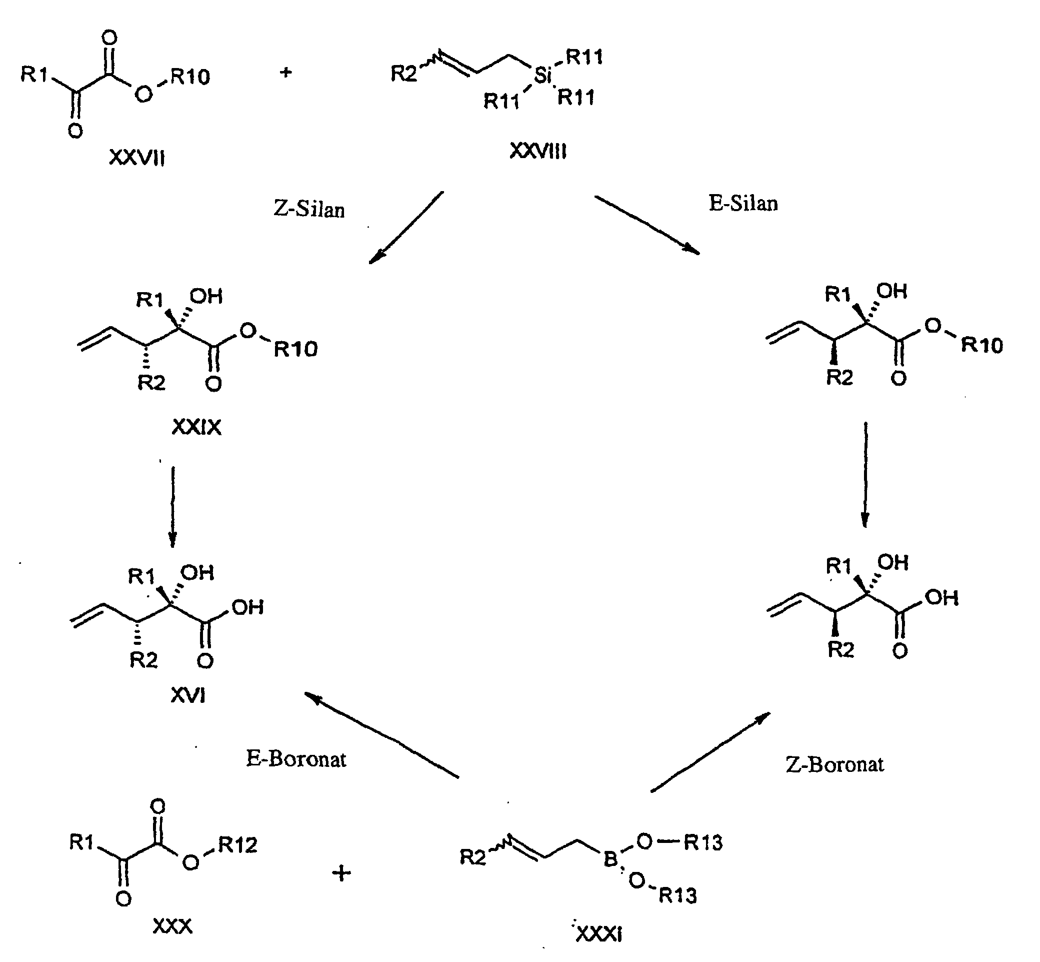
wobei
- die Reste R₁ und R₂ die vorstehend angegebene Bedeutung haben,
- der Rest R₁₀ ein ggf. verzweigtkettiger C₁-C₁₂-Alkylrest, ein Benzylrest oder eine optisch reine Verbindung ist, wie Mandelsäure, verestert mit einem linearkettigen oder verzweigtkettigen C₁-C₃-Alkylrest oder einem Benzylrest,
- der Rest R₁₁ ein linearkettiger oder verzweigtkettiger C₁-C₃-Alkylrest oder ein Chloratom ist,
- der Rest R₁₂ Natrium oder Triethylamin ist,
- der Rest R₁₃ ein Wasserstoffatom oder ein linearkettiger oder verzweigtkettiger C₁-C₃-Alkylrest ist, wobei der Rest R₁₃ auch eine mit dem Boratom einen Ring bildende Verknüpfung, wie das Diisopropyltartrat, darstellen kann,
- die Umsetzungen diastereoselektiv sind und zu den Derivaten mit der Stereochemie gemäß Formel XVI führen, wenn die Doppelbindung Z-Geometrie für die Verbindungen der Formel XXVIII und E-Geometrie für die Verbindungen der Formel XXXI hat,
- wobei die Herstellung der vorstehend erwähnten Verbindung der allgemeinen Formel XV anschließend gemäß dem nachstehend angegebenen Reaktionsschema erfolgt: 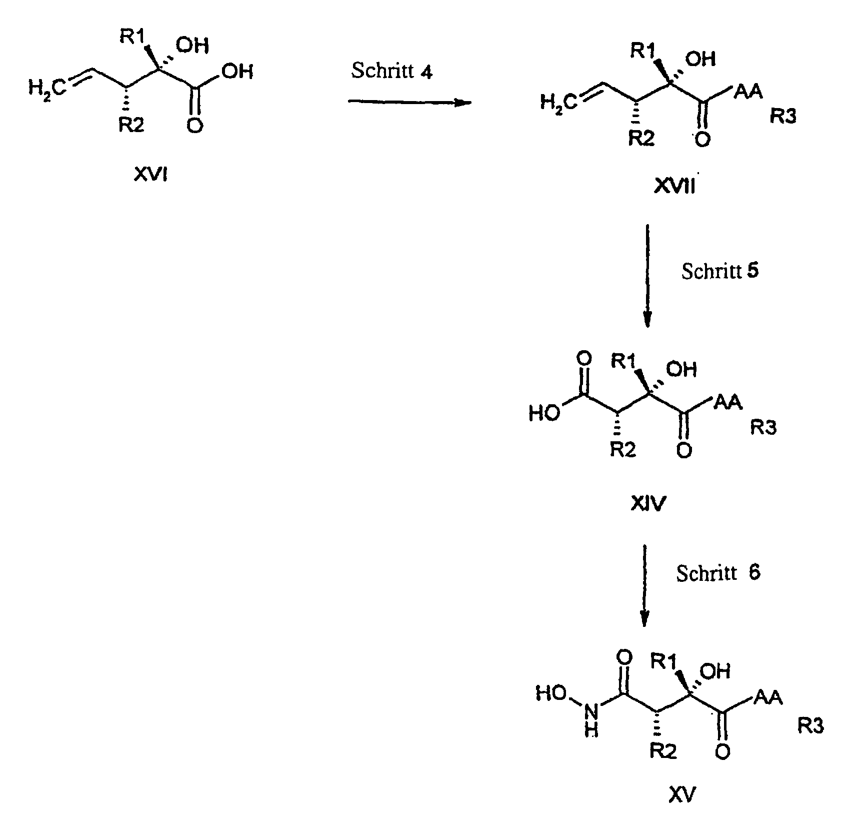
wobei:
- die Schritte 4 und 6 entsprechend den Schritten 1 und 3 des im Anspruch 17 angegebenen Reaktionsschemas ausgeführt werden, ausgehend von den Verbindungen XVI und XIV, was zu den Verbindungen der allgemeinen Formel XVII bzw. XV führt, und
- der Schritt 5 darin besteht, die ethylenische Doppelbindung der Verbindung der allgemeinen Formel XVII zur Säure zu oxidieren, vorzugsweise **durch** Ozonolyse (z.B. bei -60°C in CH₂Cl₂, bis eine bleibende Blaufärbung entsteht) und anschließende Oxidation (vorzugsweise bei Raumtemperatur mit NaClO₂ und NaH₂PO₄ in tBuOH-H₂O binnen 15 h) oder direkt **durch** KMnO₄/NaIO₄ (vorzugsweise bei Raumtemperatur in einem Gemisch aus
tBuOH-H₂O binnen 1 bis 10 h), um die Verbindung der allgemeinen Formel XIV zu erhalten.

19. Verfahren zur Herstellung von Verbindungen der allgemeinen Formel X, wie in Anspruch 1 definiert, in der Y einen der folgenden Reste darstellt:
• - SH,
• - einen Rest der Formel 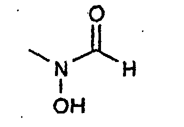 oder
• - einen Rest der allgemeinen Formel 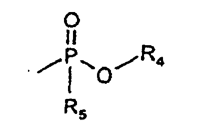 in der die Reste R₄ und R₅ im Anspruch 1 definiert sind, wobei das Verfahren nach dem folgenden Reaktionsschema ausgeführt wird: 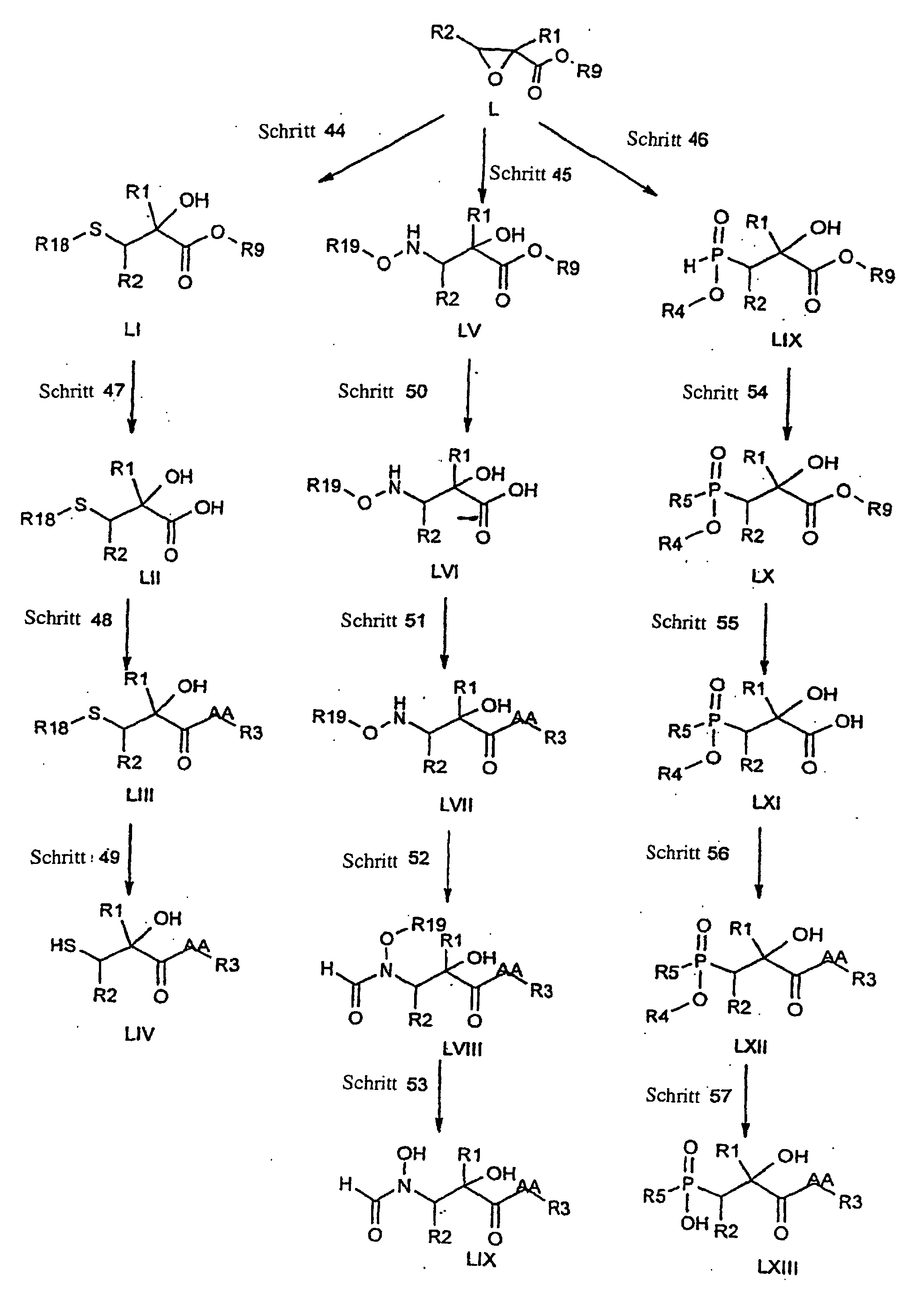
wobei
- der Schritt 44 darin besteht, das Epoxid der Formel L zu öffnen, in der die Reste R₁ und R₂ wie im Anspruch 1 definiert sind und der Rest R₉ eine Schutzgruppe für die Carbonsäure ist, vorzugsweise ein Benzylrest, der gegenüber einer katalytischen Hydrogenolyse empfindlich ist, wobei die Öffnung des Epoxids L durch ein Nukleophil erfolgt, z.B. einem mit dem Rest R₁₈ geschützten Thiol, kompatibel mit dem Rest R₉, z.B. einem Benzylrest in Methanol für 1 h bei 60°C,
- der Schritt 47 darin besteht, den Ester LI zu entschützen, z.B. wie vorstehend mit Trifluoressigsäure,
- Schritt 48 dem Schritt 1 des Reaktionsschemas von Anspruch 17 entspricht, ausgehend von der Verbindung LII, die im vorhergehenden Schritt erhalten wurde,
- Schritt 49 darin besteht, den Schwefel zu entschützen, z.B. mit Natrium in flüssigem Ammoniak, vorzugsweise bei -60°C binnen 5 bis 15 Minuten,
- Schritt 45 darin besteht, das Epoxid L mit einem geschützten Hydroxylamin zu öffnen, wie in Schritt 3 des Verfahrens gemäß Anspruch 17 angegeben (wobei z.B. R₁₉ = Benzyl oder THP),
- Schritt 50 darin besteht, den Ester LV mit einem Verfahren zu entschützen, das mit dem Rest R₁₉ kompatibel ist,
- Schritt 51 identisch ist mit Schritt 48 und ausgehend von der im vorhergehenden Schritt erhaltenen Verbindung LVI ausgeführt wird,
- Schritt 52 darin besteht, das Hydroxylamin LVII mit Ameisensäure und Essigsäureanhydrid umzusetzen, vorzugsweise bei einer Temperatur von mindestens 100°C binnen 1 bis 15 h,
- Schritt 53 darin besteht, den Rest R₁₉ der Verbindung LVIII mit z.B. H₂ Pd/C oder 1N HCl, je nach der vorstehend angegebenen Struktur des Rests R₁₉, abzuspalten,
- der Schritt 46 darin besteht, das Epoxid L mit hypophosphoriger Säure der Formel H₃PO₂ zu öffnen und anschließend eine Veresterung durchzuführen mit einem Kupplungsreagens, wie DCC und einer Verbindung R₄OH, in der der Rest R₄ wie im Anspruch 1 definiert ist, in Gegenwart von z.B. Trimethylorthoformiat und Tetramethylguanidin bei Raumtemperatur binnen 5 h,
- Schritt 54 darin besteht, die Verbindung LIX mit einer Verbindung der allgemeinen Formel 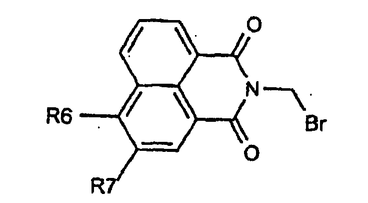 (hergestellt nach den in der Literatur beschriebenen Verfahren), in der die Reste R₆ und R₇ die vorstehend angegebene Bedeutung haben, vorzugsweise in CH₂Cl₂ in Gegenwart von Bistrimethylsilylacetamid bei Raumtemperatur binnen 5 h zu behandeln, unter Bildung der Verbindung der allgemeinen Formel LX, in der der Rest R₅ die allgemeine Formel: 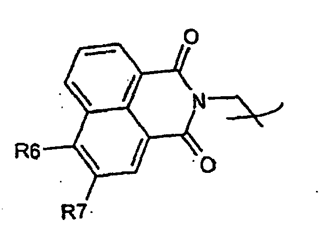 darstellt,
- Schritt 55 darin besteht, den Ester R₉ mittels eines Verfahrens zu spalten, das den vorstehend angegebenen Rest R₄ unberührt lässt,
- Schritt 56 identisch ist mit Schritt 48 und ausgeführt wird ausgehend von der im vorhergehenden Schritt erhaltenen Verbindung LXI und
- Schritt 57 darin besteht, den Rest R₄ der im vorhergehenden Schritt erhaltenen Verbindung LXII abzuspalten, unter Verwendung von z.B. NaI in Aceton unter Rückfluss binnen 15 h.

## Claims

1. Compounds of the following general formula (X): 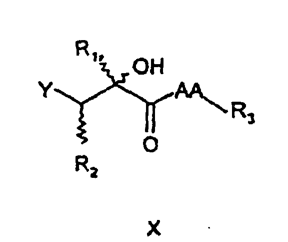 wherein:
- Y is:
• -CONHOH, or
• -SH, or
• a group of the formula 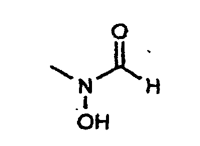 or
• a group of the formula 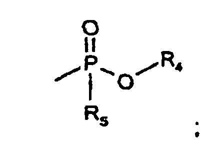 wherein:
• • R₄ is -H, or C₁-C₆ alkyl or phenylalkyl wherein the alkyl group is C₁-C6 alkyl,
• • R₅ is a group of the formula 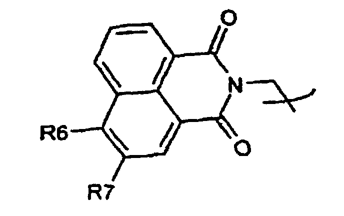 wherein:
• •• R₆ is -H, or C₁-C₆ alkoxy or benzyloxy,
• •• R₇ is -H, or halogen such as -Cl or -Br,
- R₁ is:
• a linear or branched C₃-C₁₆ alkyl or cyclized alkyl C₃-C₆ chain, said chain optionally including a heteroatom, such as O, S or N,
• a substituted or unsubstituted phenoxyalkyl or phenylalkyl or a heteroarylalkyl group, wherein the alkyl is C₂-C₅ alkyl,
- R₂ is:
• hydrogen, or
• C₁-C₅ alkyl, or C₂-C₅ alkylidene, or
• hydroxy, C₁-C₆ alkoxy or benzyloxy with the proviso that Y is -CONHOH when R₂ is hydroxy, or
• hydroxymethyl, or C₁-C₆ alcoxymethyl, or
• arylalkyl, wherein the alkyl portion is C₁-C₆ alkyl, aryloxymethyl, arylthiomethyl, heteroarylthiomethyl wherein aryl means a phenyl residue, optionally substituted in particular with -OH, -OCH₃, a linear or branched C₁-C₃ alkyl, a halogen such as -Cl or -Br, an amino group such as -NH₂, -NHCOCH₃, -NHCOOR₁₀, wherein R₁₀ is a linear or branched C₁-C₃ alkyl, or
• phtalimidoalkyl, wherein the alkyl portion is C₁-C₆ alkyl, or
• alkoxycarbonylmethyl (alkoxy meaning methoxy, ethoxy), benzyloxycarbonylmethyl, acetylmethyl with the proviso that in these three cases, Y is -SH,
- AA is an amino acid, or a chain of amino acids, wherein the amino acids are either natural or not, and advantageously, with the absolute S configuration, in particular an amino acid of formula 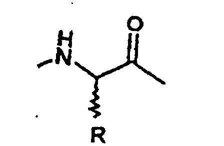 wherein R is:
• a C₁-C₄ linear or branched alkyl chain,
• -CH₂-Y' wherein Y' is a cycle with 4 to 6 carbon atoms in the cycle, optionally including one or more heteroatoms such as O, S or N, wherein said cycle is either aromatic or not, optionally substituted in particular with one or more -OCH₃, -NO₂, -NH₂ groups, or with one or more halogens selected in particular from -Cl, -Br, -F and -I,
• a group of formula 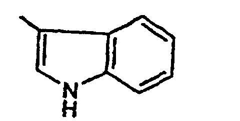
- R₃ is a group of formula -NH-(R₈)ₙ-R₉ wherein:
• n is 0 or 1,
• R₈ is a linear or branched alkyl chain with 1 to 8 carbon atoms, optionally including one or more heteroatoms, such as O or S,
• R₉ is hydrogen or methyl, nitrile, morpholino, phenyl, methoxy, hydroxy, thiomethyl, or a group of formula -CH(NH₂)=N-OH, or -N(CH₃)₂.

2. The compounds according to claim 1, **characterized by** the following formula (Xa): 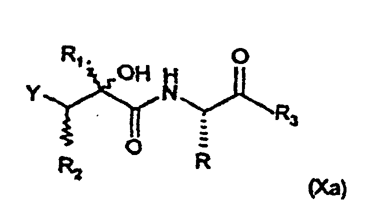 wherein:
- Y is:
• -CONHOH,
- R₁ is:
• -CH(CH₃)₂,
• -CH₂-CH(CH₃)₂,
• 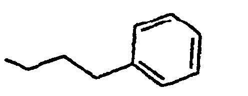
- R₂ is:
• an alkyl group with 1 to 5 carbon atoms, in particular methyl or propyl.
• hydroxy, or
• an alkoxy group with 1 to 5 carbon atoms, in particular methoxy,
- R is:
• -CH(CH₃)₃,
• -CH₂-CH(CH₃)₂,
• an aromatic or non-aromatic group 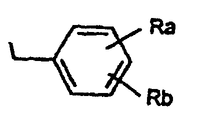 wherein Rₐ and R_{b}, independently, represent -H, -Cl, -Br, -I, -F, -OCH₃, -NO₂, -NH₂,
• a group of formula 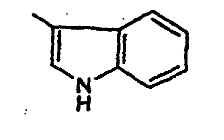
- R₃ is -NH-(CH₂)ₙ₁-R₉ wherein:
• n₁ is 0, 1 or 2,
• R₉ is -CH₃, -C≡N, -COOCH₃, -SCH₃, -O-(CH₂)₂-OH, -O-(CH₂)₂-OCH₃, -CH(NH₂)=N-OH,

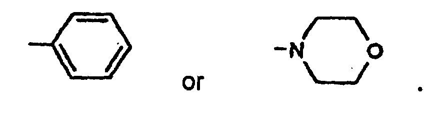

3. The compounds according to claim 1 or claim 2, **characterized in that** they have a stereochemistry such that the R₁ and R₂ substituents are in the anti position with respect to the succinic residue according to the following formula (XI. 1):

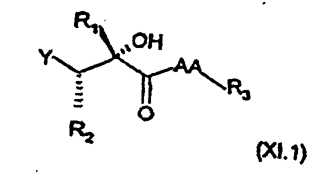

4. The compounds according to any of claims 1 to 3, **characterized in that** R is a group of formula: 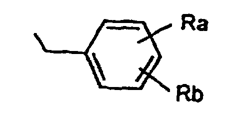 wherein Rₐ and R_{b} represent halogen, in particular a chlorine atom.

5. The compounds according to any of claims 1 to 4, **characterized in that** R₃ is a group of formula
-NH-(CH₂)₂-SCH₃.

6. A mixture **characterized in that** it comprises compounds of the following formula (XI.1): 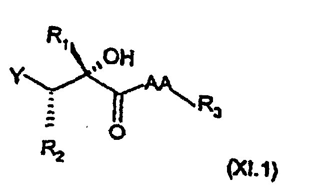 wherein Y, R₁ and R₂, AA and R₃ are as defined in any of claims 1 to 5, and compounds of the following formula (XI.2): 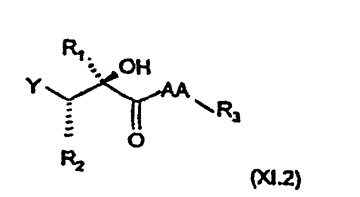 wherein Y, R₁, R₂, AA and R₃ have the aforementioned meaning,
wherein the proportion of compounds (XI.1) and (XI.2) in the mixture is advantageously from about 50% to about 99% for the compound of formula (XI.1) and from about 50% to about 1% for the compound of formula (XI.2).

7. The compound according to any of claim 1 to 5, **characterized by** the following formulae:

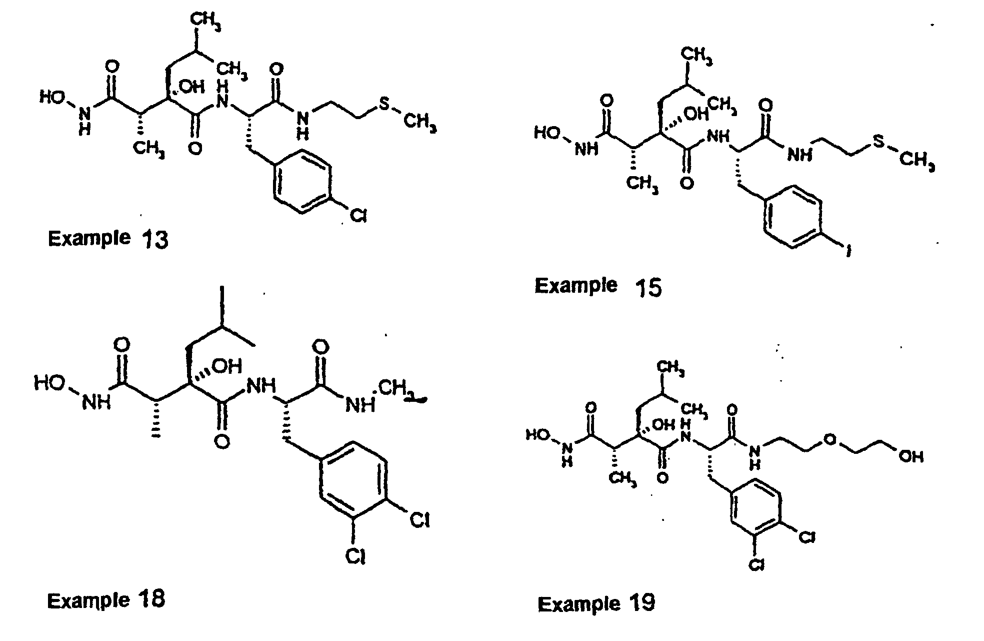

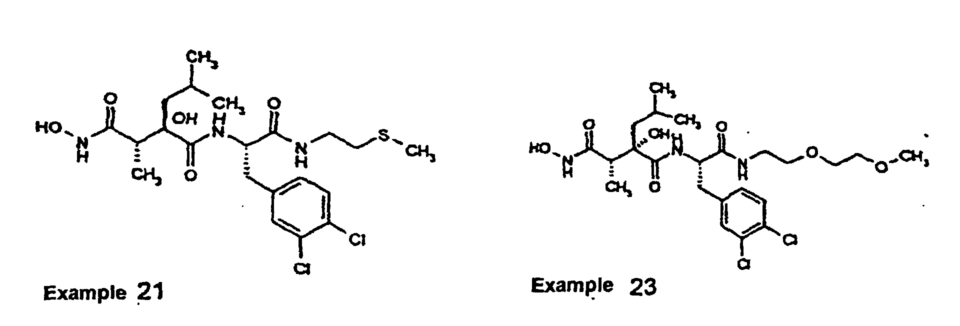

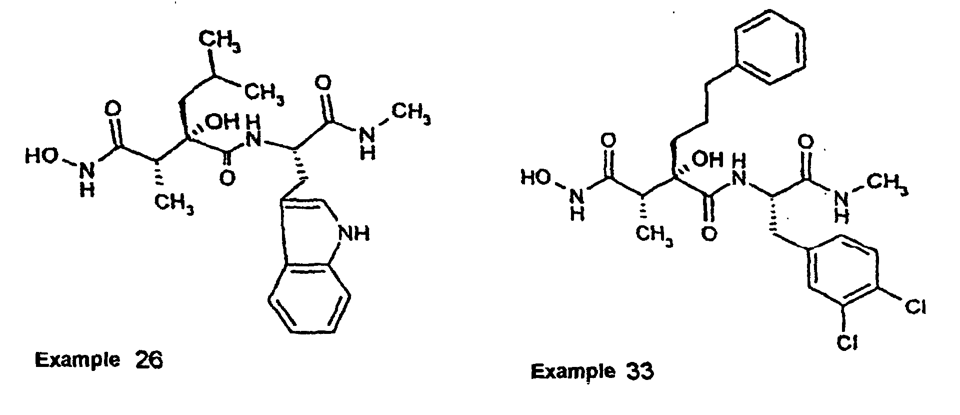

8. A pharmaceutical composition **characterized in that** it comprises as an active ingredient, one (or more) compound(s) and/or one (or more) mixture(s), according to any of claims 1 to 5, in combination with a pharmaceutically acceptable carrier.

9. The pharmaceutical composition according to claim 8, **characterized in that** it is in an oral, parenteral or rectal dosage form.

10. The pharmaceutical composition according to claim 8 or claim 9, **characterized in that** the active ingredient dosage is from about 0,1 to about 500 mg/kg/day, preferably from 1 to 300 mg/kg/day orally and rectally, and from about 0,1 µg/kg/day to 1 mg/kg/day parenterally.

11. The pharmaceutical composition according to any of claims 8 to 10, **characterized in that** it is in an oral dosage form, with a unit dosage of 1 mg to 250 mg of active ingredient per administration, and preferably from 10 mg to 250 mg of active ingredient per administration, with 1 to 4 administrations per day.

12. The pharmaceutical composition according to any of claims 8 to 10, **characterized in that** it is in aparenteral dosage form, with a unit dose of 1 µg to 50 mg of active ingredient per injection, with 1 to 2 injections per day.

13. The use of one (or more) compound(s) according to any of claims 1 to 5, and/or of one (or more) mixture(s) according to claim 6, for preparing a drug for the treatment of human or animal pathologies involving metalloproteinases and/or TNFα and/or TGFα.

14. The use according to claim 13, for preparing a drug with the property of inhibiting the action of metalloproteinases involved in the degradation of the extra-cellular matrix, such as collagenases, gelatinases, and stromelysins, wherein this drug is for treating human or animal pathologies, related to this metalloproteinase action, in particular for treating:
- rheumatoid arthritis,
- osteoarthritis,
- osteoporosis,
- corneal ulceration,
- periodontitis,
- gingivitis,
- tumoral invasions,
- metastasis proliferation,
- atherosclerosis,
- AIDS,
- chronic bowel inflammatory diseases,
- neurodegenerative diseases such as Alzheimer's disease, multiple sclerosis.

15. The use according to claim 13, for preparing a drug with the property of inhibiting release of TNFα from its inactive precursor, wherein this drug is for treating human or animal pathologies involving TNFα, in particular for treating malignant, infectious, immunological, inflammatory pathologies, such as:
- rheumatoid arthritis,
- Crohn's disease,
- multiple sclerosis,
- septical shock,
- cancer,
- cachexia associated with immunodeficiency.

16. The use according to claim 13, for preparing a drug with the property of inhibiting production of TGFα, wherein this drug is for treating human or animal pathologies involving TGFα, such as:
- cancer,
- psoriasis,
- eczema,
- cheloid formation
- diabetic retinopathy,
- atherosclerosis,
- inflammatory diseases.

17. A method for preparing compounds of formula X as defined in claim 1, wherein Y is -CONHOH (further designated hereafter as compounds of formula XV), **characterized in that** it is performed according to the following scheme: 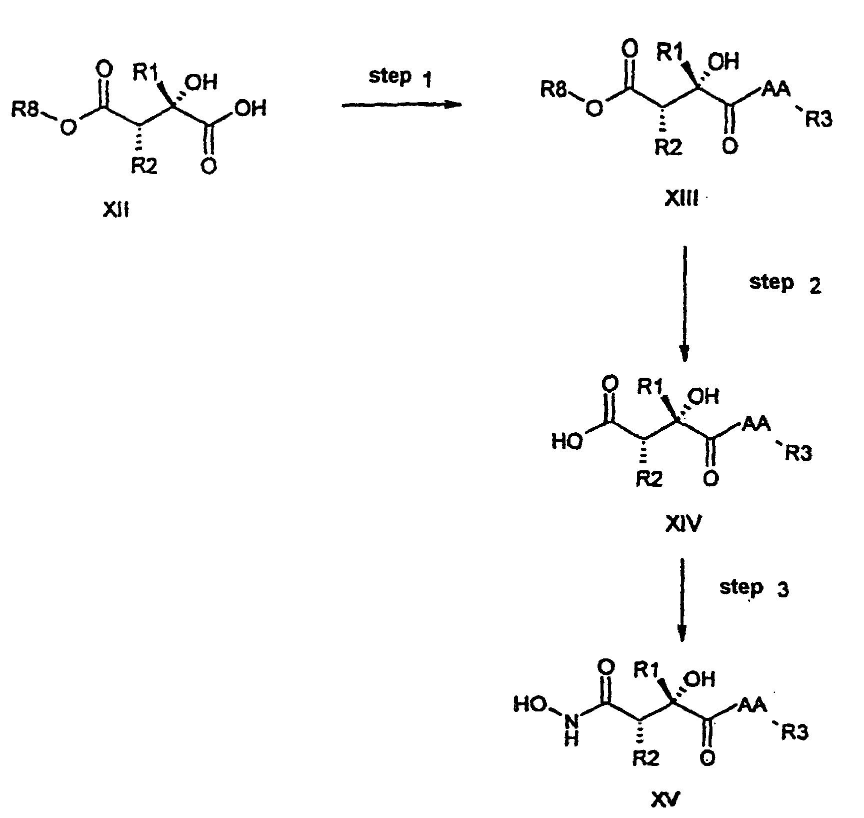 wherein:
- step 1 consists in condensing α-hydroxysuccinic acid XII (wherein R₈ is a protecting group compatible with the different components of the molecule such as t-butyl or benzyl) with an AA-R₃ residue, wherein AA and R₃ are as defined in claim 1, by a coupling method used in peptide synthesis and preferably PyBop at room temperature for 1 to 24 h (if R₂ = OH, the alcohols may be protected beforehand with a silylated derivative, for example),
- step 2 consists in hydrolyzing the ester XIII obtained in the previous step into a carboxylic acid XIV with trifluoroacetic acid, in particular at room temperature in a solvent such as CH₂Cl₂ for 1 to 10 h when R₈ is t-butyl, or in hydrogenolysing ester XIII into an acid XIV for example with H₂ Pd/C when R₈ is benzyl (in particular under atmospheric pressure in a polar solvent such as ethanol for 30 min to 10 h),
- in step 3, hydroxamic acid XV is formed by reaction of hydroxylamine, O-protected hydroxylamine, or N,O-diprotected hydroxylamine, preferably with O-THP hydroxylamine, or O-benzyl hydroxylamine (when R₂ is different from alkylidene, aryloxymethyl and heteroarylthiomethyl) in the presence of a coupling reagent such as DCC/HOBT or WSC/HOBT at room temperature in a solvent such at THF, CH₂Cl₂ or DMF for 1 to 24 h (when R₂ = OH, the alcohols are protected beforehand with TMSCI, for example); the O or N,O-(di)protected hydroxylamine are then deprotected according to the nature of the protecting group, for example in an acid medium for O-THP hydroxylamine (in particular at room temperature in a THF-H₂O mixture for 1 to 24 h) or H₂ Pd/C for O-benzyl hydroxylamine (in particular under atmospheric pressure in a polar solvent such as ethanol for 30 min to 10 h).

18. A method for preparing compounds of formula X as defined in claim 1, wherein Y is -CONHOH (further designated hereafter as compounds of formula XV), with the proviso that R₁ is not a heteroarylalkyl group and that R₂ is not a heteroarylthiomethyl group, **characterized in that** it comprises:
- an aldolization reaction, from a keto-ester XXVII and an alkene XXVIII (in particular in the presence of a Lewis acid such as SnCl₄ at -80°C, in a solvent such as CH₂Cl₂ for 5 min to 2 h), or from a keto-acid (as a sodium or triethylamine salt) XXX and an alkene XXXI (in particular at room temperature between 1 and 10 h in a THF-H₂O mixture) according to the following scheme: 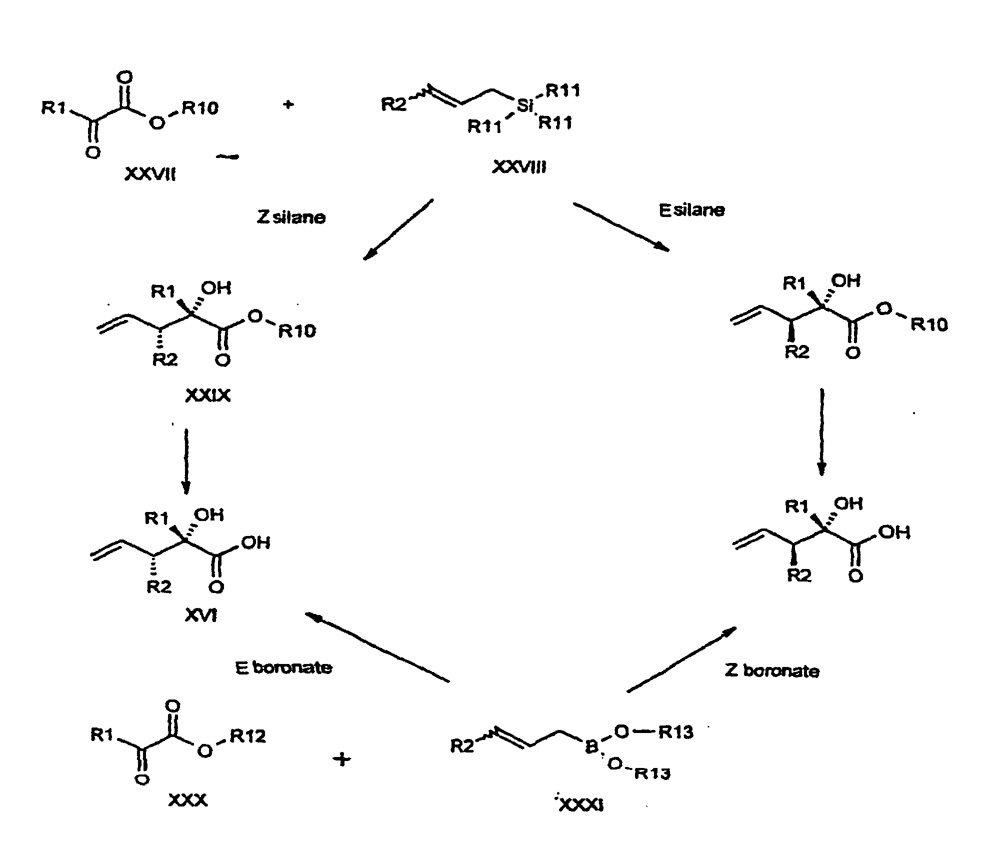
wherein:
R₁ and R₂ have the meanings given above,
R₁₀ is an optionally branched C₁-C₁₂ alkyl, benzyl, or a optically pure compound such as mandelic acid esterified by a linear or branched C₁-C₃ alkyl, or benzyl,
- R₁₁ is a linear or branched C₁-C₃ alkyl, or chloro,
- R₁₂ is sodium or triethylamine,
- R₁₃ is hydrogen, a linear or branched C₁-C₃ alkyl; R₁₃ may also be a chain forming a cycle with the boron atom as di-isopropyl tartrate for example,
- the reactions are diastereoselective and lead to stereochemical derivatives XVI if the double bond has a Z geometry for compound XXVIII and E geometry for compound XXXI,
- aforementioned compounds of formula XV are then obtained according to the following reaction scheme: 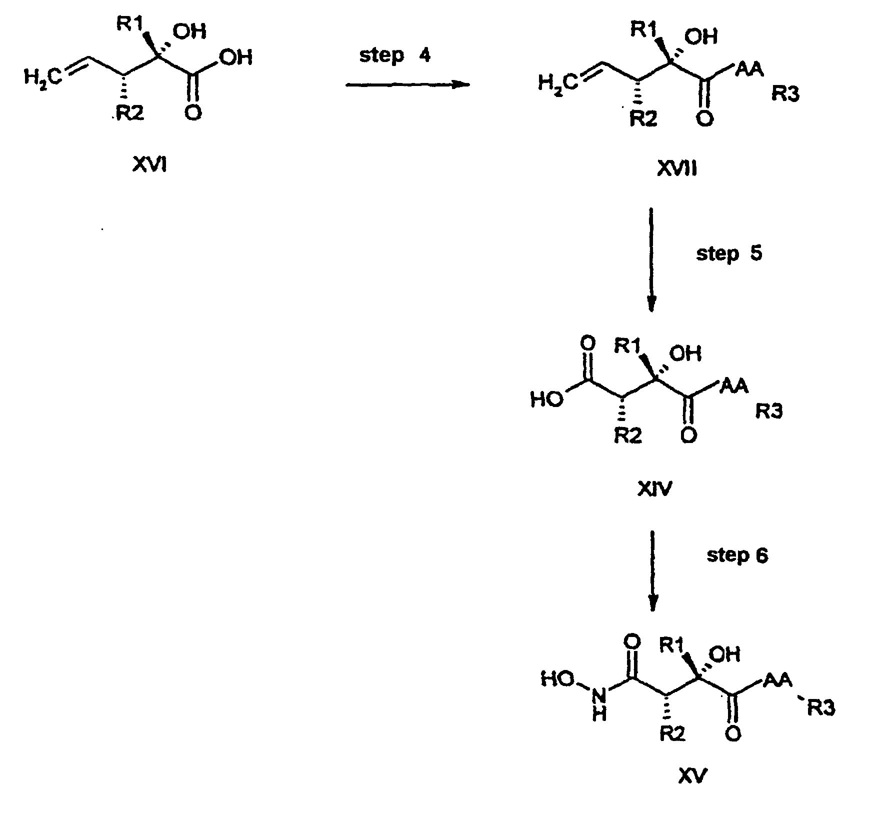 wherein:
- steps 4 and 6 are achieved as in steps 1 and 3 of the reaction scheme according to claim 17 respectively, and from compounds XVI and XIV, leading to compounds of formula XVII and XV respectively;
- step 5 consists in oxidizing the ethylene double bond of the compound of formula XVII into an acid, in particular by ozonolysis (for example, at -60°C in CH₂Cl₂ until a persisting blue color is achieved) then by oxidization (in particular at room temperature with NaClO₂ and NaH₂PO₄ in tBuOH-H₂O for 15 h) or directly with KMnO₄/NaIO₄ (in particular at room temperature in a mixture tBuOH-H₂O for 1 to 10 h), leading to the compounds of formula XIV.

19. A method for preparing compounds of formula X as defined in claim 1, wherein Y is:
• -SH, or
• a group of formula 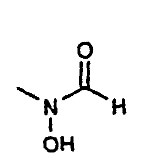 or
• a group of formula 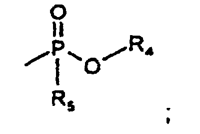
wherein R₄ and R₅ are as defined in claim 1,
wherein said method is conducted according to the following scheme: 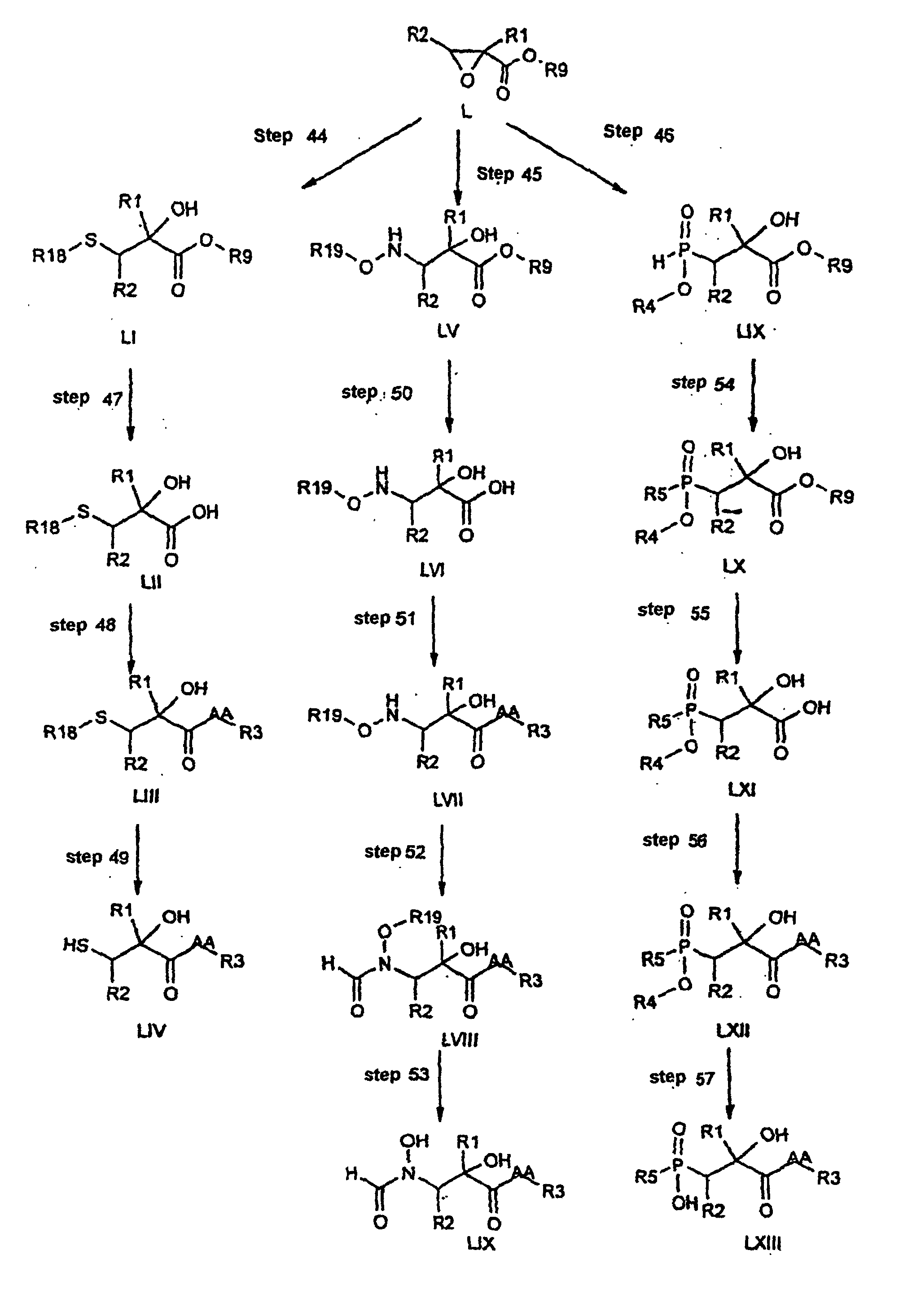 wherein:
- step 44 consists in opening an epoxide of formula L wherein R₁ and R₂ are as defined in claim 1, and R₉ is a carboxylic acid protecting group, in particular a benzyl residue sensitive to catalytic hydrogenolysis, wherein said opening of the epoxide L is carried out by a nucleophilic agent for example a thiol protected with a group R₁₈ compatible with R₉, for example a benzyl, in methanol for 1 h at 60°C,
- step 47 consists in deprotecting ester LI, for example with trifluoroacetic acid as previously,
- step 48 is identical with step 1 of the reaction scheme according to claim 17, and performed from the LII compound obtained in the previous step,
- step 49 consists in deprotecting the sulfur atom for example with sodium in liquid ammonia, in particular at -60°C for 5 to 15 min,
- step 45 consists in opening epoxide L, with the protected hydroxylamine, as defined in step 3 of the method according to claim 17 (for example, with R₁₉ = benzyl or THP),
- step 50 consists in deprotecting ester LV by a method compatible with R₁₉,
- step 51 is identical with step 48, and performed from compound LVI obtained in the previous step,
- step 52 consists in reacting the hydroxylamine LVII with formic acid and acetic anhydride, in particular at a temperature of at least 100°C for 1 to 15 h,
- step 53 consists in cleaving R₁₉ on compound LVIII for example with H₂ Pd/C or HCI 1N, according to the structure of R₁₉ as before,
- step 46 consists in opening an epoxide L with hypophosphorous acid of formula H₃PO₂ and then esterifying with a coupling agent such as DCC and a group R₄OH wherein R₄ is as defined in claim 1, in the presence for example, of trimethyl orthoformate and tetramethylguanidine at room temperature for 5 h,
- step 54 consists in treating compound LIX with a compound of formula: 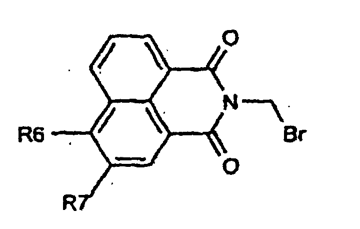 (prepared according to methods described in the literature) wherein R₆ and R₇ are as defined above, in particular in CH₂Cl₂ in the presence of bis-trimethylsilyl acetamide at room temperature for 5 h, leading to the compound of formula LX wherein R₅ is: 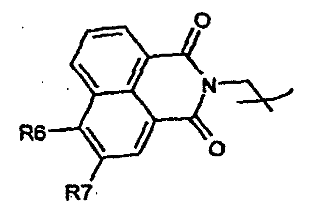
- step 55 consists in cleaving ester R₉ by a method compatible with R₄ as before,
- step 56 is identical with step 48, and performed from compound LXI as obtained in the previous step,
- step 57 consists in cleaving group R₄ of compound LXII as obtained in the previous step, for example, with Nal in refluxed acetone, for 15 h.
